# EUROPÄISCHE PATENTSCHRIFT

(11) **EP 2 578 671 B1**
(45) Veröffentlichungstag und Bekanntmachung des Hinweises auf die Patenterteilung: **30.12.2015**
(21) Anmeldenummer: 11184441.1
(22) Anmeldetag: 07.10.2011
(51) Int. Cl.: C11B 9/00, A61Q 13/00, C07C 43/305, C07C 47/11, C11D 3/50, C11C 5/00, C07C 47/225, A61Q 5/02, A61Q 5/06, A61Q 5/10, A61Q 5/12, A61Q 15/00, A61Q 17/04, A61Q 19/00, A61Q 19/10, A61K 8/35

(54) **Riechstoffe mit Maiglöckchennote**
Aromatic substances with lily of the valley note
Parfums dotés de notes de muguet

(43) Veröffentlichungstag der Anmeldung: 10.04.2013
(73) Patentinhaber: Symrise AG, 37603 Holzminden (DE)
(72) Erfinder: Singer, Emilie, 37603 Holzminden (DE); Hölscher, Bernd, 37620 Halle (DE)
(74) Vertreter: Fabry, Bernd

(56) Entgegenhaltungen:
- EP-A1- 1 529 770
- EP-A1- 2 333 038
- WO-A1-2004/089861
- JP-A- 50 094 143
- LALANDE R ET AL: "ADDITION RADICALAIRE DE L'ETHANOL ET DE L'ETHANAL SUR LE BETA-PINENE", COMPTES RENDUS HEBDOMADAIRES DES SEANCES DE L'ACADEMIE DESSCIENCES, SERIE C: SCIENCES CHIMIQUES, GAUTHIER-VILLARS. MONTREUIL, FR, Bd. 264, Nr. 12, 20. März 1967 (1967-03-20), Seiten 1083-1086, XP009041185,
- MAILLARD B ET AL: "ADDITIONS RADICALAIRES. XI. - ADDITIONS DE DIOXOLANNES AU BETA-PINENE", BULLETIN DE LA SOCIETE CHIMIQUE DE FRANCE. 1 PARTIE -CHIMIE ANALYTIQUE, MINERALE ET PHYSIQUE, SOCIETE FRANCAISE DE CHIMIE. PARIS, FR, Nr. 4, PART 02, 1. Januar 1973 (1973-01-01), Seiten 1368-1372, XP009041186,

## Beschreibung

### GEBIET DER ERFINDUNG

Die vorliegende Erfindung betrifft primär neue Verbindungen ausgewählt aus der Gruppe bestehend aus der Verbindung der Formel (la) 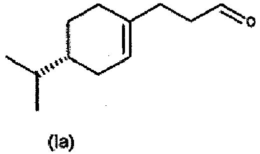 sowie Mischungen umfassend die Verbindung der Formel (la) wie oben definiert und die Verbindung der Formel (Ib) 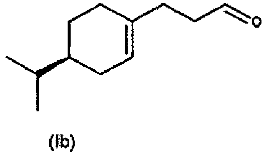 oder bestehend aus der Verbindung der Formel (la) und der Verbindung der Formel (Ib) wie oben definiert.

Daneben betrifft die Erfindung die Verwendung einer Verbindung der Formel (la) sowie von Mischungen umfassend oder bestehend aus Verbindungen der Formeln (la) und (Ib) - jeweils wie oben definiert - als Riechstoff, insbesondere als Maiglöckchen-Riechstoff, sowie Riechstoffzubereitungen und parfümierte Produkte umfassend eine (vorzugsweise sensorisch wirksame) Menge der Verbindung der Formel (la) bzw. einer Mischung der Verbindungen (la) und (Ib) - jeweils wie oben definiert - sowie Verfahren zur Herstellung einer Verbindung der Formel (la) wie oben definiert.

### STAND DER TECHNIK

Verbindungen mit blumigem Geruch sind eine unverzichtbare Komponente in der Riechstoffindustrie sowie bei der Herstellung von Kosmetika, Körperpflegemitteln sowie Wasch- und Reinigungsmitteln. Eine besonders wertvolle Klasse dieser blumigen Riechstoffe sind Verbindungen mit einer Maiglöckchen-Geruchsnote.

Einige Vertreter dieser Klasse von Riechstoffen weisen eine Struktur auf umfassend ein 4-Alkyl-Phenylpropanal als Grundgerüst, wie z.B. 3-(4-tertbutylphenyl)-2-methylpropanal (Formel (A); Lilial(R), Hersteller: Givaudan S.A.), 3-(4-tertbutylphenyl)propanal (Formel (B); Bourgeonal(R), Hersteller: Quest), 3-(4-Isopropylphenyl)-2-methylpropanal (Formel (C), Cyclamal(R), Hersteller: Givaudan S.A.) und 3-(3-Isopropylphenyl)-butanal (Formel (D), Florhydral(R), Hersteller: Givaudan S.A.).

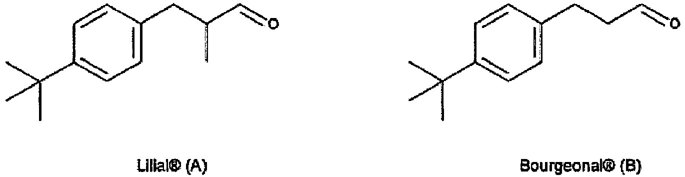

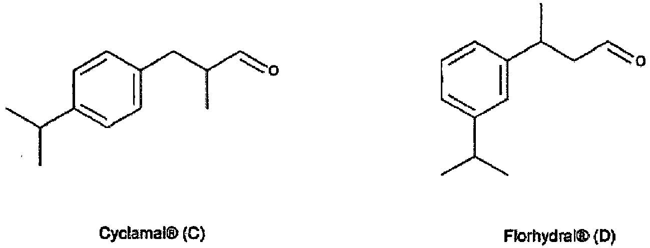

3-(4-tertbutylphenyl)-2-methylpropanal (A), ein synthetischer Riechstoff, war über lange Zeit einer der bedeutendsten industriell eingesetzten Riechstoffe für blumige Duftkompositionen, insbesondere mit Maiglöckchen-Geruchsnote, und wurde in der Kosmetik- und Seifenindustrie in großen Mengen verwendet. Dann zeigten jedoch Resultate von Tierversuchen, dass 3-(4-tertbutylphenyl)-2-methylpropanal reprotoxisch sein könnte. Ausserdem wurde festgestellt, dass diese Verbindung möglicherweise ein Allergen ist und KontaktDermatitis bei empfindlichen Personen verursachen könnte. Da die Verbindungen 3-(4-tertbutylphenyl)propanal (B), 3-(4-Isopropylphenyl)-2-methylpropanal (C) und 3-(3-Isopropylphenyl)-2-methylpropanal (D) strukturell der Verbindung 3-(4-tertbutylphenyl)-2-methylpropanal (A) sehr ähnlich sind, ist es durchaus möglich, dass diese Verbindungen in ähnlicher Weise toxisch sind.

Das Auftreten eines Geruchs mit einer Maiglöckchennote ist jedoch nicht an das oben beschriebene strukturelle Grundgerüst gebunden.

So offenbart die Patentschrift US 6,376,458 Verbindungen, die unter folgende allgemeine Formel (E) fallen, die im Gegensatz zu den oben beschriebenen Verbindungen der Formeln (A) bis (D) anstelle einer Phenylgruppe eine gesättigte oder ungesättigte Cyclohexylgruppe aufweisen:

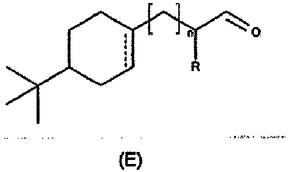

In der Formel (E) bedeutet die endocyclische gestrichelte Linie eine Einfachbindung oder eine Doppelbindung, und die Gruppe R ist eine Methylgruppe oder ein Wasserstoffatom.

Die in der Patentschrift US 6,376,458 spezifisch offenbarte Verbindung 3-(4-tertbutyl-1-cyclohexen-1-yl)-2-methylpropanal weist einen Geruch umfassend die Geruchsnoten Maiglöckchen, fast weiße Blüten und Freesien auf. Die gemäß US 6,376,458 bevorzugte Verbindung 3-(4-tertbutyl-1-cyclohexen-1-yl)propanal zeigt einen Geruch vom aldehydigen, blumig-maiglöckchenartigen, fettigen Typ mit einer Beinote von Lilial^{®}/Bourgeonal^{®}, wobei dieser Duft jedoch blumiger und die Note der weißen Blüten ausgeprägter ist als bei Lilial^{®}.

Die Geruchsnoten der in der US 7,834,219 offenbarten Verbindungen der Formel (F) weisen eine starke blumige Note und eine Maiglöckchennote auf. Die Verbindungen unterscheiden sich von den Verbindungen (A) bis (D) dadurch, dass sie statt einer Phenylgruppe eine Cyclohexylgruppe umfassen. In der Formel (F) bedeutet die Gruppe R eine Ethyl-, Isopropyl-, oder sec-Butylgruppe.

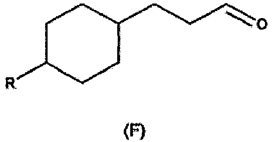

Jedoch ist keine dieser aus dem Stand der Technik bekannten Verbindungen (B) bis (F) in der Lage, den Geruchseindruck der natürlichen Maiglöckchenblüte in seiner gesamten Komplexität wiederzugeben

Andererseits sind auch Verbindungen bekannt, die trotz struktureller Ähnlichkeit zu den oben beschriebenen Verbindungen keine Maiglöckchen-Geruchsnote aufweisen, wie zum Beispiel die in der Patentschrift US 3,716,498 beschriebene Verbindung Pinoacetaldehyd der Formel (G):

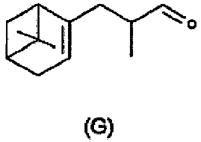

Der Geruch dieses Moleküls wird als anhaltend, sehr frisch, stechend, blumig, holzig und ozonig beschrieben.

Auch die Verbindung 3-(4-isopropyl-1-cyclohexen-1-yl)-2-methylpropanal (Formel (H) zeigt trotz struktureller Ähnlichkeit zu den Verbindungen (A) bis (F) keine Maiglöckchen-Geruchsnote. Vielmehr ist das Geruchsprofil diese Verbindung aldehydisch, süß, wässrig und blumig (Richtung Cyclamen).

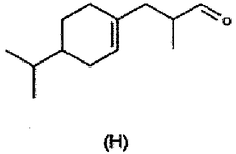

Trotz einer Vielzahl bereits vorhandener Riechstoffe besteht in der Parfümindustrie zudem auch weiterhin ein genereller Bedarf an neuen Riechstoffen, welche über ihre primären, nämlich geruchlichen, Eigenschaften hinaus zusätzliche positive Sekundäreigenschaften besitzen, wie z.B. eine höhere Stabilität unter bestimmten Anwendungsbedingungen, eine höhere Substantivität und/oder Diffusivität oder ein besseres Haftungsvermögen, oder durch Synergieeffekte mit anderen Riechstoffen zu besseren sensorischen Profilen führen.

Riechstoffe, die sich durch die oben erwähnten positiven Sekundäreigenschaften auszeichnen, ermöglichen eine erhöhte Effektivität bei der Herstellung von Riechstoffzubereitungen und parfümierten Produkten. So können z.B. durch den Einsatz von Riechstoffen mit einem besseren sensorischen Profil, einer höheren Substantivität und/oder einem besseren Haftungsvermögen die Anzahl und die Einsatzmengen von Riechstoffen in entsprechenden Formulierungen optimiert und/oder minimiert werden, was zu einer nachhaltigen Ressourcenschonung bei der Herstellung parfümierter Produkte führt.

Daher besteht in der Parfümindustrie insbesondere ein Bedarf an weiteren Riechstoffen mit verbesserten sensorischen Profilen und/oder Sekundäreigenschaften.

Primäre Aufgabe der vorliegenden Erfindung war es, einen Riechstoff mit einer blumigen, insbesondere Maiglöckchen-Geruchsnote anzugeben, dessen geruchliche Eigenschaften vergleichbar mit 3-(4-tertbutylphenyl)-2-methylpropanal (Lilial®) sind, das heißt, der Riechstoff sollte einen Geruchseindruck vermitteln, der der Komplexität des natürlichen Geruchs der Maiglöckchenblüte weitgehend entspricht. Darüber hinaus sollte dieser Riechstoff vorzugsweise hinsichtlich der Summe seiner Sekundäreigenschaften bzw. zumindest einiger seiner Sekundäreigenschaften den aus dem Stand der Technik bekannten Maiglöckchen-Riechstoffen überlegen sein.

Ein Riechstoff ist im Rahmen des vorliegenden Textes jede Substanz, die dazu geeignet ist, zum Hervorrufen eines geruchlichen Eindrucks verwendet zu werden, d.h. zum Vermitteln eines geruchlichen Eindrucks, oder zum Verändern (Modifizieren oder Verstärken) der geruchlichen Wahrnehmung einer anderen Substanz. Dabei soll diese Substanz, damit sie für parfümistische Zwecke eingesetzt werden kann, vorzugsweise keine unerwünschten Nebenwirkungen, z.B. die Gesundheit oder die Umwelt schädigende Wirkungen, oder den bestimmungsgemäßen Gebrauch eines Produkts, das diesen Riechstoff enthält, beeinträchtigende Wirkungen haben darf.

### BESCHREIBUNG DER ERFINDUNG

Die gestellte Aufgabe wird erfindungsgemäß gelöst durch die Verwendung einer Verbindung der Formel (la) 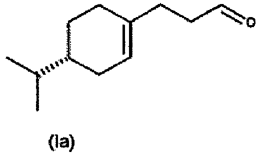 sowie Mischungen umfassend die Verbindung der Formel (la) wie oben definiert und die Verbindung der Formel (Ib) 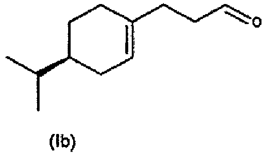 oder bestehend aus der Verbindung der Formel (la) und der Verbindung der Formel (Ib) wie oben definiert.
- als Riechstoff, insbesondere als Maiglöckchen-Riechstoff und/oder
- als Mittel zum Erhöhen der Substantivität und/oder der Retention einer Riechstoffzubereitung; und/oder
- als Fixateur.

In diesem Text bedeuten geschlängelte Linien eine Bindung, die sich (je nach vorliegendem Enantiomer) aus der Zeichenebene heraus oder hinter die Zeichenebene erstreckt.

Weitere Aspekte der Erfindung ergeben sich aus der nachfolgenden Beschreibung, den Ausführungsbeispielen, der Figur und den beigefügten Patentansprüchen.

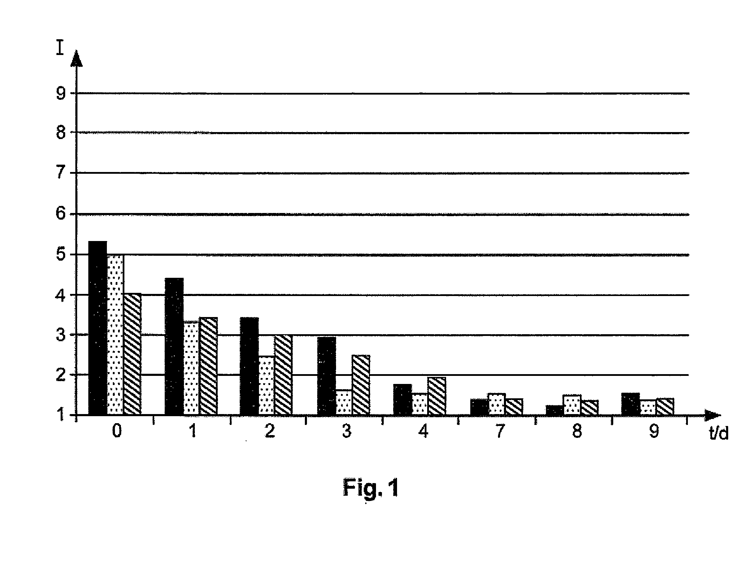

Figur 1 zeigt die Bewertung des Substantivität der Verbindung der Formel (la) und der Verbindung der Formel (Ib) im Vergleich zu Lilial® (A) in Anhängigkeit von der Zeit

Überraschenderweise hat sich gezeigt, dass eine Verbindung der Formel (I) einen Geruchseindruck vermittelt, der der Komplexität des natürlichen Geruchs der Maiglöckchenblüte sehr nahe kommt. D.h., der von der Verbindung der Formel (I) vermittelte Geruchseindruck zeichnet sich durch eine herausragende Natürlichkeit und Komplexität aus, insbesondere hinsichtlich der Maiglöckchen-Geruchsnote. Ein derartig komplexer, dem natürlichen Geruch der Maiglöckchenblüte weitgehend entsprechender Geruchseindruck ist mit den aus dem Stand der Technik bekannten Riechstoffen mit Maiglöckchen-Geruchsnote bisher nicht erreicht worden.

Die Suche nach geeigneten Stoffen mit Maiglöckchen-Geruchsnote, die zur vorliegenden Erfindung führte, wurde durch folgende Sachverhalte erschwert:
- die Mechanismen der Geruchswahrnehmung sind nicht ausreichend bekannt;
- die Zusammenhänge zwischen der speziellen Geruchswahrnehmung einerseits und der chemischen Struktur des zugehörigen Riechstoffs andererseits sind nicht hinreichend erforscht;
- häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Der Erfolg der Suche nach geeigneten Riechstoffen hängt deshalb stark von der Intuition des Suchenden ab.

Dass das Geruchsprofil der erfindungsgemäß einzusetzenden Verbindung der Formel (I) dem Geruchsprofil von 3-(4-tertbutylphenyl)-2-methylpropanal sehr ähnlich ist, war nicht zu erwarten, da sich die Verbindung der Formel (I) strukturell in mehrfacher Hinsicht von 3-(4-tertbutylphenyl)-2-methylpropanal (obige Formel (A)) unterscheidet; die Phenylgruppe der Formel (A) ist in der Formel (I) durch eine Cyclohexenylgruppe ersetzt, die tert-Butylgruppe der Formel (A) durch eine Isopropylgruppe, und die Aldehyd-Seitenkette weist in der Formel (I) im Gegensatz zu Formel (A) keine Methylgruppe am zweiten Kohlenstoffatom auf.

Die erfindungsgemäß einzusetzenden Verbindungen der Formeln (la) bzw. (Ib) zeichnen sich durch ein hohes Aufziehvermögen (Eigenhaftung auf einem Substrat) und eine hohe Substantivität (Fähigkeit, aus einer, meist wässrigen, Phase heraus auf ein Substrat aufzuziehen bzw. auch nach einem Wasch- oder Spülvorgang auf einem Substrat zu verbleiben) aus. Dieser Effekt zeigt sich insbesondere auf Substraten wie Haut, Haar und textilen Fasern (z.B. Wolle, Baumwolle, Leinen, synthetische Fasern).

Eine weitere wichtige anwendungstechnische Anforderung an Riechstoffe und Riechstoffzubereitungen, insbesondere für tensidhaltige Produkte, ist deren Substantivität gegenüber dem bzw. Retention am Substrat, insbesondere Haaren oder textilen Fasern. Die Begriffe "Substantivität" und "Retention" werden z.B. in EP 1 201 738 A1 ausführlich dargelegt, vergleiche dort die Abschnitte [0004]-[0005]. Gesucht werden generell Riechstoffe mit hoher Substantivität und/oder Retention.

Diesen Erkenntnissen entsprechend betrifft ein weiterer Aspekt der Erfindung insbesondere die Verwendung der erfindungsgemäß zu verwendenden Verbindung der Formel (**Ia**) als Mittel zum Erhöhen der Substantivität und/oder Retention einer Riechstoffzubereitung (insbesondere gegenüber bzw. auf Haaren bzw. textilen Fasern), bevorzugt einer Riechstoffzubereitung mit Maiglöckchen-Geruchsnote, vorzugsweise einer erfindungsgemäßen Riechstoffzubereitung wie weiter unten beschrieben.

Neben ihrem hohen Aufziehvermögen zeichnet sich die erfindungsgemäß zu verwendende Verbindung der Formeln (la) bzw. (Ib) durch ihre fixierenden Eigenschaften aus, d.h. sie stellen Fixateure dar. Als Fixateure erhöhen die erfindungsgemäß zu verwendenden Verbindungen der Formel (la) bzw. (Ib) die Haftfestigkeit von anderen Riechstoffen, sei es durch deren Dampfdruckerniedrigung oder geruchliche Verstärkung (z.B. Absenkung des Schwellenwertes). Die Erfindung betrifft daher auch - wie bereits erwähnt - die Verwendung der Verbindungen der Formeln (la) bzw. (Ib) als Fixateure.

Die Verbindung der Formel (la) bzw. (Ib) wurde zwar im Stand der Technik bereits erwähnt, jedoch wurde ihre Eignung als Riechstoff, insbesondere ihre besondere Eignung zum Vermitteln einer Maiglöckchen-Geruchsnote, dabei nicht erkannt.

Die Publikation Comptes Rendus des Seances de l'Academie des Sciences, Serie C: Sciences Chimiques 1967, 264, 1083-1086 berichtet über Untersuchungen der radikalischen Addition von Ethanol bzw. Ethanal an beta-Pinen. Die Verbindung p-menthen-1-yl-7-ethanal wird als ein Minderbestandteil (weniger als 5 percent) des Rohprodukts der radikalischen Addition von Ethanal an beta-Pinen mit Hilfe von Benzoylperoxid erhalten. Für diese Verbindung wird ein negativer Drehwert (-54,2 °) angegeben.

Die Publikation Zhurnal Organicheskoi Khimii 1974, 10, 1153-1157 berichtet über Untersuchungen der radikalischen Addition aliphatischer Aldehyde der Formel R-CH₂-CHO (R= H, Methyl oder Ethyl) an beta-Pinen mit Mn(III)-Acetat als Initiator, optional in Gegenwart katalytischer Mengen von Kupfer (II)-Acetat. Dabei wurde festgestellt, dass die Reaktion je nach Reaktionsbedingungen (Polarität des Lösungsmittels und Einsatzmenge des Katalysators) zu einem Aldehyd der Struktur 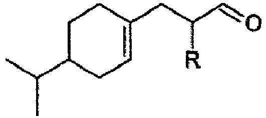 und einem Keton der Struktur 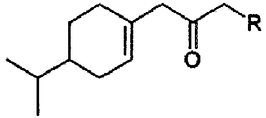 führt und/oder zu einem Dienaldehyd der folgenden Struktur 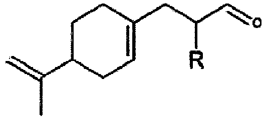 und einem Aldehydether der folgenden Struktur 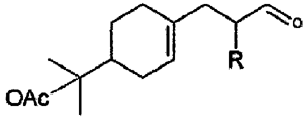 wobei R jeweils ausgewählt ist aus der Gruppe bestehend aus Wasserstoff, Methyl und Ethyl.

Die beiden letzteren Verbindungen werden in der Veröffentlichung Zhurnal Organicheskoi Khimii 1974, 10, 1153-1157 als neu eingeschätzt und ausführlich charakterisiert. Ausschließlich zu diesen Verbindungen wird in der genannten Veröffentlichung festgestellt, dass sie als Duftstoffe interessant sein können. Dies gelte insbesondere im Falle des Dienaldehyds mit R=Ethyl, da diese Verbindung ein strukturelles Analogon des Cyclamenaldehyds sei.

In der Publikation Bulletin de la Societe Chimique de France 1973, 4, 1368-1372 wird eine Verbindung der Formel R-CH₂-CHO, worin 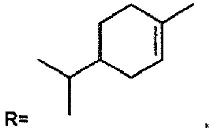 als Produkt der Oxidationsreaktion von (p-Menthen-1-yl-7)ethan-1,2-diol erwähnt, das selbst aus beta-Pinen und Ethanol durch eine radikalische Reaktion hergestellt wurde. Detaillierte Angaben zu den physikalischen und chemischen Eigenschaften der Verbindung (I) werden nicht offenbart.

Der vorstehend beschriebene Stand der Technik enthält keinerlei Angaben zu den geruchlichen Eigenschaften der Verbindung der Formel (I).

In den oben erwähnten Dokumenten aus dem Stand der Technik wird nicht zwischen den beiden Enantiomeren (Verbindung der Formel (la) wie oben definiert und Verbindung der Formel (Ib) wie oben definiert) unterschieden. Es ist daher nicht offenbart, ob und in welchen Anteilen die jeweils im Stand der Technik offenbarte Verbindung die Verbindung der Formel (la) wie oben definiert und/oder die Verbindung der Formel (Ib) wie oben definiert enthält. Der relative Anteil der beiden verschiedenen Enantiomeren wird durch die Enantiomerenzusammensetzung des jeweiligen Edukts beta-Pinen bestimmt, denn bei der radikalischen Addition eines Aldehyds an beta-Pinen bleibt die Stereochemie unverändert. beta-Pinen existiert in zwei enantiomeren Formen: (-)-beta-Pinen und (+)-beta-Pinen. In den oben erwähnten Dokumenten aus dem Stand der Technik wird lediglich offenbart, dass beta-Pinen als Edukt eingesetzt wird. Es ist daher nicht offenbart, in welchen Anteilen das als Edukt eingesetzte beta-Pinen die beiden Enantiomeren (-)-beta-Pinen bzw. (+)- beta-Pinen enthält.

Daher betrifft die vorliegende Erfindung auch eine Verbindung der Formel (la) 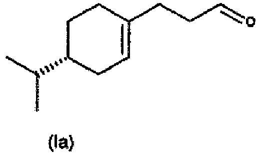 sowie eine Mischung umfassend
(i) die Verbindung der Formel (la) wie oben definiert und
(ii) die Verbindung der Formel (Ib) 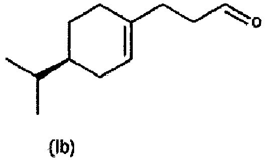
oder bestehend aus der Verbindung der Formel (la) wie oben definiert und der Verbindung der Formel (Ib) wie oben definiert.

Im vorliegenden Text wird unter der Verbindung der Formel (la) bzw. Formel (Ib) jeweils das entsprechende spezielle Enantiomer (wie oben definiert) verstanden, während die Bezeichnung "Verbindung der Formel (I)" sowohl die einzelnen Verbindungen ausgewählt aus der Gruppe bestehend aus der Verbindung der Formel (la) und der Verbindung der Formel (Ib) als auch sämtliche Mischungen (Enantiomerenmischungen) bestehend aus diesen beiden Verbindungen (in jedem beliebigen Mischungsverhältnis) umfasst. Das heißt, Ausführungen betreffend die "Verbindung der Formel (I)" gelten - wenn nicht anders ausgeführt - stets sowohl für die Verbindung der Formel (la) und die Verbindung der Formel (Ib) als auch für Mischungen bestehend aus der Verbindung der Formel (la) und der Verbindung der Formel (Ib) in jedem beliebigen Mischungsverhältnis.

In einer erfindungsgemäßen Mischung bestehend aus der Verbindung der Formel (la) wie oben definiert und der Verbindung der Formel (Ib) ist das Massenverhältnis der Verbindung der Formel (la) zur Verbindung der Formel (Ib) bevorzugt so gewählt, dass der Drehwert der Mischung nicht -54,2 ° beträgt. Bevorzugt ist in einer solchen Mischung das Massenverhältnis der Verbindung der Formel (la) zur Verbindung der Formel (Ib) so gewählt, dass ein Drehwert grösser als -54.2° resultiert, weiter bevorzugt grösser als -54° noch weiter bevorzugt grösser als -50 °.

Unter den Mischungen, die die Verbindungen der Formel (la) wie oben definiert und (ii) die Verbindung der Formel (Ib) wie oben definiert umfassen oder aus diesen beiden Verbindungen bestehen, sind erfindungsgemäß Mischungen bevorzugt mit
(a) einem Massenverhältnis der Verbindung der Formel (la) zu der Verbindung der Formel (Ib) im Bereich von 0.01 bis 99.99, vorzugsweise im Bereich von 0.1 bis 99.9, bevorzugt im Bereich von 0.5 bis 99.5, bevorzugt im Bereich von 1.0 bis 99.0, bevorzugt im Bereich von 1.5 bis 98.5, bevorzugt im Bereich von 2.0 bis 98.0 und/oder
(b) einem Gesamtanteil der Verbindungen der Formeln (la) und (Ib) grösser als 0.01 Gew.- percent, vorzugsweise grösser als 1 Gew.- percent, bevorzugt grösser als 10 Gew.- percent, weiter bevorzugt grösser als 50 Gew.- percent, besonders bevorzugt grösser als 90 Gew.- percent, bezogen auf das Gesamtgewicht der Mischung umfassend Verbindung (la) und/oder (Ib)

Das Geruchsprofil der Verbindung der Formel (la) wie oben definiert wird wie folgt beschrieben: Maiglöckchen-Geruchsnote, blumig, süß, wässrig, pudrig und ozonig, natürlich, pflegend, komplex und strahlend. Dabei ist insbesondere der komplexe, dem natürlichen Geruch der Maiglöckchenblüte weitgehend entsprechende Geruchseindruck bemerkenswert. Hinsichtlich der geruchlichen Eigenschaften ist die Verbindung der Formel (la) somit ähnlich zu 3-(4-tertbutylphenyl)-2-methylpropanal (Formel (A)).

Das Geruchsprofil der Verbindung der Formel (Ib) wie oben definiert wird wie folgt beschrieben: Maiglöckchen-Geruchsnote, fruchtig, grün, wässrig und aldehydig.

Die Verbindung der Formel (la) ist aufgrund ihres Geruchsprofils besonders geeignet zum Vermitteln, Modifizieren oder Verstärken einer Maiglöckchen-Geruchsnote und optional einer, mehrerer oder sämtlicher Geruchsnoten ausgewählt aus der Gruppe bestehend aus blumig, süß, wässrig, pudrig und ozonig.

Darüber hinaus hat sich gezeigt, dass die Verbindung der Formel (la) im Zusammenwirken mit einem oder mehreren weiteren Riechstoffen in der Lage ist, weitere für parfümistische Zwecke erwünschte Geruchsnoten hervorzurufen bzw. zu verstärken, insbesondere die Geruchsnoten cremig (siehe dazu Beispiele 10 und 14), kosmetisch (siehe Beispiel 10), rund aldehydisch (siehe Beispiel 12), grün (siehe Beispiel 13) und wässrig-fruchtig (siehe Beispiel 15).

Die Verbindung der Formel (Ib) ist aufgrund ihres Geruchsprofils besonders geeignet zum Vermitteln, Modifizieren oder Verstärken einer Maiglöckchen-Geruchsnote und optional einer, mehrerer oder sämtlicher Geruchsnoten ausgewählt aus der Gruppe bestehend aus fruchtig, grün, wässrig und aldehydig.

Daher wird die Verbindung der Formel (la) wie oben definiert erfindungsgemäß bevorzugt verwendet zum Vermitteln, Modifizieren oder Verstärken einer Maiglöckchen-Geruchsnote und optional einer, mehrerer oder sämtlicher Geruchsnoten ausgewählt aus der Gruppe bestehend aus blumig, süß, wässrig, pudrig und ozonig und/oder eines, mehrerer oder sämtlicher Eindrücke ausgewählt aus der Gruppe bestehend aus natürlich, pflegend, komplex und strahlend.

Alternativ oder zusätzlich wird die Verbindung der Formel (Ib) wie oben definiert erfindungsgemäß bevorzugt verwendet zum Vermitteln, Modifizieren oder Verstärken einer Maiglöckchen-Geruchsnote und optional einer, mehrerer oder sämtlicher Geruchsnoten ausgewählt aus der Gruppe bestehend aus fruchtig, grün, wässrig und aldehydig.

Ein weiterer Aspekt der vorliegenden Erfindung betrifft Riechstoffzubereitungen, insbesondere Parfümöle, wobei erfindungsgemäß aufgrund der oben beschriebenen Geruchsprofile der Verbindung der Formel (la) und der Verbindung der Formel (Ib) insbesondere Riechstoffzubereitungen bzw. Parfümöle mit einer Maiglöckchen-Geruchsnote von Interesse sind.

Eine Riechstoffzubereitung ist im Rahmen des vorliegenden Textes eine Mischung verschiedener Stoffe, welche gemäß einem Rezept oder einer Rezeptur nach einem vorgegebenen Verfahren aus den entsprechenden Stoffen erzeugt wird. Solche Zubereitungen werden gezielt zu dem Zweck hergestellt und eingesetzt, einen gewünschten, üblicherweise als angenehm oder in einer sonstigen Weise positiv empfundenen Geruchseindruck zu vermitteln, zu modifizieren oder zu verstärken. Zubereitungen, wie sie in der präparativen organischen Chemie eingesetzt werden, insbesondere die aus dem oben beschriebenen Stand der Technik bekannten Zubereitungen für die radikalische Addition an beta-Pinen, sind daher keine Riechstoffzubereitungen im Sinne der vorliegenden Erfindung. Die gegebenenfalls in den Veröffentlichungen Zhurnal Organicheskoi Khimii 1974, 10, 1153-1157, Comptes Rendus des Seances de l'Academie des Sciences, Serie C: Sciences Chimiqes 1967, 264, 1083-1086 bzw. Bulletin de la Societe Chimique de France 1973, 4, 1368-1372 beschriebenen Mischungen, die die Verbindung der Formel (I) umfassen, sind nicht Gegenstand der vorliegenden Erfindung und sind insbesondere keine erfindungsgemäßen Riechstoffzubereitungen.

Die folgenden Mischungen bzw. Kombinationen sind daher vorzugsweise ebenfalls keine erfindungsgemäßen Riechstoffzubereitungen:
(i) eine Mischung enthaltend die Verbindung der Formel (I) wie oben definiert, Dimethylsulfoxid sowie gegebenenfalls Tosylat von (p-Menthen-1-yl-7)-2-ethanol wie in Comptes Rendus des Seances de l'Academie des Sciences, Serie C: Sciences Chimiques 1967, 264, 1083-1086 beschrieben,
(ii) eine Mischung enthaltend die Verbindung der Formel (I) wie oben definiert, die in Comptes Rendus des Seances de l'Academie des Sciences, Serie C: Sciences Chimiques 1967, 264, 1083-1086 beschriebene Verbindung der Formel (IV) (wie dort definiert) sowie gegebenenfalls beta -Pinen und gegebenenfalls Ethanal und gegebenenfalls Di-tert.-butylperoxid,
(iii) eine Mischung enthaltend die Verbindung der Formel (I) wie oben definiert, die in Bulletin de la Societe Chimique de France 1973, 4, 1368-1372 beschriebene Verbindung der Formel (4b) (wie dort definiert) und/oder die in Bulletin de la Societe Chimique de France 1973, 4, 1368-1372 beschriebene Verbindung der Formel (1b) (wie dort definiert) sowie gegebenenfalls beta -Pinen und gegebenenfalls Ethanol und gegebenenfalls Di-tert.-butylperoxid und/oder Benzoylperoxid,
(iv) eine Mischung enthaltend die Verbindung der Formel (I) wie oben definiert und Heptan, Essigsäure, Kupfer(II)-acetat, Mangan(III)-Acetat und/oder Mangan(II)-Acetat, die in Zhurnal Organicheskoi Khimii 1974, 10, 1153-1157 beschriebene Verbindung der Formel (la) (wie dort definiert) und/oder die in Zhurnal Organicheskoi Khimii 1974, 10, 1153-1157 beschriebene Verbindung der Formel (IIa) (wie dort definiert) und/oder die in Zhurnal Organicheskoi Khimii 1974, 10, 1153-1157 beschriebene Verbindung der Formel (IIIa) (wie dort definiert) sowie gegebenenfalls beta-Pinen und gegebenenfalls Ethanal.

Eine erfindungsgemäße Riechstoffzubereitung ist bevorzugt keine Mischung enthaltend
(i) die Verbindung der Formel (I) wie oben definiert und Heptan, Essigsäure, Mangan(III)-Acetat und/oder Mangan(II)-Acetat, die in Zhurnal Organicheskoi Khimii 1974, 10, 1153-1157 beschriebene Verbindung der Formel (la) (wie dort definiert) und/oder die in Zhurnal Organicheskoi Khimii 1974, 10, 1153-1157 beschriebene Verbindung der Formel (IIa) (wie dort definiert) und/oder die in Zhurnal Organicheskoi Khimii 1974, 10, 1153-1157 beschriebene Verbindung der Formel (IIIa) (wie dort definiert), sowie gegebenenfalls beta-Pinen und gegebenenfalls Ethanal. bzw.
(ii) die Verbindung der Formel (I) wie oben definiert und Heptan, Essigsäure, Kupfer(II)-acetat, Mangan(III)-acetat und/oder Mangan(II)-Acetat, sowie gegebenenfalls beta-Pinen und gegebenenfalls Ethanal. bzw.
(iii) die Verbindung der Formel (I) wie oben definiert, gegebenenfalls beta -Pinen und gegebenenfalls Ethanal und gegebenenfalls Di-tert.-butylperoxid bzw.
(iv) die Verbindung der Formel (I) wie oben definiert, die in Bulletin de la Societe Chimique de France 1973, 4, 1368-1372 beschriebene Verbindung der Formel (1 b) (wie dort definiert) sowie gegebenenfalls beta-Pinen und gegebenenfalls Ethanol und gegebenenfalls Di-tert.-butylperoxid und/oder Benzoylperoxid.

Noch weiter bevorzugt ist eine erfindungsgemäße Riechstoffzubereitung keine Mischung enthaltend
(i) die Verbindung der Formel (I) wie oben definiert und Heptan, Essigsäure, Mangan(III)-acetat und/oder Mangan(II)-Acetat sowie gegebenenfalls beta-Pinen und gegebenenfalls Ethanal bzw.
(ii) die Verbindung der Formel (I) wie oben definiert, die in Bulletin de la Societe Chimique de France 1973, 4, 1368-1372 beschriebene Verbindung der Formel (1b) (wie dort definiert) und gegebenenfalls Di-tert.-butylperoxid und/oder Benzoylperoxid.

Ganz besonders bevorzugt ist die erfindungsgemäße Riechstoffzubereitung keine Mischung enthaltend die Verbindung der Formel (I) wie oben definiert und eine oder mehrere Substanzen ausgewählt aus der Gruppe bestehend aus Heptan, Essigsäure, beta -Pinen, Ethanal, Mangansalze, Kupfersalze, Verbindung der Formel (la) aus der Veröffentlichung Zhurnal Organicheskoi Khimii 1974, 10, 1153-1157 (wie dort definiert), Verbindung der Formel (IIa) aus der Veröffentlichung Zhurnal Organicheskoi Khimii 1974, 10, 1153-1157 (wie dort definiert), Verbindung der Formel (IIIa) aus der Veröffentlichung Zhurnal Organicheskoi Khimii 1974, 10, 1153-1157 (wie dort definiert), Dimethylsulfoxid, Tosylat von (p-Menthen-1-yl-7)-2-ethanol, Di-tert.-butylperoxid, Benzoylperoxid, die in Bulletin de la Societe Chimique de France 1973, 4, 1368-1372 beschriebene Verbindung (4b) (wie dort definiert) und die in Bulletin de la Societe Chimique de France 1973, 4, 1368-1372 beschriebene Verbindung (1 b) (wie dort definiert).

Eine erfindungsgemäße Riechstoffzubereitung, insbesondere in Form eines Parfümöls, vorzugsweise mit einer Maiglöckchen-Geruchsnote, besteht aus oder umfasst
(i) Verbindung der Formel (la) wie oben definiert oder eine Mischung bestehend aus der Verbindung (la) und der Verbindung (Ib) - jeweils wie oben definiert - und
(ii) einen, 2, 3, 4, 5, 6, 7, 8, 9, 10 oder mehr weitere Riechstoffe, die keine Verbindung der Formel (I) sind.

Insbesondere durch Kombination der Verbindungen der Formel (la) und (Ib) wie oben definiert, vorzugsweise in einer der als bevorzugt angegebenen Ausgestaltungen, mit einem oder mehreren weiteren Riechstoffen (vorzugsweise mit einer, mehreren oder sämtlichen Geruchsnoten ausgewählt aus der Gruppe bestehend aus blumig, süß, wässrig, ozonig, holzig, ambriert, Moschus, fruchtig, animalisch) lassen sich interessante neue Riechstoffzubereitungen herstellen. Auf diese Weise lassen sich Mischungen mit besonders interessanten, natürlichen, neuen und originellen Noten kreieren. Riechstoffe, die im Sinne der obigen Definition als weitere Riechstoffe für den Einsatz in einer erfindungsgemäßem Riechstoffzubereitung geeignet sind, finden sich z. B. in S. Arctander, Perfume and Flavor Materials, Vol. I und II, Montclair, N. J. 1969, Eigenverlag**, oder** K. Bauer et al., Common Fragrance and Flavor Materials, 4th Edition, Wiley-VCH, Weinheim 2001**.**

Im Einzelnen seien genannt: Extrakte aus natürlichen Rohstoffen wie Etherische öle, Concretes, Absolüs, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos-Absolü; Bayöl; Beifussöl; Benzöresin; Bergamotteöl; Bienenwachs-Absolü; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolü; Castoreum-absolü; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolü; Eichenmoos-Absolü; Elemiöl; Estragonöl; Eucalyptus-citriodora-öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolü; Helichrysumöl; Ingweröl; Iriswurzel-Absolü; Iriswurzelöl; Jasmin-Absolü; Kalmusöl; Kamillenöl blau; Kamillenöl römisch; Karottensamenöl; Kaskarillaöl; Kiefernnadelöl; Krauseminzöl; Kümmelöl; Labdanumöl; Labdanum-Absolü; Labdanumresin; Lavandin-Absolü; Lavandinöl; Lavendel-Absolü; Lavendelöl; Lemongrasöl; Liebstocköl; Limetteöl destilliert; Limetteöl gepresst; Linalööl; Litsea-cubeba-öl; Lorbeerblätteröl; Macisöl; Majoranöl; Mandarinenöl; Massoirindenöl; Mimosa-Absolü; Moschuskörneröt; Moschustinktur; Muskateller-Salbei-öl; Muskatnussöl; Myrrhen-Absolü; Myrrhenöl; Myrtenöl; Nelkenblätteröl; Nelkenblütenöl; Neroliöl; Olibanum-Absolü; Olibanumöl; Opopanaxöl; Orangenblüten-Absolü; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolü; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-öl; Terpentinöl; Thymianöl; Tolubalsam; Tonka-Absolü; Tuberosen-Absolü; Vanilleextrakt; Veilchenblätter-Absolü; Verbenaöl; Vetiveröl; Wacholderbeeröl; Weinhefenöl; Wermutöl; Wintergrünöl; Ylangöl; Ysopöl; Zibet-Absolü; Zimtblätteröl; Zimtrindenöl sowie Fraktionen davon, bzw. daraus isolierten Inhaltsstoffen; Einzel-Riechstoffe aus der Gruppe der Kohlenwasserstoffe, wie z.B. 3-Caren; a-Pinen; beta -Pinen; a-Terpinen; ?-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan; der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren Acetale wie z.B. Hexanal; Heptanal; Octanal; Nonanal; Decanal; Undecanal; Dodecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxypropoxy)-(E/Z)-3-hexen;der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on; der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercapto hexylbutyrat; 3-Acetylthiohexylacetat; 1-Menthen-8-thiol; der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril; der Ester von aliphatischen Carbonsäuren wie z.B. (E)- und (Z)-3-Hexenylformiat; Ethylacetoacetat; Isoamylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadien-oat;4-Methyl-2-pentyl-crotonat;der acyclischen Terpenalkohole wie z. B. Geraniol; Nerol; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate; der acyclischen Terpenaldehyde und -ketone wie z. B. Citronellal;; 7-Methoxy-3,7-dimethyloctanal; 2,6,10-Trimethyl-9-undecenal; Geranylaceton; sowie die Dimethyl- und Diethylacetale von Geranial, Neral, der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate; der cyclischen Terpenaldehyde und -ketone wie z. B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethyl-ionon; alpha-Iron; beta-Damascenon; 1-(2,4,4-Trimethyl-2-cyclohexen-1-yl)-2-buten-1-on; 1,3,4,6,7,8a-Hexahydro-1,1,5,5-tetramethyl-2H-2,4a-methano-naphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon); der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;der cyclischen und cycloaliphatischen Ether wie z.B. Cineol; Cedrylmethylether; Cyclododecylmethylether; 1,1-Dimethoxycyclododecan; (Ethoxymethoxy)cyclo-dodecan; alpha-Cedrenepoxid; 3a,6,6,9a-Tetramethyldodecahydro-naphtho[2,1-b]furan; 3a-Ethyl-6,6,9a-trimethyldodecahydronaphtho[2,1 b]furan; 1,5,9-Tri-methyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan; der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentyl-cyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopenta-decanon; 4-(1-Ethoxyvinyl)-3,3,5,5-tetramethylcyclohexanon; 4-tert.-Pentylcyclo-hexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;der cycloaliphatischen Aldehyde wie z.B. 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton; der Ester cyclischer Alkohole wie z.B. 2-tert-Butylcyclohexylacetat; 4-tert-Butylcyclohexylacetat; 2-tert-Pentylcyclohexylacetat; 4-tert-Pentyl-cyclo-hexyl-acetat; 3,3,5-Trimethylcyclohexylacetat; Decahydro-2-naphthylacetat;2-Cyclo-pentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexa-hydro-5, bzw. 6-indenylacetat; 4,7-Methano-3a,4,5,6,7,7a-hexahydro-5, bzw. 6-indenyl-propionat; 4,7-Methano-3a,4,5,6,7,7a-hexahydro-5, bzw. 6-inden-ylisobutyrat; 4,7-Methanooctahydro-5, bzw. 6-indenylacetat; der Ester cycloaliphatischer Alkohole wie z.B.1-Cyclohexylethylcrotonat;der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;der araliphatischen Alkohole wie z.B. Benzylalkohol; 1-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenyl-propanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol; der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethyl-isoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetra-hydroindeno[1,2-d]-m-dioxin; 4,4a,5,9b-Tetrahydro-2,4-dimethylindeno[1,2-d]-m-dioxin;der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-isobutylphenyl)propanal; 3-(4-tert.-Butylphenyl)propanal; Zimtaldehyd; alpha-Butylzimtaldehyd; alpha-Hexylzimtaldehyd; 3-Methyl-5-phenylpentanal; 4-Methoxybenzaldehyd; 4-Hydroxy-3-methoxybenz-aldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal; der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-di-methyl-4-indanylmethylketon; 1-[2,3-dihydro-1,1,2,6-tetramethyl-3-(1-methylethyl) -1H-5-indenyl]ethanon; 5',6',7',8'-Tetrahydro-3',5',5',6',8',8'-hexamethyl-2-aceto-naphthon;der aromatischen und araliphatischen Carbonsäuren und deren Ester wie z.B. Benzösäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat; der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentan-säurenitril; Methylanthranilat; Methy-N-methylanthranilat; Schif'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropyl-chinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;der Phenole, Phenylether und Phenylester wie z.B. Estragol; Anethol; Eugenylmethylether; Isöugenol; Isöugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat; der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on; der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; 1,16-Hexadeca-nolid; 9-Hexadecen-1,16-olid; 10-Oxa-1,16-hexadecanolid; 11-Oxa-1,16-hexa-decanolid; 12-Oxa-1,16-hexadecanolid; Ethylen-1,12-dodecandioat; Ethylen-1,13-tridecandioat; 2,3-Dihydrocumarin; Octahydrocumarin.

In einer erfindungsgemäßen Riechstoffzubereitung, insbesondere in Form eines Parfümöls, liegt die Menge der Verbindung der Formel (la) wie oben definiert vorzugsweise im Bereich von 0,0001 bis 40 Gew.- percent liegt, vorzugsweise im Bereich von 0,001 bis 25 Gew.- percent, jeweils bezogen auf das Gesamtgewicht der Riechstoffzubereitung.

Hinsichtlich des bevorzugten Massenverhältnisse der Verbindung der Formel (la) zu der Verbindung der Formel (Ib) gilt dasselbe wie für erfindungsgemäße Mischungen umfassend die oder bestehend aus den Verbindungen der Formel (la) und der Formel (Ib) (siehe oben).

Neben den oben genannten Riechstoffen enthält eine erfindungsgemäße Riechstoffzubereitung gegebenenfalls weitere Bestandteile, die selbst keine Riechstoffe im Sinne der obenstehenden Definition sind wie z.B. Lösungsmittel, Lösungsvermittler, Emulgatoren, Stabilisatoren, Radikalfänger.

In einer bevorzugten Ausführungsform umfasst eine erfindungsgemäße Riechstoffzubereitung, insbesondere in Form eines Parfümöls, einen, 2, 3 oder mehr weitere Riechstoffe mit einer Maiglöckchen-Geruchsnote und optional einer, mehreren oder sämtlichen Geruchsnoten ausgewählt aus der Gruppe bestehend aus blumig, süß, wässrig, ozonig, holzig, ambriert, Moschus, fruchtig, animalisch. Entsprechende Riechstoffe sind dem Fachmann bekannt.

Besonders bevorzugt ist eine erfindungsgemäße Riechstoffzubereitung, in welcher
(i) die Menge der Verbindung der Formel (la) ausreicht, um der Riechstoffzubereitung eine Maiglöckchen-Geruchsnote und optional eine, mehrere oder sämtliche Geruchsnoten ausgewählt aus der Gruppe bestehend aus blumig, süss, wässrig, pudrig und ozonig zu vermitteln, und/oder
(ii) ein, mehrere oder sämtliche weiteren Riechstoffe der Riechstoffzubereitung eine Maiglöckchen-Geruchsnote und optional eine, mehrere oder sämtliche Geruchsnoten ausgewählt aus der Gruppe bestehend aus blumig, süss, wässrig, pudrig und ozonig vermitteln, wobei die Menge der Verbindung der Formel (la) ausreicht, um im Vergleich zu einer ansonsten identisch zusammengesetzten Vergleichs-Riechstoffzubereitung ohne die Verbindung der Formel (la) die Maiglöckchen-Geruchsnote und optional eine, mehrere oder sämtliche der Geruchsnoten blumig, süß, wässrig, pudrig und ozonig zu modifizieren und/oder zu verstärken, und/oder
(iii) die Menge der Verbindung der Formel (Ib) ausreicht, um der Riechstoffzubereitung eine Maiglöckchen-Geruchsnote und optional eine, mehrere oder sämtliche Geruchsnoten ausgewählt aus der Gruppe bestehend aus fruchtig, grün, wässrig und aldehydig zu vermitteln, und/oder
(iv) ein, mehrere oder sämtliche weiteren Riechstoffe der Riechstoffzubereitung eine Maiglöckchen-Geruchsnote und optional eine, mehrere oder sämtliche Geruchsnoten ausgewählt aus der Gruppe bestehend aus fruchtig, grün, wässrig und aldehydig vermitteln und die Menge der Verbindung der Formel (Ib) ausreicht, um im Vergleich zu einer ansonsten identisch zusammengesetzten Vergleichs-Riechstoffzubereitung ohne die Verbindung der Formel (Ib) die Maiglöckchen-Geruchsnote und optional eine, mehrere oder sämtliche der Geruchsnoten ausgewählt aus der Gruppe bestehend aus fruchtig, grün, wässrig und aldehydig zu modifizieren und/oder zu verstärken.

Erfindungsgemäße Riechstoffzubereitungen sind z.B. erhältlich durch ein Verfahren umfassend den Schritt des Mischens von
(i) Verbindung der Formel (la) oder einer Mischung umfassend oder bestehend aus Verbindungen der Formeln (la) und (Ib) - jeweils wie oben definiert - und
(ii) einem, 2, 3, 4, 5, 6, 7, 8, 9, 10 oder mehren weiteren Riechstoffen, die keine Verbindung der Formel (la) oder (Ib) sind,
(iii) sowie gegebenenfalls weiteren Bestandteilen, die keine Riechstoffe sind.

Die erfindungsgemäß als Riechstoff zu verwendende Verbindung der Formel (la) bzw. der erfindungsgemäßen Mischungen wie oben definiert sowie erfindungsgemäße Riechstoffzubereitungen wie oben definiert werden insbesondere zur Herstellung parfümierter Produkte (parfümierte Artikel) eingesetzt.

Dementsprechend betrifft ein weiterer Aspekt der vorliegenden Erfindung ein parfümiertes Produkt. Ein erfindungsgemäßes parfümiertes Produkt umfasst die Verbindung der Formel (la) wie oben definiert, vorzugsweise in einer sensorisch wirksamen Menge.

Dabei ist die Verbindung der Formel (la) vorzugsweise Bestandteil einer erfindungsgemäßen Mischung bzw. Riechstoffzubereitung. Ein parfümiertes Produkt umfasst daher in einer bevorzugten Ausführungsform eine erfindungsgemäße Mischung bzw. Riechstoffzubereitung, sowie einen Träger oder ein Substrat, wobei der Träger bzw. das Substrat in direktem Kontakt mit dieser Mischung bzw. Riechstoffzubereitung steht. Das Substrat ist beispielsweise ein festes bzw. der Träger ist beispielsweise ein fester Träger.

Bevorzugte erfindungsgemäße parfümierte Produkte sind Wasch- und Reinigungsmittel, Hygiene- oder Pflegeprodukte, insbesondere Produkte aus dem Bereich der Körper- und Haarpflege, der Kosmetik und des Haushalts.

Hinsichtlich bevorzugter Ausgestaltungen gelten die obigen Ausführungen entsprechend.

In diesem Zusammenhang besonders bevorzugt ist ein erfindungsgemäßes parfümiertes Produkt, in welchem die Menge der Verbindung der Formel (la) wie oben definiert im Bereich von 0,0001 bis 5 Gew.- percent liegt, vorzugsweise im Bereich von 0,001 bis 2,5 Gew.- percent, jeweils bezogen auf das Gesamtgewicht des Produkts.

Hinsichtlich des bevorzugten Massenverhältnisses der Verbindung der Formel (la) zu der Verbindung der Formel (Ib) gilt dasselbe wie für erfindungsgemäße Mischungen umfassend die oder bestehend aus den Verbindungen der Formel (la) und der Formel (Ib).

Bevorzugte erfindungsgemäße parfümierte Produkte sind ausgewählt aus der Gruppe bestehend aus:

Parfüm-Extraits, Eau de Parfums, Eau de Toilettes, Rasierwässer, Eau de Colognes, Pre-shave-Produkte, Splash-Colognes, parfümierte Erfrischungstücher, Parfüms für saure, alkalische und neutrale Reinigungsmittel, Waschmittel, Waschtabletten, Desinfektionsmitteln, sowie von Luftverbesserern, Ärosolsprays, Wachse und Polituren, sowie Körperpflegemitteln, Badeölen, kosmetischen Emulsionen, wie z.B. Hautcremes- und - lotionen, Sonnenschutzcremes- und -lotionen, After-sun-cremes und -lotionen, Handcremes und -lotionen, Fusscremes und -lotionen, Enthaarungscremes und -lotionen, After-shave-Cremes und - lotionen, Bräunungscremes und -lotionen, Haarpflegeprodukten wie z.B. Haarsprays, Haargelen, festigende Haarlotionen, Haarspülungen, Haarfärbemitteln, Haarverformungsmitteln und Haarglättungsmitteln, Haarwässern, Haarcremes und - lotionen, Deodorantien und Antiperspirantiens, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Erfindungsgemäße Riechstoffzubereitungen enthaltend die Verbindung der Formel (la) oder eine wie oben definierte erfindungsgemäße Mischung können allgemein (z.B. in konzentrierter Form, in Lösungen oder in unten beschriebener modifizierter Form) verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Ärosolsprays, Wachsen und Polituren wie Möbelpolituren, Fussbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom öl-in-Wasser-, vom Wasser-in-öl- und vom Wasser-in-öl-in-Wasser-Typ wie z.B. Hautcremes- und -lotionen, Gesichtscremes und -lotionen, Sonnenschutzcremes und - lotionen, After-sun-cremes und - lotionen, Handcremes und -lotionen, Fußcremes und - lotionen, Enthaarungscremes und - lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und - lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Bevorzugte erfindungsgemäße parfümiertes Produkte sind Produkte ausgewählt aus der Gruppe bestehend aus Wasch- und Reinigungsmitteln, Hygiene- oder Pflegeprodukten, insbesondere aus dem Bereich der Körper- und Haarpflege, der Kosmetik und des Haushalt, Fine Fragrance (Parfüm), Air Care (insbesondere Kerzen), Lufterfrischer.

Die erfindungsgemäßen zuvor genannten Riechstoffzubereitungen bzw. die erfindungsgemäß in den entsprechenden Produkten einzusetzenden Riechstoffzubereitungen können in flüssiger Form, unverdünnt oder mit einem Lösungsmittel verdünnt für Parfümierungen eingesetzt werden. Geeignete Lösungsmittel hierfür sind z.B. Ethanol, Isopropanol, Diethylenglycolmonöthylether, Glycerin, Propylenglycol, 1,2-Butylenglycol, Dipropylenglycol, Diethylphthalat, Triethylcitrat, Isopropylmyristat usw. Für die explizit genannten Lösungsmittel gilt, dass diese im Rahmen des vorliegenden Textes im Falle des Vorhandenseins eigener olfaktorischer Eigenschaften ausschließlich dem Bestandteil "Lösungsmittel" und nicht den "Riechstoffen" zuzuordnen sind.

Die in den erfindungsgemäßen parfümierten Produkten enthaltene Verbindung der Formel (I), eine wie oben definierte erfindungsgemäße Mischung oder eine wie oben definierte erfindungsgemäße Riechstoffzubereitung können dabei in einer bevorzugten Ausführungsform an einem Trägerstoff absorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die in den erfindungsgemäßen parfümierten Produkten enthaltene Verbindung der Formel (la), eine wie oben definierte erfindungsgemäße Mischung oder eine wie oben definierte erfindungsgemäße Riechstoffzubereitung können auch mikroverkapselt, sprühgetrocknet, als Einschluss-Komplexe oder als Extrusions-Produkte vorliegen und in dieser Form dem zu parfümierenden Produkt, bzw. Artikel, hinzugefügt werden.

Gegebenenfalls können die Eigenschaften der derart modifizierten Riechstoffzubereitungen durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Riechstoffzubereitungen kann beispielsweise durch das sogenannte Koazervationsverfahren mit Hilfe von Kapselmaterialien, z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riechstoffzubereitung und Cyclodextrinen oder Harnstoffderivaten in ein geeignetes Lösungsmittel, z.B. Wasser, hergestellt werden. Extrusions-Produkte können durch Verschmelzen der Riechstoffzubereitung mit einem geeigneten wachsartigen Stoff und durch Extrusion mit nachfolgender Erstarrung, ggf. in einem geeigneten Lösungsmittel, z.B. Isopropanol, erfolgen.

Die erfindungsgemäßen Riechstoffzubereitungen können demnach, wie bereits erwähnt, in konzentrierter Form, in Lösungen oder in oben beschriebener modifizierter Form für die Herstellung der entsprechenden erfindungsgemäßen parfümierten Artikel verwendet werden

Zutaten, mit denen die erfindungsgemäße Verbindung der Formel (I), eine wie oben definierte erfindungsgemäße Mischung oder eine wie oben definierte erfindungsgemäße Riechstoffzubereitung vorzugsweise kombiniert werden kann, sind beispielsweise: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Polyole, Tenside, Elektrolyte, organische Lösungsmittel oder Silikonderivate.

Mischungen, wie sie in der präparativen organischen Chemie eingesetzt werden, insbesondere die aus dem oben beschriebenen Stand der Technik bekannten Zubereitungen für die radikalische Addition an beta-Pinen, sind keine parfümierten Produkte im Sinne der vorliegenden Erfindung. Die gegebenenfalls in den Veröffentlichungen Zhurnal Organicheskoi Khimii 1974, 10, 1153-1157, Comptes Rendus des Seances de l'Academie des Sciences, Serie C: Sciences Chimiques 1967, 264, 1083-1086 bzw. Bulletin de la Societe Chimique de France 1973, 4, 1368-1372 beschriebenen Mischungen, die die Verbindung der Formel (I) umfassen, sind nicht Gegenstand der vorliegenden Erfindung und sind insbesondere keine erfindungsgemäßen parfümierten Produkte.

Die folgenden Mischungen bzw. Kombinationen sind daher vorzugsweise ebenfalls keine erfindungsgemäßen parfümierten Produkte:
(i) eine Mischung enthaltend die Verbindung der Formel (la) bzw. (Ib) - jeweils wie oben definiert, Dimethylsulfoxid sowie gegebenenfalls Tosylat von (p-Menthen-1-yl-7)-2-ethanol wie in Comptes Rendus des Seances de l'Academie des Sciences, Serie C: Sciences Chimiques 1967, 264, 1083-1086 beschrieben,
(ii) eine Mischung enthaltend die Verbindung der Formel Formel (la) bzw. (Ib) - jeweils wie oben definiert - die in Comptes Rendus des Seances de l'Academie des Sciences, Serie C: Sciences Chimiques 1967, 264, 1083-1086 beschriebene Verbindung der Formel (IV) (wie dort definiert) sowie gegebenenfalls beta -Pinen und gegebenenfalls Ethanal und gegebenenfalls Di-tert.-butylperoxid,
(iii) eine Mischung enthaltend die Verbindung der Formel (la) bzw. (Ib) - jeweils wie oben definiert - die in Bulletin de la Societe Chimique de France 1973, 4, 1368-1372 beschriebene Verbindung der Formel (4b) (wie dort definiert) und/oder die in Bulletin de la Societe Chimique de France 1973, 4, 1368-1372 beschriebene Verbindung der Formel (1 b) (wie dort definiert) sowie gegebenenfalls beta -Pinen und gegebenenfalls Ethanol und gegebenenfalls Di-tert.-butylperoxid und/oder Benzoylperoxid,
(iv) eine Mischung enthaltend die Verbindung der Formel (la) bzw. (Ib) - jeweils wie oben definiert - und Heptan, Essigsäure, Kupfer(II)-acetat, Mangan(III)-Acetat und/oder Mangan(II)-Acetat, die in Zhurnal Organicheskoi Khimii 1974, 10, 1153-1157 beschriebene Verbindung der Formel (la) (wie dort definiert) und/oder die in Zhurnal Organicheskoi Khimii 1974, 10, 1153-1157 beschriebene Verbindung der Formel (IIa) (wie dort definiert) und/oder die in Zhurnal Organicheskoi Khimii 1974, 10, 1153-1157 beschriebene Verbindung der Formel (IIIa) (wie dort definiert) sowie gegebenenfalls beta-Pinen und gegebenenfalls Ethanal.

Ein erfindungsgemäßes parfümiertes Produkt ist bevorzugt keine Mischung enthaltend
(i) die Verbindung der Formel (la) bzw. (Ib) - jeweils wie oben definiert - und Heptan, Essigsäure, Mangan(III)-Acetat und/oder Mangan(II)-Acetat, die in Zhurnal Organicheskoi Khimii 1974, 10, 1153-1157 beschriebene Verbindung der Formel (la) (wie dort definiert) und/oder die in Zhurnal Organicheskoi Khimii 1974, 10, 1153-1157 beschriebene Verbindung der Formel (IIa) (wie dort definiert) und/oder die in Zhurnal Organicheskoi Khimii 1974, 10, 1153-1157 beschriebene Verbindung der Formel (IIIa) (wie dort definiert), sowie gegebenenfalls beta-Pinen und gegebenenfalls Ethanal bzw.
(ii) die Verbindung der Formel (la) bzw. (Ib) - jeweils wie oben definiert - und Heptan, Essigsäure, Kupfer(II)-acetat, Mangan(III)-acetat und/oder Mangan(II)-Acetat, sowie gegebenenfalls beta-Pinen und gegebenenfalls Ethanal bzw.
(iii) die Verbindung der Formel (la) bzw. (Ib) - jeweils wie oben definiert - gegebenenfalls beta -Pinen und gegebenenfalls Ethanal und gegebenenfalls Di-tert.-butylperoxid bzw.
(iv) die Verbindung der Formel (la) bzw. (Ib) - jeweils wie oben definiert - die in Bulletin de la Societe Chimique de France 1973, 4, 1368-1372 beschriebene Verbindung der Formel (1b) (wie dort definiert) sowie gegebenenfalls beta -Pinen und gegebenenfalls Ethanol und gegebenenfalls Di-tert.-butylperoxid und/oder Benzoylperoxid. Noch weiter bevorzugt ist ein erfindungsgemäßes parfümiertes Produkt keine Mischung enthaltend
(v) die Verbindung der Formel (la) bzw. (Ib) - jeweils wie oben definiert - und Heptan, Essigsäure, Mangan(III)-acetat und/oder Mangan(II)-Acetat sowie gegebenenfalls beta-Pinen und gegebenenfalls Ethanal bzw.
(vi) die Verbindung der Formel (la) bzw. (Ib) - jeweils wie oben definiert - die in Bulletin de la Societe Chimique de France 1973, 4, 1368-1372 beschriebene Verbindung der Formel (1b) (wie dort definiert) und gegebenenfalls Di-tert.-butylperoxid und/oder Benzoylperoxid.

Ganz besonders bevorzugt ist das erfindungsgemäße parfümierte Produkt keine Mischung enthaltend die Verbindung der Formel (la) wie oben definiert und eine oder mehrere Substanzen ausgewählt aus der Gruppe bestehend aus Heptan, Essigsäure, beta-Pinen, Ethanal, Mangansalze, Kupfersalze, Verbindung der Formel (la) aus der Veröffentlichung Zhurnal Organicheskoi Khimii 1974, 10, 1153-1157 (wie dort definiert), Verbindung der Formel (IIa) aus der Veröffentlichung Zhurnal Organicheskoi Khimii 1974, 10, 1153-1157 (wie dort definiert), Verbindung der Formel (IIIa) aus der Veröffentlichung Zhurnal Organicheskoi Khimii 1974, 10, 1153-1157 (wie dort definiert), Dimethylsulfoxid, Tosylat von (p-Menthen-1-yl-7)-2-ethanol, Di-tert.-butylperoxid, Benzoylperoxid, die in Bulletin de la Societe Chimique de France 1973, 4, 1368-1372 beschriebene Verbindung (4b) (wie dort definiert) und die in Bulletin de la Societe Chimique de France 1973, 4, 1368-1372 beschriebene Verbindung (1 b) (wie dort definiert).

Ein weiterer Aspekt der vorliegenden Erfindung betrifft ein Verfahren zum Vermitteln, Verstärken und/oder Modifizieren von Geruchsnoten, insbesondere einer Maiglöckchen-Geruchsnote. Ein erfindungsgemäßes Verfahren zum Vermitteln, Verstärken und/oder Modifizieren von Geruchsnoten, insbesondere einer Maiglöckchen-Geruchsnote und optional einer, mehrerer oder sämtlicher Geruchsnoten ausgewählt aus der Gruppe bestehend aus blumig, süß, wässrig, pudrig und ozonig umfassend den folgenden Schritt:
Mischen oder in Kontakt bringen
   (i) der Verbindung der Formel (la) wie oben definiert, oder
   (ii) einer erfindungsgemäßen Mischung wie oben definiert, oder
   (iii) einer erfindungsgemäßen Riechstoffzubereitung wie oben definiert vorzugsweise in einer sensorisch wirksamen Menge mit einem Erzeugnis.

Hinsichtlich bevorzugter Ausgestaltungen gelten die obigen Ausführungen entsprechend.

Das vorstehend-beschriebene erfindungsgemäße Verfahren ist auch geeignet im Rahmen der Herstellung eines erfindungsgemäßen parfümierten Produkts, wobei als Ergebnis des Schrittes Mischen oder in Kontakt bringen
(i) der Verbindung der Formel (la) wie oben definiert, oder
(ii) einer erfindungsgemäßen Mischung wie oben definiert, oder
(iii) einer erfindungsgemäßen Riechstoffzubereitung wie oben definiert vorzugsweise in einer sensorisch wirksamen Menge mit einem Erzeugnis das erfindungsgemäße parfümierte Produkt gebildet wird. Unter "Erzeugnis" ist in diesem Fall die Gesamtheit der Bestandteile des erfindungsgemäßen parfümierten Produkts zu verstehen mit Ausnahme der eingesetzten Verbindung der Formel (I), der eingesetzten erfindungsgemäßen Mischung bzw. der eingesetzten erfindungsgemäßen Riechstoffzubereitung.

Bei dem erfindungsgemäßen Verfahren erweist sich die hohe Substantivität der erfindungsgemäßen Verbindungen (la) und (Ib) als vorteilhaft, denn je höher die Substantivität eines Riechstoffs, desto geringer ist die zum Aufrechterhalten eines Geruchs über eine bestimmte Zeit benötigte Einsatzmenge des Riechstoffs.

Ein weiterer Aspekt der vorliegenden Erfindung betrifft ein Verfahren zum Versehen von Haut, Haaren, Oberflächen oder textilen Fasern mit einer Geruchsnote, insbesondere einer Maiglöckchen-Geruchsnote. Ein erfindungsgemäßes Verfahren zum Versehen von Haut, Haaren, Oberflächen oder textilen Fasern mit einer Geruchsnote, insbesondere einer Maiglöckchen-Geruchsnote umfasst folgende Schritte:
(a) Bereitstellen einer Riechstoffzubereitung umfassend
(b) Verbindung der Formel (Ia) oder einer Mischung der Verbindungen (Ia) und (Ib) - jeweils wie in oben definiert - und
(c) einen oder mehrere weitere Riechstoffe, die keine Verbindung der Formel (la) oder (Ib) sind, wobei in der Riechstoffzubereitung die Menge der Verbindung der Formel (la) bzw. der Mischung der Verbindungen (la) und (Ib) ausreicht, um eine Maiglöckchen-Geruchsnote zu vermitteln, zu modifizieren und/oder zu verstärken;
(d) Applizieren der Riechstoffzubereitung auf Haut, Haare oder textile Fasern.

Dabei kann die Riechstoffzubereitung Bestandteil eines erfindungsgemäßen parfümierten Produkts sein, beispielsweise eines Körper-, Haar- oder Textilpflegeprodukts.

Hinsichtlich bevorzugter Ausgestaltungen gelten die obigen Ausführungen entsprechend.

Die erfindungsgemäß als Riechstoff zu verwendende Verbindung der Formel (I) ist u.a. erhältlich durch radikalische Addition von Ethanal an (+)-beta-Pinen. Die erfindungsgemäßen Verbindungen ausgewählt aus der Gruppe bestehend aus der Verbindung der Formel (la) und der Verbindung der Formel (Ib) sind u.a. erhältlich durch radikalische Addition von Ethanal an (+)-beta -Pinen bzw. (-)- beta-Pinen.

Eine weitere Aufgabe der vorliegenden Erfindung bestand darin, neue Verfahren anzugeben zur Herstellung der erfindungsgemäß als Riechstoff zu verwendenden Verbindung (Ia).

Ein erfindungsgemäßes Verfahren zum Herstellen der Verbindung der Formel (la) wie oben definiert umfasst den Schritt säurekatalysiertes Umsetzen eines Acetals der Formel (IIa), so dass die Verbindung der Formel (la) gebildet wird 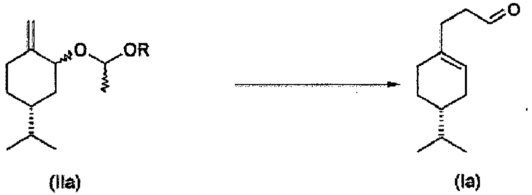 wobei die Gruppe R in dem Acetal der Formel (IIa) eine verzweigte oder unverzweigte, gesättigte oder ungesättigte Alkylgruppe mit 1 bis 10 Kohlenstoff-Atomen ist, bevorzugt eine Butylgruppe.

Das in dem erfindungsgemäßen Verfahren eingesetzte Acetal der Formel (IIa) 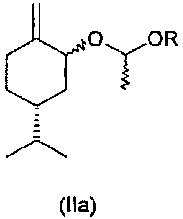 wobei die Gruppe R in dem Acetal der Formel (IIa) eine verzweigte oder unverzweigte, gesättigte oder ungesättigte Alkylgruppe mit 1 bis 10 Kohlenstoff-Atomen ist, bevorzugt eine Butylgruppe, ist neu und ebenfalls Gegenstand der vorliegenden Erfindung. Dabei umfasst die Bezeichnung "Acetal der Formel (IIa)" sowohl die einzelnen Enantiomere (Acetal der Formel (IIa) wie weiter unten definiert und Acetal der Formel (IIb) wie weiter unten definiert) als auch sämtliche Mischungen (Enantiomerenmischungen) bestehend aus diesen beiden Verbindungen (in jedem beliebigen Mischungsverhältnis). D.h., Ausführungen betreffend das "Acetal der Formel (II)" gelten stets sowohl für das Acetal der Formel (IIa) und das Acetal der Formel (IIb) als auch für Mischungen bestehend aus dem Acetal der Formel (IIa) und dem Acetal der Formel (IIb) in jedem beliebigen Mischungsverhältnis.

Das Acetal der Formel (IIa) ist auf verschiedenen Wegen (siehe unten) erhältlich, wobei das Edukt jeweils vorzugsweise d-(+)-Limonen ist. Dabei bestimmt die Stereochemie des eingesetzten Edukts die Stereochemie der erhaltenen Verbindung der Formel (I). Wird d-(+)-Limonen als Edukt eingesetzt, so resultiert die Verbindung der Formel (la), wird I-(-)-Limonen als Edukt eingesetzt, so resultiert die Verbindung der Formel (Ib). Ein weiterer Gegenstand der vorliegenden Erfindung ist somit die Verwendung
(I) von d-(+) Limonen als Edukt zur Herstellung der Verbindung der Formel (la) wie oben definiert,
(II) einer Mischung enthaltend d-(+) Limonen und I-(-) Limonen als Edukt zur Herstellung einer Mischung enthaltend die Verbindung der Formel (la) wie oben definiert und die Verbindung der Formel (Ib) wie oben definiert.

Der Vorteil der erfindungsgemäßen Verwendung von d-(+)-Limonen und/oder I-(-)-Limonen als Edukt besteht darin, dass es sich dabei um leicht zugängliche, d.h. im -Handel erhältliche, und preiswerte Edukte aus nachwachsenden Rohstoffen handelt. Dabei sind durch den selektiven Einsatz von d-(+) bzw. I-(-)-Limonen die Verbindung der Formel (la) bzw. der Formel (Ib) jeweils selektiv zugänglich.

Zwei erfindungsgemäße Synthesewege, die jeweils ausgehend von Limonen zu der Verbindung der Formel (la) bzw. (Ib) führen, werden nachstehend beschrieben.

### Syntheseweg via Epoxidierung von hydriertem d- bzw. I-Limonen

Das Edukt Limonen (VI) (d-(+)-Limonen und/oder I-(-)-Limonen) wird zunächst an sich bekannter Weise katalytisch zu p-Menth-1-en (Formel (V)) monohydriert. Dieses wird mit einer Persäure in an sich bekannter Weise epoxidiert. Das erhaltene Epoxid der Formel (IV) wird in Gegenwart von Aluminiumisopropylat erhitzt, so dass eine Ringöffnung zum Allylalkohol der Formel (III) erfolgt. Durch Reaktion des Allylalkohols der Formel (III) mit einem Alkylvinylether wie z.B. Butylvinylether in Gegenwart einer protischen Säure wie z.B. Phosphorsäure wird das Acetal der Formel (II) hergestellt. Anschliessend wird das Acetal der Formel (II) mit katalytischen Mengen an Säure wie z.B. Hexansäure in einem hochsiedende Lösungsmittel wie z.B. Malotherm S über das Zwischenprodukt 4-Isopropyl-1-methylen-2-vinyloxy-cyclohexan zu der erfindungsgemäß zu verwendenden Verbindung der Formel (I) umgesetzt (siehe unten Reaktionsschema (i)).

### Reaktionsschema (i) ausgehend von d-(+)-Limonen

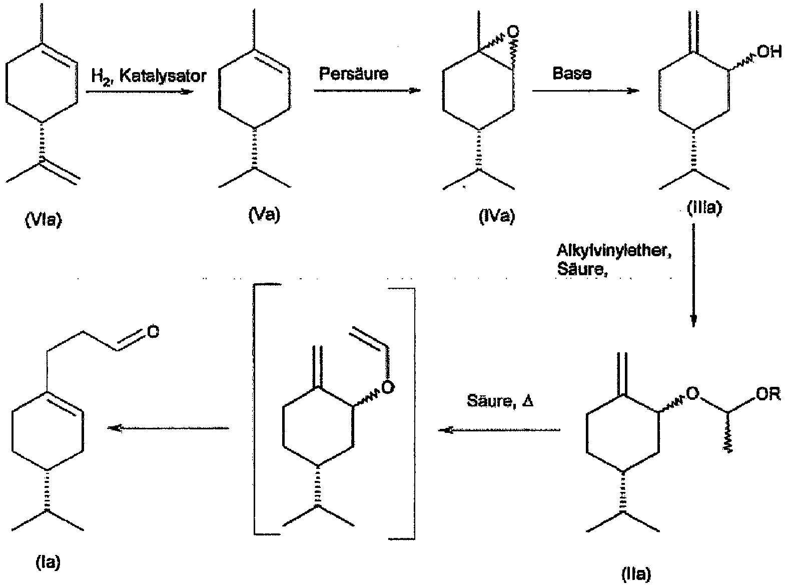

### Reaktionsschema ausgehend von I-(-)-Limonen

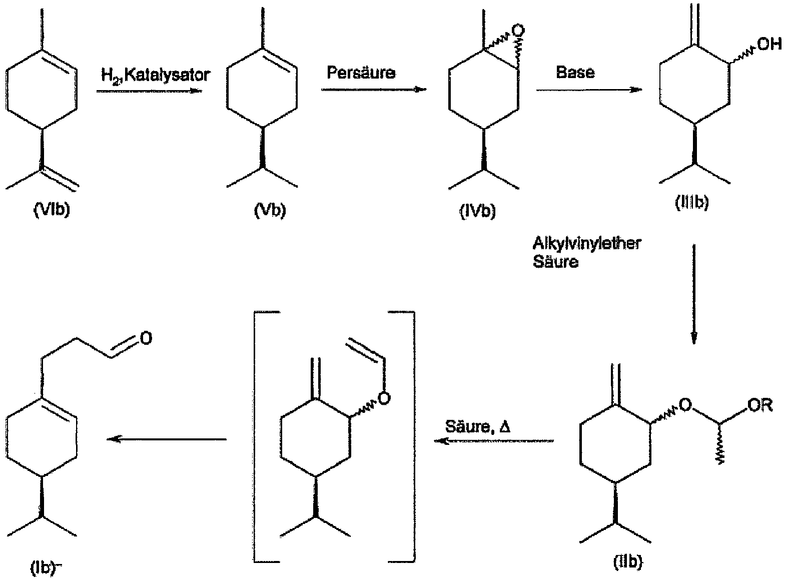

Die erste Stufe des erfindungsgemäßen Synthesewegs (i), die selektive Hydrierung der exozyklischen Doppelbindung von Limonen (Formel (VIa), (VIb)), wurde oft in der Literatur beschrieben. Typische Reagenzien für diese Reaktion sind Platin(IV)oxid wie z.B. in der Publikation J. Chem. Soc., Chem Commun. 1994, 24, 2759-2760 oder W-4 Raney-Nickel wie in J. Org. Chem. 1982, 10, 1824-1831 publiziert. Allerdings ist die Wervendung von Ruthenium auf Kohle leichter zu handeln als das brennbare Raney-Nickel und preiswerter als Platin(IV)oxid.

Die Epoxidierung von *p*-Menth-1-en (Formel (Va), (Vb)) wird mit Persäuren wie Perbenzösäure wie z.B. in der Veröffentlichung Helv. Chim. Acta 1984, 67, 1248-1253 vorgenommen, vorzugsweise jedoch mit Peressigsäure in Dichlormethan, Chloroform oder Benzen nach den üblichen, in der Literatur, z.B. in der JACS 1981, 103, 1813-1821, beschriebenen Verfahren, durchgeführt. Das Lösungsmittel Benzol wird aufgrund seiner Toxizität mit Toluol ersetzt.

Die nächste Stufe ist die selektive Ringöffnung des Epoxids der Formel (IVa), (VIb) zum Allylalkohol der Formel (IIIa), (IIIb). Bie dieser Reaktion können verschiedene Produkte entstehen, nämlich der gewünschte Allylalkohol mit der exocyclischen Doppelbindung und der entsprechende Allylalkohol mit endocyclicher Doppelbindung. In der Literatur werden für diese Reaktion als Base u.a. Lithiumdiisopropylamid oder *tert*Butanolat beschrieben [Roczniki Chemii Ann. Soc. Chim. Polonorum 1976, 50, 1709-1717 und Helv. Chim. Acta 1984, 67, 1249-1253]. Nachteilig bei allen diesen Verfahren ist, dass relativ hohe äquivalentmengen an Base eingesetzt werden müssen, sowie die teilweise schwierige Handhabbarkeit von Basen wie z.B. Lithiumdiisopropylamid und die geringe Selktivität und/oder Ausbeute. Beispeilweise entsteht bei Verwendung von *tert*Butanolat in Pyridin unter in der obigen Literatur angegebenen Bedingungen eine Mischung aus Produkt (65 percent), Edukt (28 percent) und einer unbekannten Nebenverbindung (7 percent).

In Zusammenhang mit der vorliegenden Erfindung war daher ein Verfahren bereitzustellen, bei dem der Allylalkohol der Formel (IIIa), (IIIb) ausgehend von dem Epoxid der Formel (IVa), (IVb) mit hoher Selektivität und Ausbeute erhältlich ist. Es wurde gefunden, dass dies unerwarteterweise möglich ist, wenn man die Reaktion mit katalytischen Mengen von Aluminiumisopropylat in Xylol durchführt, analog zu der Herstellung von Pinocarveol in DE 31 432 27 und Methylcyclododecatri-2,5,9-en-1-ol in US 4,853,368. Durch Erhitzen des Epoxidierungsproduktes in Gegenwart einer katalytischen Menge von Aluminiumisopropylat wird eine selektive Öffnung des Epoxyringes durch Umlagerung erreicht.

Die weitere Umsetzung wird dann in zwei Stufen durchgeführt, wie z.B. in US 3,716,498 für die Herstellung von Pinoacetaldehyd erläutert. Zunächst wird durch Reaktion des Allylalkohols der Formel (IIIa), (IIIb) mit einem Alkylvinylether wie z.B. Butylvinylether in Gegenwart einer protischen Säure wie z.B. Phosphorsäure das Acetal der Formel (IIa), (IIb) hergestellt. Anschliessend wird das Acetal der Formel (IIa), (IIb) mit katalytischen Mengen an Säure wie z.B. Hexansäure in einem hochsiedende Lösungsmittel wie z.B. Malotherm S über das Zwischenprodukt 4-Isopropyl-1-methylen-2-vinyloxy-cyclohexan zu der erfindungsgemäß zu verwendenden Verbindung der Formel (la), (Ib) umgesetzt. Alternativ kann man das Zwischenprodukt 4-Isopropyl-1-methylen-2-vinyloxy-cyclohexan, wie dem Fachmann bekannt ist, aus dem Allylalkohol der Formel (IIIa) bzw. (IIIb) und Acetylen gezielt herstellen.

Ein erfindungsgemäß bevorzugtes Verfahren zum Herstellen der Verbindung der Formel (la) wie oben und/oder der Verbindung der Formel (Ib) wie oben definiert umfasst demnach folgende Schritte
(a) Bereitstellen von d-(+) Limonen (VIa) und/oder I-(-) Limonen (Vlb) 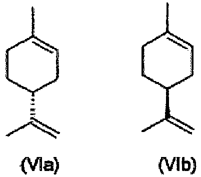 als Edukt;
(b) Hydrieren des bereitgestellten Edukts (VIa, Vlb), so dass ausgehend von
   (b1) d-(+) Limonen (VIa) die Verbindung der Formel (Va) gebildet wird; 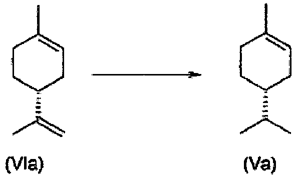
   (b2) I-(-) Limonen (Vlb) die Verbindung der Formel (Vb) gebildet wird, 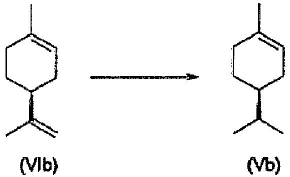
(c) Epoxidieren der durch Hydrieren des Edukts (VIa, Vlb) gebildeten Verbindung (Va, Vb), so dass ausgehend von
   (c1) der Verbindung der Formel (Va) das Epoxid der Formel (IVa) gebildet wird, 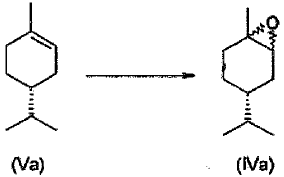
   (c2) der Verbindung der Formel (Vb) das Epoxid der Formel (IVb) gebildet wird, 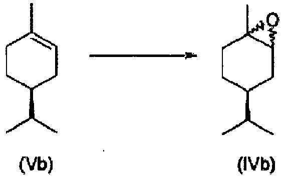
(d) Ringöffnung des durch Epoxidieren der Verbindung (Va, Vb) gebildeten Epoxids (IVa, IVb), so dass ausgehend von
   (d1) dem Epoxid der Formel (IVa) der Allylalkohol der Formel (IIIa) gebildet wird, 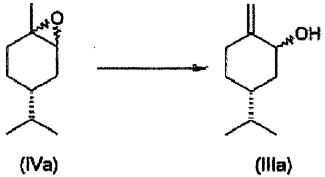
   (d2) dem Epoxid der Formel (IVb) der Allylalkohol der Formel (IIIb) gebildet wird, 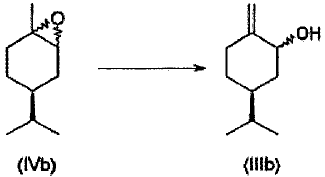
(e) Addition der bei der Ringöffnung des Epoxids (IVa, IVb) gebildeten Allylalkohols (IIIa, IIIb) an einen Alkylvinylether, vorzugsweise Butylvinylether, so dass ausgehend von
   (e1) dem Allylalkohol der Formel (IIIa) das Acetal der Formel (IIa) gebildet wird, 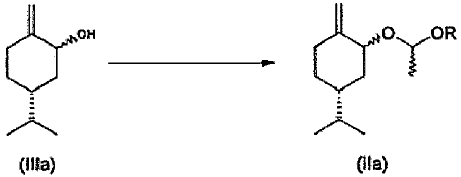
   (e2) dem Allylalkohol der Formel (IIIb) das Acetal der Formel (IIb) gebildet wird, 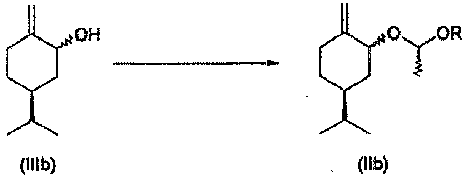
(f) Säurekatalysierte Umsetzung der bei der Addition des Allylalkohols (IIIa, IIIb) an einen Alkylvinylether, vorzugsweise Butylvinylether gebildeten Acetals (IIa, IIb) so dass ausgehend von
   (f1) dem Acetal der Formel (IIa) die Verbindung der Formel (la) gebildet wird, 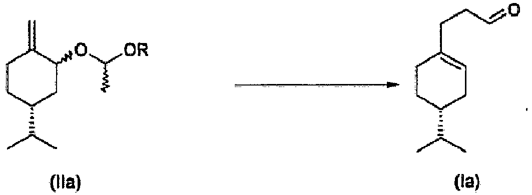
   (f2) dem Acetal der Formel (IIb) die Verbindung der Formel (Ib) gebildet wird.

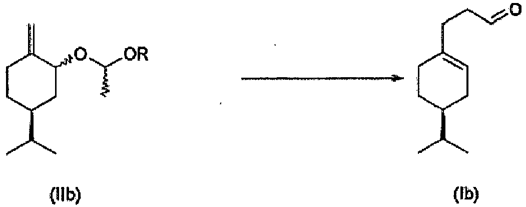

### Syntheseweg via Photooxidation von hydriertem d-(+)- bzw. I-(-)-Limonen

Für diesen Syntheseweg (siehe nachfolgendes Reaktionsschema (ii)) wird Limonen (Formel (VIa), (VIb)) zunächst wie oben beschrieben zu p-Menth-1-en (Formel (Va), (Vb)) hydriert. p-Menth-1-en (Formel (Va), (Vb)) wird dann in einem Lösungsmittel, z.B. Methanol, in Gegenwart eines Sensibilisators, z.B. Bengalrosa bei Raumtemperatur einer Photooxidation unterzogen und die dabei gebildeten Hydroperoxyde (im nachfolgenden Schema nicht dargestellt) werden mit einem geeigneten Reduktionsmittel, z.B. Natriumsulfit zu den entsprechenden isomeren Allyalkoholen (Formeln (IIIa), (IIIa'), (III'a'), (IIIb), (IIIb'), (IIIs")) reduziert. Dann wird wie oben für Syntheseweg (i) beschrieben durch Reaktion des Allylalkohols der Formel (IIIa), (IIIb) mit einem Alkylvinylether wie z.B. Butylvinylether in Gegenwart einer protischen Säure wie z.B. Phosphorsäure das Acetal der Formel (IIa), (IIb) hergestellt. Anschliessend wird das Acetal der Formel (IIa), (IIb) mit katalytischen Mengen an Säure wie z.B. Hexansäure in einem hochsiedende Lösungsmittel wie z.B. Malotherm S über das Zwischenprodukt 4-Isopropyl-1-methylen-2-vinyloxy-cyclohexan zu der erfindungsgemäß zu verwendenden Verbindung der Formel (Ia), (Ib) umgesetzt.

### Reaktionsschema (ii) ausgehend von d-(+)-Limonen

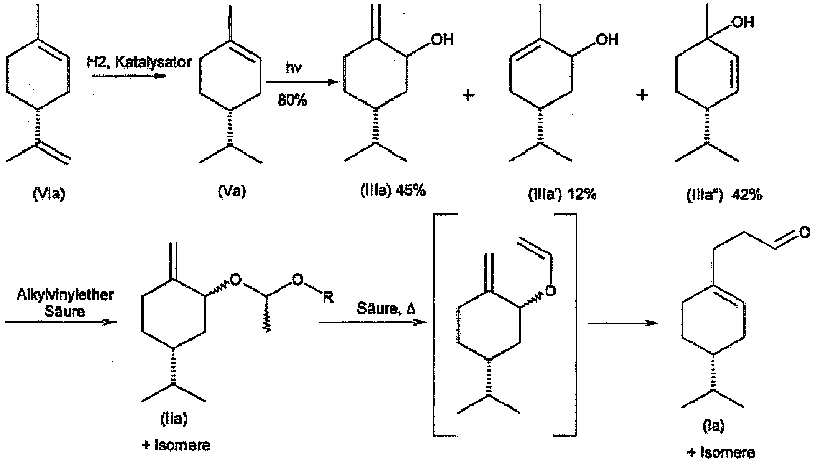

### Reaktionsschema ausgehend von I-(-)-Limonen

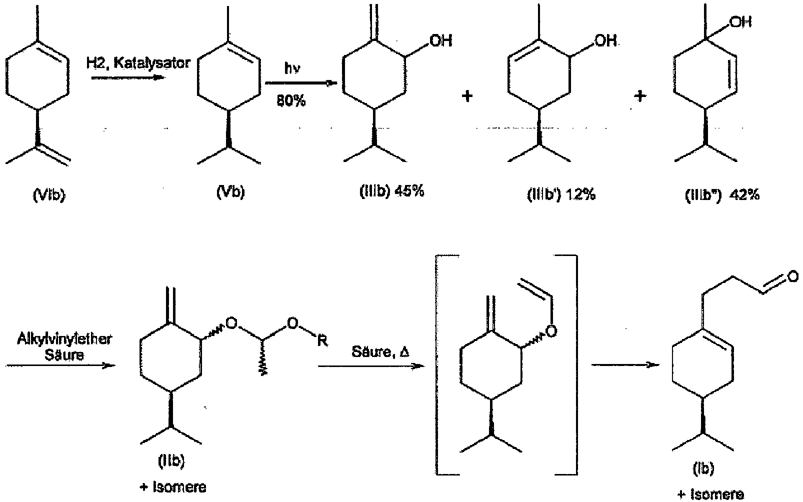

Ein auf dem obigen Reaktionsschema (ii) basierendes Verfahren zum Herstellen der Verbindung der Formel (la) wie oben definiert und/oder der Verbindung der Formel (Ib) wie oben definiert umfasst demnach die folgenden Schritte:
(a) Bereitstellen von d-(+) Limonen (Via) und/oder I-(-) Limonen (VIb) 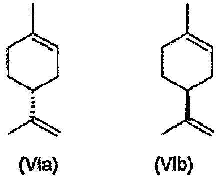 als Edukt
(b) Hydrieren des bereitgestellten Edukts (VIa, Vlb), so dass ausgehend von
   (b1) d-(+) Limonen (VIa) die Verbindung der Formel (Va) gebildet wird, 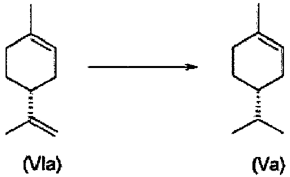
   (b2) I-(-) Limonen (Vlb) die Verbindung der Formel (Vb) gebildet wird, 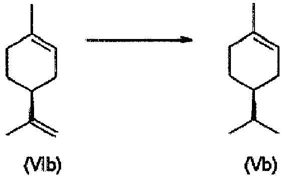
(c) Photooxidation der durch Hydrierung des Edukts (VIa, VIb) gebildeten Verbindung (Va, Vb) und Reduktion der dabei entstandenen Hydroperoxide, so dass ausgehend von
   (c1) der Verbindung (Va) der Allylalkohol (IIIa) und dessen Isomere (IIIa', IIIa") gebildet werden 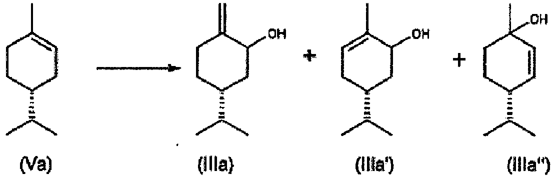
   (c2) der Verbindung (Vb) der Allylalkohol (IIIb) und dessen Isomere (IIIb', IIIb") gebildet werden 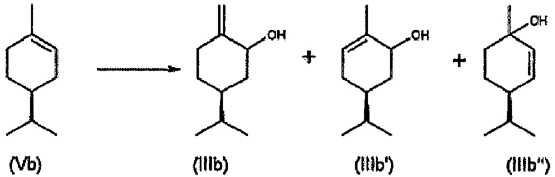
(d) Addition der bei der Photooxidation des Verbindung (Va, Vb) gebildeten Allylalkohols (IIIa, IIIb) an einen Alkylvinylether, vorzugsweise Butylvinylether, so dass ausgehend von
   (d1) dem Allylalkohol der Formel (IIIa) das Acetal der Formel (IIa) gebildet wird, 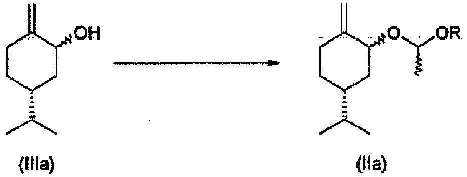
   (d2) dem Allylalkohol der Formel (IIIb) das Acetal der Formel (IIb) gebildet wird, 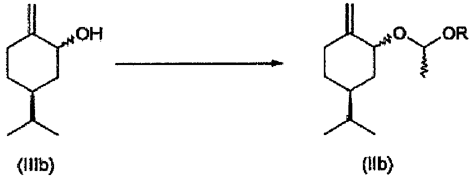
(e) Säurekatalysierte Umsetzung des bei der Addition des Allylalkohols (IIIa, IIIb) an einen Alkylvinylether, vorzugsweise Butylvinylether, gebildeten Acetals (IIa, IIb), so dass ausgehend von
   (e1) dem Acetal der Formel (IIa) die Verbindung der Formel (la) gebildet wird, 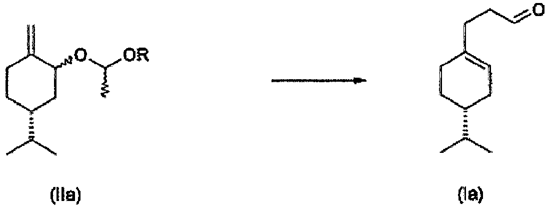
   (e2) dem Acetal der Formel (IIb) die Verbindung der Formel (Ib) gebildet wird 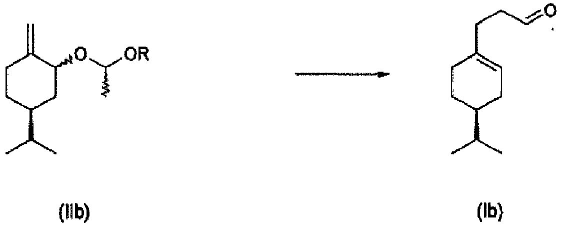
(f) Optional Abtrennen der Verbindung der Formel (la) und/oder der Formel (Ib) von den Isomeren.

Dieses Verfahren ist wegen der Notwendigkeit, die bei der Photooxidation und anschließenden Reduktion gebildeten isomeren Allylalkohole der Formeln (III') und (III") bzw. deren Folgeprodukte abzutrennen, erfindungsgemäß etwas weniger bevorzugt.

Ein alternatives erfindungsgemäßes Verfahren zum Herstellen der Verbindung der Formel (I) wie oben definiert umfasst den Schritt Pyrolysieren des Spirolactons der Formel (VIII) (8-Isopropyl-1-oxaspiro[4.5]decan-2-on) unter Bildung der Verbindung der Formel (I)

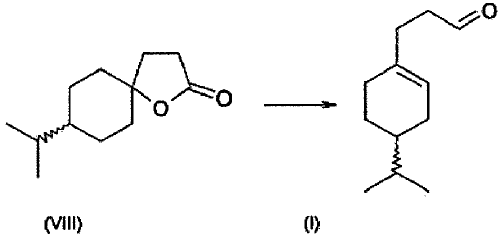

bevorzugt in Gegenwart eines Cerium enthaltenden Katalysators, insbesondere Cerium(III)acetat-Hydrat.

8-Isopropyl-1-oxaspiro[4.5]decan-2-on ist durch eine radikalische Reaktion von 4-Isopropylcyclohexanol (Formel (IX)) mit Acrylsäuremethylester in Gegenwart von Ditert-butylperoxid erhältlich. 4-Isopropylcyclohexanol (Formel (IX)) wiederum ist durch Hydrierung von 4-Isopropylphenol (Formel (X)) erhältlich:

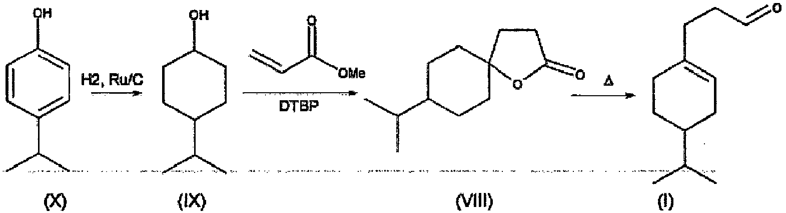

Die Herstellung des 8-Isopropyl-1-oxaspiro[4.5]decan-2-on wird in EP 0 105 157 beschrieben. Dieses Dokument offenbart jedoch nicht die Pyrolyse derartiger Spirolactone; Spirolactone sind das Zielprodukt des in EP 0 105 157 beschriebenen Verfahrens.

Das Dokument WO 2004/089861 A1 beschreibt die Pyrolyse eines Spirolactons der allgemeinen Formel

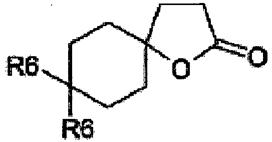

wobei die Gruppe R6 ein Wasserstoffatom oder eine Alkylgruppe mit 1 bis 4 Kohlenstoff-atomen ist, in Anwesenheit eines Reduktionsmittels und eines Katalysators. WO 2004/089861 offenbart aber weder die Verbindung der Formel (VIII) noch deren Pyrolyseprodukt (I). Ein ähnliches Verfahren beschreibt die Veröffentlichung Helv. Chim. Acta 2004, 87, 1697-1703**.**

Ein bewährtes Verfahren zur Herstellung der Verbindungen der Formel (la) und (Ib) - jeweils wie oben definiert - umfasst die oder besteht aus den folgenden Schritten
(a) Bereitstellen von 4-Isopropyl-Phenol (X) als Edukt 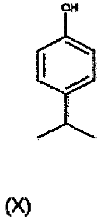
(b) Hydrieren des bereitgestellten Edukts (X) zu 4-Isopropyl-Hydroxycyclohexan (IX) 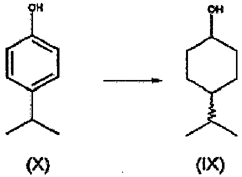
(c) Umsetzen von 4-Isopropyl-Hydroxycyclohexan (IX) mit Acrylsäuremethylester unter Bildung der Spirolactons der Formel (VIII) 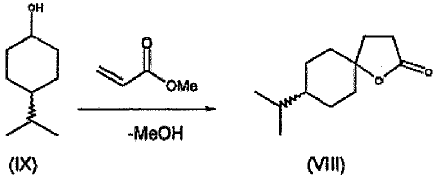
(d) Pyrolysieren der Spirolactons der Formel (VIII) unter Bildung der Verbindung der Formel (I) 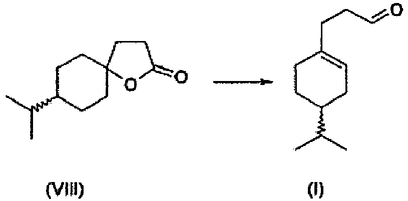
(e) optional Abtrennen des Enantiomeren der Formel (la) wie oben definiert und/oder des Enantiomeren der Formel (Ib) wie oben definiert.

In eigenen Untersuchungen wurde festgestellt, dass der Einsatz von auf Bimssteinen aufgezogenem Cerium(III)acetat-Hydrat als Katalysator beim Schritt der Pyrolyse (d) die besten Ergebnisse liefert. Es wurde eine Rohausbeute von 28 % erreicht, wobei das Produkt ein Racemat ist.

Ein spezielles Verfahren zur Herstellung der erfindungsgemäßen Verbindung der Formel (la) wie oben definiert und/oder der erfindungsgemäßen Verbindung der Formel (Ib) wie oben definiert umfasst die folgenden Schritte oder besteht aus den folgenden Schritten
(i) Schritte (a) und (b) wie oben beschrieben
(ii) nach Schritt (b) Trennen der cis und trans Isomeren (IXa, IXb) 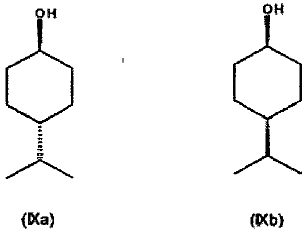
(iii) separates Umsetzen des Enantiomers der Formel (IXa) und/oder des Enantiomers der Formel (IXb) entsprechend den Schritten (c) und (d) des oben definierten Verfahrens zur Verbindung der Formel (la) bzw. (Ib).

### BEISPIELE

### BEISPIEL 1

### Synthese von 4-Isopropylcyclohexanol (IX)

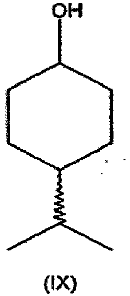

In einem Autoklav werden 1 kg 4-Isopropylphenol (Formel (X) wie oben definiert), 20 g Ruthenium auf Kohle und 2.5 L Ethanol vorgelegt. Das 4-Isopropylphenol wird 6 Stunden bei 140 °C und einem Wasserstoffdruck von 40 bar hydriert. Nach Filtration und Entfernung des Lösungsmittels erhält man 1.04 kg Rohprodukt in einer Reinheit von 99.8 % (*cis*/*trans* Isomere im Verhältnis 1:1, quantitative Ausbeute).

### BEISPIEL 2

### Synthese von 8-Isopropyl-4-oxaspiro[4.5]decan-3-on (VIII)

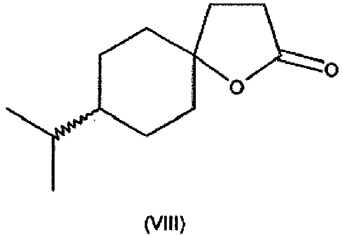

Eine Mischung aus 768 g 4-Isopropylcyclohexanol (5.40 mol, 4.50 Eq., Formel (IX) wie oben definiert) und 1.90 g Ditert-butylperoxid (13.0 mmol, 0.01 Eq.) wird auf 140 °C erwärmt. Bei dieser Temperatur wird eine Mischung aus 257 g 4-Isopropylcyclohexanol (1.80 mol, 1.50 Eq.) und 103 g Methylacrylat (1.20 mol, 1.00 Eq.) über 6 Stunden dazu getropft. Dabei werden die entstehenden Leichtsieder abgenommen. Anschließend werden 6.5 g Di-*tert*.-butylperoxid (44.0 mmol, 0.04 Eq.) zugegeben, und es wird 2 Stunden bei 160 °C nachgerührt. Nach Abkühlung wird der Ansatz mit 150 g 10 %iger Natriumsulfit Lösung versetzt und eine Stunde bei 60°C gerührt. Die Phasen werden getrennt, die organische Phase mit 100 g Wasser gewaschen. Nach Entfernung des Lösungsmittels wird das Rohprodukt durch eine Destillation gereinigt. Es werden 102 g (3*R*)-3-Isopropyl-6-methyl-7-oxabicyclo[4.1.0]heptan in einer Reinheit von 90 % isoliert *(cis*/*trans* Isomere im Verhältnis 40:60). Dies entspricht einer Ausbeute von 43 % der Theorie. Der Siedepunkt des Endproduktes beträgt 90°C bei 0.1 mbar.

***trans*-8-Isopropyl-4-oxaspiro[4.5]decan-3-on**: MS: m/z (%) = 196 (M⁺, 5), 181 (6), 167 (3), 153 (10), 136 (24), 123 (23), 111 (100), 98 (30), 85 (27), 67 (16), 55 (29), 43 (31), 28 (10).^{- 1}H NMR (400 MHz, CDCl₃): ? (ppm) = 0.87 (d, J = 6.8 Hz, 6 H), 1.02-1.14 (m, 1 H), 1.36-1.52 (m, 5 H), 1.57-1.64 (m, 2 H), 1.89-1.94 (m, 2 H), 1.97 (t, J = 8.3 Hz, 2 H), 2.58 (t, J = 8.3 Hz, 2 H).^{- 13}C NMR (100 MHz, CDCl₃): ? (ppm) = 20.0 (2 x CH₃), 26.3 (2 x CH₂), 28.7 (CH₂), 30.4 (CH₂), 32.0 (CH), 36.4 (2 x CH₂), 42.6 (CH), 87.3 (C_{quart.}), 176.7 (C_{quart.}).

***cis*-8-Isopropyl-4-oxaspiro[4.5]decan-3-on:** MS: m/z (%) = 196 (M^{+.}, 3), 181 (5), 167 (3), 153 (7), 136 (19), 123 (23), 111 (100), 98 (27), 85 (25), 67 (17), 55 (28), 43 (30), 28 (10).⁻¹H NMR (400 MHz, CDCl₃): ? (ppm) = 0.88 (d, J = 6.8 Hz, 6 H), 1.02-1.14 (m, 3 H), 1.36-1.52 (m, 1 H), 1.68-1.82 (m, 6 H), 2.06 (t, J = 8.3 Hz, 2 H), 2.58 (t, J = 8.3 Hz, 2 H).⁻¹³C NMR (100 MHz, CDCl₃): ? (ppm) = 19.8 (2 x CH₃), 25.4 (2 x CH₂), 28.7 (CH₂), 32.5 (CH), 34.2 (CH₂), 37.2 (2 x CH₂), 43.0 (CH), 85.9 (C_{quart.}), 177.0 (C_{quart.}).

### BEISPIEL 3

### Synthese von 3-(4-Isopropylcyclohexen-1-yl)propanal (I) durch Pyrolyse von 8-Isopropyl-4-oxaspiro[4.5]decan-3-on (VIII)

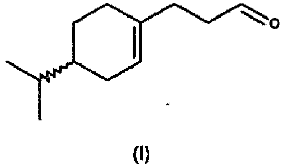

**Vorbereitung des Katalysators.** Zu einer Mischung aus 34 g Cerium(III)acetat Hydrat und 87 g Bimssteinen werden 450 g Wasser zugegeben. Die Mischung wird eine Stunde bei 50 °C gerührt, und anschließend wird das Wasser am Rotationsverdampfer entfernt. Das Rohr der Pyrolyseanlage wird mit 35 g der vorbereiteten Bimssteine gefüllt (11 cm hoch) und eine Stunde bei 440 °C geheizt.

***Pyrolyse.*** Eine Mischung aus 2 g 8-Isopropyl-4-oxaspiro[4.5]decan-3-on (93 Gew.-%ig, 9.5 mmol, Formel (VIII) wie oben definiert) und 18 g Ameisensäure wird bei 440 °C und über 2 Stunden mit einem N₂-Fluss von 16 L/h in die Anlage mit dem oben beschriebenen Katalysator eindosiert. Das Pyrolyseprodukt wird in einer Kühlfalle aufgefangen, anschließend mit MTBE verdünnt und mit gesättigter Natriumhydrogencarbonat-Lösung neutralisiert. Die Phasen werden getrennt, und nach Entfernung des Lösungsmittels erhält man 1.5 g Rohprodukt in einer Reinheit von 32 %. Dies entspricht eine Rohausbeute von 28 %.

***Auswertung.*** Die NMR- und MS-Daten des erhaltenen Produkts entsprechen den in Beispiel 8 angegebenen Daten.

### BEISPIEL 4

### Synthese von (+)-p-Menth-1-en (Va) ausgehend von d-(+)-Limonen (VIa)

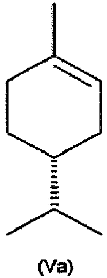

Eine Mischung aus 1.5 kg d-(+)-Limonen (98,7 %ig, Formel (VIa) wie oben definiert), 3.0 L Methanol und 4.5 g Raney-Nickel wird in einem Autoklav bei Raumtemperatur unter einem Druck von 50 bar hydriert. Nach Ende der Reaktion wird die Reaktionsmischung filtriert und anschließend destilliert. Es werden 1.3 kg (+)-*p*Menth-1-en (Formel (Va) wie oben definiert) in einer Reinheit von 91 % isoliert. Dies entspricht einer Ausbeute von 78 % der Theorie.

Siedepunkt: 102 °C / 110 mbar. - MS: m/z (%) = 138 (M^{+.}, 32), 123 (13), 109 (5), 95 (100), 81 (23), 68 (50), 55 (19), 53 (9), 43 (7), 41 (20), 39 (10), 27 (10).^{- 1}H NMR (200 MHz, CDCl₃): ω (ppm) = 0.87 (d, J = 6.8 Hz, 3 H), 0.89 (d, J = 6.8 Hz, 3 H), 1.15-1.26 (m, 2 H), 1.41-1.50 (m, 1 H), 1.62-1.65 (m, 3 H), 1.72-1.78 (m, 2 H), 1.87-2.04 (m, 3 H), 5.35-5.40 (m, 1 H).⁻¹³C NMR (50 MHz, CDCl₃): ? (ppm) = 19.7 (CH₃), 20.0 (CH₃), 23.5 (CH₃), 26.6 (CH₂), 29.0 (CH₂), 30.9 (CH₂), 32.4 (CH), 40.1 (CH), 121.1 (CH), 133.8 (C_{quart.}).

### BEISPIEL 5

### Synthese von (3R)-3-Isopropyl-6-methyl-7-oxabicyclo[4.1.0]heptan (IVa)

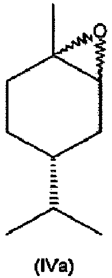

In einer Mischung aus 152 g (+)-p-Menth-1-en (91 % ig, 1.00 mol, 1.00 Eq., Formel (Va) wie oben definiert) und 53.0 g Natriumcarbonat (0.50 mol, 0.50 Eq.) in 500 mL Toluol bei 0 °C werden 238 g Peressigsäure (40 % ig, 1.25 mol, 1.25 Eq.) zugetropft. Anschließend wird die Reaktionsmischung auf Raumtemperatur erwärmt und für 3 Stunden gerührt. Die Reaktionsmischung wird mit Wasser versetzt, die organische Phase abgetrennt und mit einer 20 Gew.-%igen Lösung von Natriumsulfit für 2 Stunden bei 60 °C gerührt. Danach werden die Phasen wieder getrennt und die organische Phase zweimal mit Wasser gewaschen. Nach Entfernung des Lösungsmittels wird das Rohprodukt durch eine Destillation gereinigt. Es werden 133 g (3*R*)-3-Isopropyl-6-methyl-7-oxabicyclo[4.1.0]heptan (Formel (IVa) wie oben definiert) in einer Reinheit von 96 % isoliert (*cis*/*trans* Isomere im Verhältnis ca. 1:1). Dies entspricht einer Ausbeute von 83 % der Theorie. Der Siedepunkt des Endproduktes beträgt 102 °C bei 40 mbar.

**(1*R*, 3*R*, 6*S*)-3-Isopropyl-6-methyl-7-oxabicyclo[4.1.0]heptan:** MS: m/z (%) = 154 (M^{+.}, 2), 139 (20), 125 (14), 111 (62), 95 (17), 83 (25), 69 (47), 55 (49), 43 (100), 27 (14). - ¹H NMR (400 MHz, CDCl₃): d (ppm) = 0.83 (d, J = 6.8 Hz, 6 H), 0.92-1.07 (m, 1 H), 1.11 (dd, J = 12.4, 4.1 Hz, 1 H), 1.30 (s, 3 H), 1.31-1.47 (m, 3 H), 1.62 (ddd, J = 14.5, 12.2, 4.9 Hz, 1 H), 1.95 (dddd, J = 15.2, 6.0, 5.5, 2.0 Hz, 1 H), 1.93 (ddd, J = 14.6, 3.8, 2.4 Hz, 1 H), 2.97 (d, J = 5.3 Hz, 1 H). - ¹³C NMR (100 MHz, CDCl₃): d (ppm) = 19.4 (CH₃), 19.7 (CH₃), 22.5 (CH₂), 23.1 (CH₃), 27.9 (CH₂), 31.0 (CH₂), 32.3 (CH), 39.3 (CH), 57.8 (C_{quart.}), 59.7 (CH).

**(1*S*, 3*R*, 6*R*)-3-Isopropyl-6-methyl-7-oxabicyclo[4.1.0]heptan:** MS: m/z (%) = 154 (M^{+.}, 2), 139 (18), 125 (14), 111 (57), 93 (18), 83 (29), 69 (47), 55 (54), 43 (100), 27 (15). - ¹H NMR (400 MHz, CDCl₃): d (ppm) = 0.84 (d, J = 6.7 Hz, 3 H), 0.85 (d, J = 6.7 Hz, 3 H), 0.89-0.96 (m, 1 H), 1.30 (s, 3 H), 1.20-1.28 (m, 1 H), 1.31-1.47 (m, 3 H), 1.79-1.85 (m, 2 H), 2.08 (ddd, J = 14.6, 4.3, 2.0 Hz, 1 H), 3.02 (t, J = 2.1 Hz, 1 H). -¹³C NMR (100 MHz, CDCl₃): d (ppm) = 19.7 (2 x CH₃), 24.5 (CH₃), 24.9 (CH₂), 29.2 (CH₂), 29.3 (CH₃), 31.7 (CH), 35.1 (CH), 37.6 (C_{quart.}), 61.0 (CH).

### BEISPIEL 6

### Synthese von (5R)-5-Isopropyl-2-methylencyclohexanol (IIIa)

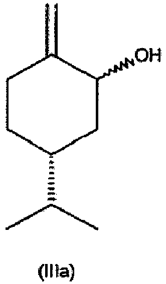

Zu einer Mischung aus 401 g (3*R*)-3-Isopropyl-6-methyl-7-oxabicyclo[4.1.0]heptan (96 Gew.-%ig, 2.48 mol, 1.00 Eq., Formel (IVa) wie oben definiert) in 1.4 L Xylol werden 49.0 g Aluminiumisopropylat (0.24 mol, 0.10 Eq.) vorsichtig zugetropft. Anschließend wird der Ansatz 4 Stunden am Rückfluss gekocht. Die Reaktionsmischung wird auf Raumtemperatur abgekühlt, mit einer 10 Gew.-%igen Salzsäurelösung versetzt und die Phasen getrennt. Die wässrige Phase wird mit Xylol extrahiert und die vereinigten organischen Phasen werden mit Wasser gewaschen. Das Rohprodukt wird durch eine Destillation gereinigt. Es werden 327 g (5*R*)-5-Isopropyl-2-methylencyclohexanol (Formel (IIIa) wie oben definiert) in einer Reinheit von 71 % isoliert (enthält 62 g von (5*R*)-5-Isopropyl-2-methylcyclohex-2-en-I-ol). Dies entspricht einer Ausbeute von 60 % der Theorie. Der Siedepunkt des Endproduktes beträgt 75 °C bei 25 mbar.

**(1*R*, 5*R*)-5-Isopropyl-2-methylencyclohexanol:** MS: m/z (%) = 154 (M^{+.}, 14), 139 (7), 121 (13), 111 (51), 97 (18), 93 (48), 82 (60), 69 (63), 55 (100), 53 (20), 41 (83), 27 (27). - ¹H NMR (400 MHz, CDCl₃): d (ppm) = 0.88 (2d, J = 6.8 Hz, 6 H), 0.96-1.07 (m, 2 H), 1.33-1.42 (m, 1 H), 1.46-1.54 (m, 1 H), 1.71-1.78 (m, 1 H), 1.93-2.03 (m, 1 H), 2.11 (dddd, J = 11.8, 5.2, 2.9, 2.2 Hz, 1 H), 2.42 (ddd, J = 13.5, 4.2, 2.6 Hz, 1 H), 4.01-4.09 (m, 1 H), 4.75 (q, J = 1.8 Hz, 1 H), 4.91 (dd, J = 1.8, 1.5 Hz, 1 H). - ¹³C NMR (100 MHz, CDCl₃): d (ppm) = 19.7 (CH₃), 19.9 (CH₃), 30.7 (CH₂), 32.3 (CH), 33.8 (CH₂), 40.5 (CH₂), 42.9 (CH), 72.5 (CH), 103.3 (CH₂), 152.0 (C_{quart.}).

**(1*S*, 5*R*)-5-Isopropyl-2-methylencyclohexanol:** MS: m/z (%) = 154 (M^{+.}, 9), 136 (7), 125 (4), 121 (18), 111 (56), 97 (18), 93 (72), 83 (61), 77 (30), 67 (64), 55 (100), 53 (23), 41 (91), 27 (39). - ¹H NMR (400 MHz, CDCl₃): d (ppm) = 0.87 (d, J = 6.8 Hz, 3 H), 0.88 (d, J = 6.8 Hz, 3 H), 1.09 (tdd, J = 12.7, 11.5, 4.1 Hz, 1 H), 1.34 (ddd, J = 13.6, 12.1, 3.1 Hz, 1 H), 1.42-1.51 (m, 1 H), 1.64-1.74 (m, 1 H), 1.75-1.81 (m, 1 H), 1.90 (ddd, J = 13.6, 3.5, 2.3 Hz, 1 H), 2.18 (ddd, J =13.5, 4.0, 3.5 Hz, 1 H), 2.36-2.45 (m, 1 H), 4.33 (t, J = 3.4 Hz, 1 H), 4.74 (dd, J = 2.0, 0.6 Hz, 1 H), 4.82 (dd, J = 2.1, 1.5 Hz, 1 H). - ¹³C NMR (100 MHz, CDCl₃): d (ppm) = 19.7 (CH₃), 19.9 (CH₃), 30.0 (CH₂), 30.5 (CH₂), 31.9 (CH), 36.9 (CH), 37.5 (CH₂), 72.6 (CH), 109.2 (CH₂), 150.6 (C_{quart.}).

### BEISPIEL 7

### (4R)-2-(1-butoxyethoxy)-4-isopropyl-I-methylencyclohexan (IIa)

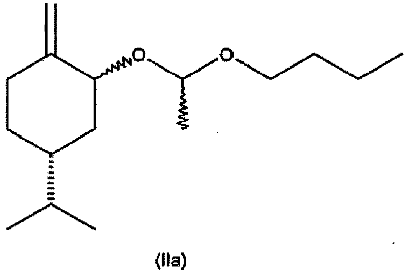

Zu einer Mischung aus 42.9 g (5*R*)-5-Isopropyl-2-methylencyclohexanol (70 Gew.-%ig, 195 mmol, 1.00 Eq., Formel (IIIa) wie oben definiert)) und 55.7 g Butylvinylether (556 mmol, 2.90 äquiv.) bei 0 °C werden portionsweise 317 mg p-Toluolsulfonsäuremonohydrat (1.70 mmol, 0.6 Mol-%.) zugegeben. Die Reaktion ist exotherm und die freigesetzte Wärme wird mittels eines Eisbades bei 17 °C abgefangen. Anschließend wird 2 Stunden bei 0 bis 10 °C nachgerührt. Die Reaktionsmischung wird mit MTBE versetzt, dreimal mit einer 5 % igne Na₂CO₃ Lösung und einmal mit Wasser gewaschen. Die organische Phase wird dann abgetrennt und über Na₂CO₃ destilliert. Es werden 58.4 g (4*R*)-2-(1-butoxyethoxy)-4-isopropyl-1-methylencyclohexan in einer Reinheit von 71 % isoliert (4 Isomere im Verhältnis ca. 1: 1: 1.5: 1.3). Dies entspricht einer Ausbeute von 83 % der Theorie.

MS: m/z (%) = 137 (23), 101 (78), 81 (32), 67 (8), 57 (52), 45 (100), 41 (24), 29 (13).

### BEISPIEL 8

### Synthese von 3-[(4R)-4-Isopropylcyclohexen-1-yl]propanal (Ia)

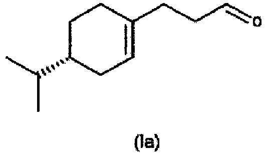

Eine Mischung aus 24 g (4*R*)-2-(1-Butoxyethoxy)-4-isopropyl-1-methylencyclohexan (77 Gew.-%ig, 72 mmol, 1.00 Eq., Formel (IIa) wie oben definiert) und 2.5 g Capronsäure (21 mmol, 0.30 äquiv.) in 25 g Malotherm S wird bei 200 °C für 2 Stunden geheizt. Dabei werden die Leichtsieder abdestilliert. Anschließend wird der Ansatz bei Raumtemperatur gekühlt und über Soda destilliert. Es werden 14.7 g 3-[(4*R*)-4-Isopropylcyclohexen-1-yl]-propanal (Formel (la) wie oben definiert) in einer Reinheit von 52 % und mit 96.6 % ee isoliert. Dies entspricht einer Ausbeute von 58 % der Theorie. Ein Teil des Produktes wird über eine Säule gereinigt (Cyclohexan: Essigester 100:3, 3.34 g, Reinheit 96,5 %).

[a]_{D}²² = +108 °(c = 0.07 in Ethanol). - GC mit chiraler stationärer Phase (Hydrodex-b-TBADc, 25 m x 0.25 mm, 40 °C - 1 °C/ min. - 180 °C): Rₜ = 79.08 (98.3 %), 79.30 (1.7 %). - MS: m/z (%) = 180 (M^{+.}, 10), 162 (7), 147 (7), 136 (34), 119 (24), 109 (18), 93 (100), 79 (41), 67 (56), 55 (34), 41 (69), 27 (33). - ¹H NMR (400 MHz, CDCl₃): d (ppm) = 0.87 (d, J = 6.8 Hz, 3 H), 0.88 (d, J = 0.88 Hz, 3 H), 1.16-1.28 (m, 2 H), 1.42-1.50 (m, 1 H), 1.66-1.80 (m, 2 H), 1.94-2.06 (m, 3 H), 2.28 (t, J = 7.5 Hz, 2 H), 2.49-2.54 (m, 2 H), 5.39-5.43 (m, 1 H), 9.75 (t, J = 1.9 Hz, 1 H). - ¹³C NMR (100 MHz, CDCl₃): d (ppm) = 19.6 (CH₃), 19.9 (CH₃), 26.3 (CH₂), 28.8 (CH₂), 29.2 (CH₂), 29.7 (CH₂), 32.2 (CH), 40.0 (CH), 41.9 (CH₂), 121.8 (CH), 135.5 (C_{quart.}), 202.8 (CHO).

### BEISPIEL 9A

### Fine Fragrance (Parfümöl) (Mengenangaben in Gew.-%)

| **RIECHTOFFE** | **Parfümöl A1 (Vergleich)** | **Parfümöl A2 (Erfindungsgemäß)** |
|---|---|---|
| ETHYL ACETOACETATE | 4,0 | 4,0 |
| HEXENOL CIS-3 10 % DPG | 3,0 | 3,0 |
| HEXENYL ACETATE CIS-3 10 % DPG | 3,0 | 3,0 |
| HEXENYL BENZOATE CIS-3 10 % DPG | 3,0 | 3,0 |
| VERTOCITRAL 10 % DPG | 5,0 | 5,0 |
| CYCLOGALBANAT(R) 10 % DPG | 3,0 | 3,0 |
| STYRALYL ACETATE 10 % DPG | 6,0 | 6,0 |
| BERGAMOT OIL BERGAPTEN FREE FF | 6,0 | 6,0 |
| MANDARIN OIL DIST. DECOL. | 15,0 | 15,0 |
| METHYL ANTHRANILATE 10 % DPG | 5,0 | 5,0 |
| RED BERRY EXTR. | 2,0 | 2,0 |
| DECALACTONE GAMMA | 2,0 | 2,0 |
| ALLYL CAPROATE 10 % DPG | 5,0 | 5,0 |
| PRUNELLA TYPE BASE | 6,0 | 6,0 |
| HELIONAL | 20,0 | 20,0 |
| MUGETANOL | 10,0 | 10,0 |
| ETHYL LINALOOL | 35,0 | 35,0 |
| CITRONELLOL 950 | 10,0 | 10,0 |
| GERANIOL SUPER | 3,0 | 3,0 |
| ROSAPHEN(R) | 8,0 | 8,0 |
| CITRONELLYL ACETATE EXTRA | 2,0 | 2,0 |
| DAMASCONE BETA 10 % DPG | 6,0 | 6,0 |
| BENZYL ACETATE | 10,0 | 10,0 |
| HEDION HC/30 | 30,0 | 30,0 |
| HEDIONE | 140,0 | 140,0 |
| HEXYL CINNAMIC ALDEHYDE ALPHA | 30,0 | 30,0 |
| LACTOJASMONE | 3,0 | 3,0 |
| BENZYL SALICYLATE | 80,0 | 80,0 |
| HEXENYL SALICYLATE CIS-3 | 30,0 | 30,0 |
| METHYL IONONE GAMMA CÖUR | 3,0 | 3,0 |
| CLOVE BUD OIL | 1,0 | 1,0 |
| VANILLIN | 1,5 | 1,5 |
| COUMARIN | 1,0 | 1,0 |
| AMBERWOOD(R) F | 5,0 | 5,0 |
| CASHMERAN | 8,0 | 8,0 |
| CEDRENE | 15,0 | 15,0 |
| ISO E SUPER NON DISCOLORING | 50,0 | 50,0 |
| BRAHMANOL(R) | 25,0 | 25,0 |
| SANDALORE | 5,0 | 5,0 |
| AMBROXIDE | 2,0 | 2,0 |
| AMBRETTOLIDE | 5,0 | 5,0 |
| AURELIONE(R) | 16,0 | 16,0 |
| GLOBALIDE(R) | 24,0 | 24,0 |
| MACROLIDE(R) SUPRA | 16,0 | 16,0 |
| INDOLE FF 10 % DPG | 5,0 | 5,0 |
| **3-[(4*R*)-4-Isopropylcyclohexen-1-yl]propanal** | | **50,0** |
| DIPROPYLENE GLYCOL | 332,5 | 282,5 |
| | 1.000,0 | 1.000,0 |

Bei einer Dosierung von 6 Gew.-% des Parfümöls A1 bzw. A2 in Ethanol ergibt sich folgender Befund: Durch den Zusatz von 3-[(4*R*)-4-Isopropylcyclohexen-1-yl]propanal im Parfümöl A2 wird die blumige Note gegenüber dem Parfümöl A1 (ohne 3-[(4*R*)-4-Isopropylcyclohexen-1-yl]propanal) verstärkt. Weiterhin verleiht 3-[(4*R*)-4-Isopropylcyclohexen-1-yl]propanal der Komposition mit dem Parfümöl A2 mehr Kraft und Fülle als der Komposition mit dem Parfümöl A1.

### BEISPIEL 10A

### Bodylotion (Mengenangaben in Gew.-%)

| **KOMPONENTE** | **A** | **B** |
|---|---|---|
| Paraffinöl | 5,00 | 5,00 |
| Isopropylpalmitat | 5,00 | 5,00 |
| Cetylalkohol | 2,00 | 2,00 |
| Bienenwachs | 2,00 | 2,00 |
| Ceteareth-20 | 2,00 | 2,00 |
| PEG-20-Glycerylstearat | 1,50 | 1,50 |
| Glycerin | 3,00 | 3,00 |
| Phenoxyethanol | 0,50 | - |
| Parabene (Mischung aus Methyl-, Ethyl-, Propyl-, Butyl-, Isobutylparaben) | - | 0,50 |
| Parfümöl B1 bzw. B2 | 0,50 | 0,50 |
| Wasser | Ad 100 | Ad 100 |

### BEISPIEL 10B

### Zusammensetzung des Parfümöls B1 bzw. B2 (Mengenangaben in Gew.-%)

| **RIECHSTOFFE** | **Parfümöl B1 (Vergleich)** | **Parfümöl B2 (Erfindungsgemäß)** |
|---|---|---|
| NONADIENAL TRANS,CIS-2,6 5 % TEC 20 % DPG | 2,0 | 2,0 |
| ETHYL ACETOACETATE | 3,0 | 3,0 |
| FARENAL(R) 10 % DPG | 5,0 | 5,0 |
| VERTOCITRAL | 3,0 | 3,0 |
| CYCLOGALBANAT(R) 10 % DPG | 2,0 | 2,0 |
| STYRALYL ACETATE | 3,0 | 3,0 |
| MELONAL(R) | 0,5 | 0,5 |
| DIHYDRO MYRCENOL | 15,0 | 15,0 |
| LINALYL ACETATE | 20,0 | 20,0 |
| LEMON OIL TERPENES FLAVOR WONF | 8,0 | 8,0 |
| EUCALYPTOL NAT. 10 % DPG | 0,5 | 0,5 |
| HEXYL ACETATE | 1,5 | 1,5 |
| ISOAMYL ACETATE 10 % DPG | 4,0 | 4,0 |
| PRENYL ACETATE 10 % DPG | 4,0 | 4,0 |
| ALDEHYDE C14 SO-CALLED | 2,0 | 2,0 |
| ETHYL METHYL BUTYRATE-2 | 1,0 | 1,0 |
| ALLYL CYCLOHEXYL PROPIONATE | 2,0 | 2,0 |
| ALDEHYDE C16 SO-CALLED | 1,0 | 1,0 |
| FRAGOLANE(R) | 0,5 | 0,5 |
| MAJANTOL(R) | 25,0 | 25,0 |
| LINALOOL | 40,0 | 40,0 |
| DIMETHYL BENZYL CARBINOL | 10,0 | 10,0 |
| TERPINEOL PURE | 10,0 | 10,0 |
| PHENIRAT(R) | 30,0 | 30,0 |
| CITRONELLOL 950 | 15,0 | 15,0 |
| GERANIOL 60 | 10,0 | 10,0 |
| CITRONELLYLACETATE EXTRA | 2,0 | 2,0 |
| HEDIONE | 90,0 | 90,0 |
| HEXYL CINNAMIC ALDEHYDE ALPHA | 35,0 | 35,0 |
| HEXYL SALICYLATE | 160,0 | 160,0 |
| METHYL OCTIN CARBONATE 10 % DPG | 2,0 | 2,0 |
| CALONE 195110 % DPG | 1,0 | 1,0 |
| GALAXOLIDE 50 % IN IPM | 20,0 | 20,0 |
| ANETHOL SUPRA 21,5 CELSIUS | 2,0 | 2,0 |
| AGRUMEXHC | 15,0 | 15,0 |
| ORYCLON SPECIAL | 40,0 | 40,0 |

| | | |
|---|---|---|
| **3-[(4*R*)-4-Isopropylcyclohexen-1-yl]propanal** | | **15,0** |
| DIPROPYLENE GLYCOL | 415,0 | 400,0 |
| | 1.000,0 | 1.000,0 |

Bei einer Dosierung von 0.5 Gew.- % des Parfümöls B1 bzw. B2 in der Bodylotion ergibt sich folgender Befund: Aufgrund des Anteils von 3-[(4R)-4-Isopropylcyclohexen-1-yl]propanal weist die das Parfümöl B2 enthaltende Bodylotion eine natürlichere und stärkere blumige Note auf als die Bodylotion, die das Parfümöl B1 (ohne 3-[(4R)-4-Isopropylcyclohexen-1-yl] propanal) enthält. Weiterhin zeigt die Bodylotion mit dem Parfümöl B2 eine kosmetischere Topnote und einen cremigeren Nachgeruch als die Bodylotion mit dem Parfümöl B1.

### BEISPIEL 11A

### Weichspüler (Mengenangaben in Gew.-%)

| **Material** | **Hersteller** | **Chemisher Name** | **Funktion** | **Anteil** |
|---|---|---|---|---|
| Entionisiertes Wasser | | Wasser | Lösungsmittel | 72,4 |
| Rewoquat WE 18 | Evonic Goldschmidt GmbH | Dialkylesterammoniumethosulfat | Kationisches Tensid | 16,6 |
| Mergal K9N | Honeywell Austria GmbH | 5-Chloro-2-methyl-3-(2H)-isothiazolon und 2-methyl-3-(2H)-isothiazolon | Konservierungsmittel | 0,10 |
| Dow Corning 1520 Antifoam | Dow Corning GmbH, | Polydimethylsiloxan | Entschäumer | 0,30 |
| Magnesium Chlorid 1%ige Lösung | | Magnesiumchlorid-Lösung | Konsistenzgeber | 10,00 |
| Parfümöl C1 bzw. C2 | Symrise | | Parfum (Fragrance) | 0,60 |

### BEISPIEL 11B

### Zusammensetzung des Parfümöls C1 bzw. C2 (Mengenangaben in Gew.-%)

| **RIECHSTOFFE** | **Parfümöl C1 (Vergleich)** | **Parfümöl C2 (Erfindungsgemäß)** |
|---|---|---|
| ALDEHYDE C10 | 0,5 | 0,5 |
| ALDEHYDE C11 ISO | 2,0 | 2,0 |
| ALDEHYDE C11 UNDECYLENIC | 12,0 | 12,0 |
| ALDEHYDE C12 MNA | 6,0 | 6,0 |
| FARENAL(R) | 1,0 | 1,0 |
| VERTOCITRAL | 8,0 | 8,0 |
| ALLYL AMYL GLYCOLATE | 1,0 | 1,0 |
| STYRALYL ACETATE | 1,5 | 1,5 |
| DIHYDRO MYRCENOL | 65,0 | 65,0 |
| AGRUNITRIL | 1,0 | 1,0 |
| PEONILE | 15,0 | 15,0 |
| METHYL ANTHRANILATE | 10,0 | 10,0 |
| NEROLIONE 10 % DPG | 1,5 | 1,5 |
| ROSEMARY OIL BM | 7,0 | 7,0 |
| SAGE OFFICINALE OIL DALM. | 3,0 | 3,0 |
| ISOBORNYL ACETATE | 40,0 | 40,0 |
| PRENYL ACETATE | 0,5 | 0,5 |
| ISOAMYL BUTYRATE | 0,5 | 0,5 |
| ALDEHYDE C14 SO-CALLED | 5,0 | 5,0 |
| MANZANATE | 0,5 | 0,5 |
| MUGETANOL | 5,0 | 5,0 |
| DIMETHYL BENZYL CARBINYL ACETATE | 2,5 | 2,5 |
| ROSE OXIDE D | 3,0 | 3,0 |
| ANTHER | 0,5 | 0,5 |
| PHENYLETHYL ALCOHOL | 35,0 | 35,0 |
| CITRONELLOL 950 | 15,0 | 15,0 |
| GERANIOL 60 | 10,0 | 10,0 |
| ISODAMASCON(R) | 2,0 | 2,0 |
| ROSACETATE | 35,0 | 35,0 |
| CRESYL METHYL ETHER PARA | 0,5 | 0,5 |
| BENZYL ACETATE | 20,0 | 20,0 |
| HEXYL CINNAMIC ALDEHYDE ALPHA | 40,0 | 40,0 |
| METHYL BENZOATE | 1,0 | 1,0 |
| AMYL SALICYLATE N/ISO | 10,0 | 10,0 |
| BENZYL SALICYLATE | 70,0 | 70,0 |
| MYSORE ACETATE | 15,0 | 15,0 |
| PARMANYL(R) | 0,5 | 0,5 |
| EUGENOL | 10,0 | 10,0 |
| ETHYL VANILLIN | 0,5 | 0,5 |
| COUMARIN | 3,0 | 3,0 |
| AGRUMEX HC | 45,0 | 45,0 |
| HERBAFLORAT | 25,0 | 25,0 |
| AMBERWOOD(R) F | 5,0 | 5,0 |
| ISO E SUPER | 170,0 | 170,0 |
| PATCHOULI OIL DECOL. | 2,5 | 2,5 |
| SANDRANOL(R) | 10,0 | 10,0 |
| ISOBUTYL QUINOLINE | 1,0 | 1,0 |
| EVERNYL | 0,5 | 0,5 |
| AMBROXIDE | 1,0 | 1,0 |
| GALAXOLIDE 50 % IN IPM | 120,0 | 120,0 |
| INDOFLOR(R) CRYST. | 0,5 | 0,5 |
| **3-[(4*R*)-4-Isopropylcyclohexen-1-yl]propanal** | | **60,0** |
| DIPROPYLENE GLYCOL | 220,0 | 160,0 |
| | 1.000,0 | 1.000,0 |

Bei einer Dosierung von 0.6 Gew.- % des Parfümöls C1 bzw. C2 im Weichspüler ergibt sich folgender Befund: Aufgrund des Anteils von 3-[(4R)-4-Isopropylcyclohexen-1-yl]propanal weist der das Parfümöl C2 enthaltende Weichspüler eine natürlichere, frischere blumige Note auf als der Weichspüler, der das Parfümöl C1 (ohne 3-[(4R)-4-Isopropylcyclohexen-1-yl] propanal) enthält.

### BEISPIEL 12A

### Seife (Mengenangaben als Gew.-%)

| **Material** | **Hersteller** | **Chemischer Name** | **Funktion** | **Anteil** |
|---|---|---|---|---|
| Entionisiertes Wasser | | Water | Lösungsmittel | 2,0 |
| Seifen Basen Mix | Verschiedene | Sodium tallowates / palmitates | Tenside | 95,8 |
| Titan Dioxid | Kronos Titan GmbH, Deutschland | Titanium Dioxide | Farbstoff / Aufheller | 1,0 |
| Parfumöl D1 bzw. D2 | Symrise | | Parfum (Fragrance) | 1,2 |

### BEISPIEL 12B

### Zusammensetzung des Parfümöls D1 bzw. D2 (Mengenangaben als Gew.-%)

| **RIECHSTOFFE** | **Parfümöl D1 (Vergleich)** | **Parfümöl D2 (Erfindungsgemäß)** |
|---|---|---|
| ALDEHYDE C 8 | 10,0 | 10,0 |
| ALDEHYDE C10 | 14,0 | 14,0 |
| ALDEHYDE C11 UNDECYLIC | 5,0 | 5,0 |
| ALDEHYDE C12 MNA | 4,0 | 4,0 |
| ISOAMYL ALCOHOL | 6,0 | 6,0 |
| VERTOCITRAL | 4,0 | 4,0 |
| ALLYL-AMYL GLYCOLATE | 4,0 | 4,0 |
| DIHYDRO MYRCENOL | 120,0 | 120,0 |
| CITRAL 95 | 30,0 | 30,0 |
| AGRUNITRIL | 80,0 | 80,0 |
| CITRONITRILE | 40,0 | 40,0 |
| CITRYLAL | 4,0 | 4,0 |
| ORANGE OIL TERPENES | 250,0 | 250,0 |
| METHYL ANTHRANILATE | 1,5 | 1,5 |
| TERPINEOL HEAD FRACTION | 10,0 | 10,0 |
| EUCALYPTOL NAT. | 10,0 | 10,0 |
| THYMOL CRYST | 6,0 | 6,0 |
| BORNYL ACETATE L CRYST. | 82,5 | 82,5 |
| CAMPHOR DL | 20,0 | 20,0 |
| JASMAPRUNAT | 5,0 | 5,0 |
| ALDEHYDE C14 SO-CALLED | 2,0 | 2,0 |
| MANZANATE | 0,5 | 0,5 |
| ALLYL HEPTOATE | 2,5 | 2,5 |
| ROSE OXIDE L | 0,5 | 0,5 |
| DAMASCONE ALPHA | 0,5 | 0,5 |
| HEDIONE | 10,0 | 10,0 |
| AMYL SALICYLATE N/ISO | 60,0 | 60,0 |
| HEXYL SALICYLATE | 35,0 | 35,0 |
| COUMARIN | 2,5 | 2,5 |
| AGRUMEXHC | 30,0 | 30,0 |
| ORYCLON SPECIAL | 55,0 | 55,0 |
| PATCHOULI OIL DECOL. | 2,0 | 2,0 |
| SANDRANOL(R) | 1,5 | 1,5 |
| TONALIDE | 2,0 | 2,0 |
| **3-[(4*R*)-4-Isopropylcyclohexen-1-yl]propanal** | | **40,0** |
| DIPROPYLENE GLYCOL | 130,0 | 90,0 |
| | 1.000,0 | 1.000,0 |

Bei einer Dosierung von 1.2 Gew.- % des Parfümöls D1 bzw. D2 in der Seife ergibt sich folgender Befund: Aufgrund des Anteils von 3-[(4R)-4-Isopropylcyclohexen-1-yl]pro-panal weist die das Parfümöl D2 enthaltende Seife eine runde aldehydische Note auf im Gegensatz zu der Seife, die das Parfümöl D1 (ohne 3-[(4R)-4-Isopropylcyclohexen-1-yl] propanal) enthält. Weiterhin strahlt die Seife mit dem Parfümöl D2 mehr Natürlichkeit und Fülle aus als die Seife mit dem Parfümöl D1.

### BEISPIEL 13A

### Waschpulver (Mengenangaben als Gew.-%)

| **Material** | **Hersteller** | **Chemicher Name** | **Funktion** | **Anteil** |
|---|---|---|---|---|
| Natrium Metasilicat Pentahydrat | Akzo Nobel Chemicals, Deutschland | Sodium Metasilicate Pentahydrate | | 48,0 |
| Natriumhydrogencarbonat | Verschiedene | Sodium hydrogen carbonate | Alkali | 15,0 |
| Natriumpercarbonat | Verschiedene | Sodium carbonate peroxyhydrate | Bleichmittel | 15,0 |
| Peractive AC Blü | Clariant GmbH, Deutschland | TÄD / Na-Carboxymethylcellulose | Aktivator | 5,00 |
| Genapol OA-080 | Clariant GmbH, Deutschland | Oxoalkohol C14-15, 8EO | Nichtionisches Tensid | 3,00 |
| Texapon K12 Pulver | Cognis Deutschland GmbH | Sodium Lauryl Sulphate C12 | Anionisches Tensid | 7,00 |
| Tinopal CBS-X | Ciba, Deutschland | | Aufheller | 0,50 |
| Savinase 6.0 T, Type W | Novozymes | Protease | Enzym | 0,40 |
| Termamyl 120 T | Novozymes | Alpha-Amylase | Enzym | 0,30 |
| Natriumsulfat | Verschiedene | Sodium Sulphate | Füllstoff | 5,50 |
| Parfumöl E1 bzw. E2 | Symrise | | Parfum (Fragrance) | 0,30 |

### BEISPIEL 13B

### Zusammensetzung des Parfümöls E1 bzw. E2 (Mengenangaben als Gew.-%)

| **RIECHSTOFFE** | **Parfümöl E1 (Vergleich)** | **Parfümöl E2 (Erfindungsgemäß)** |
|---|---|---|
| ALDEHYDE C 8 | 0,5 | 0,5 |
| HEXENOL CIS-3 | 3,5 | 3,5 |
| VERTOCITRAL | 15,0 | 15,0 |
| STYRALYL ACETATE | 3,0 | 3,0 |
| MELONAL(R) | 2,0 | 2,0 |
| DIHYDRO MYRCENOL | 60,0 | 60,0 |
| CITRAL 95 | 6,0 | 6,0 |
| ORANGE OIL TERPENES | 20,0 | 20,0 |
| EUCALYPTUS OIL GLOBULUS 80/85 % | 10,0 | 10,0 |
| MENTHONE UISOMENTHONE D 82/18 | 1,5 | 1,5 |
| HEXYL ACETATE 10 % DPG | 5,0 | 5,0 |
| ISOPENTYRATE | 4,0 | 4,0 |
| METHYL CAPROATE | 1,0 | 1,0 |
| ETHYL METHYL BUTYRATE-2 | 6,0 | 6,0 |
| LINALOOL | 10,0 | 10,0 |
| DIMETHYL BENZYL CARBINYL BUTYRAT | 15,0 | 15,0 |
| PHENYLETHYL ALCOHOL | 15,0 | 15,0 |
| CITRONELLOL 950 | 20,0 | 20,0 |
| GERANIOL 60 | 20,0 | 20,0 |
| ISODAMASCON(R) | 10,0 | 10,0 |
| CRESYL METHYL ETHER PARA | 1,0 | 1,0 |
| BENZYL ACETON | 50,0 | 50,0 |
| HEXYL CINNAMIC ALDEHYDE ALPHA | 60,0 | 60,0 |
| DIHYDRO JASMONE | 1,0 | 1,0 |
| METHYLBENZOAT | 0,5 | 0,5 |
| HEXYL SALICYLATE | 10,0 | 10,0 |
| LEGUMINAL | 6,0 | 6,0 |
| IONONE BETA | 20,0 | 20,0 |
| ISORALDEINE 70 | 45,0 | 45,0 |
| EUGENOL | 2,0 | 2,0 |
| ACETOPHENONE 1 % DPG | 5,0 | 5,0 |
| AGRUMEX HC | 60,0 | 60,0 |
| ORYCLON SPECIAL | 40,0 | 40,0 |
| HERBAFLORAT | 10,0 | 10,0 |
| HERBYL PROPIONATE | 20,0 | 20,0 |
| KOAVONE | 20,0 | 20,0 |
| VERTOFIX | 40,0 | 40,0 |
| ISO E SUPER | 150,0 | 150,0 |
| SANDRANOL(R) | 20,0 | 20,0 |
| AMBROXIDE | 2,0 | 2,0 |
| **3-[(4*R*)-4-Isopropylcyclohexen-1-yl]propanal** | | **60,0** |
| DIPROPYLENE GLYCOL | 270,0 | 210,0 |
| | 1.000,0 | 1.000,0 |

Bei einer Dosierung von 0.3 Gew.- % des Parfümöls E1 bzw. E2 im Waschpulver (powder detergent) ergibt sich folgender Befund: der Anteil von 3-[(4R)-4-Isopropylcyclohexen-1-yl]propanal bewirkt in dem das Parfümöl E2 enthaltenden Waschpulver eine Verstärkung der grünen Noten gegenüber dem Waschpulver, das das Parfümöl E1 (ohne 3-[(4R)-4-Isopropylcyclohexen-1-yl]propanal) enthält. Weiterhin strahlt die Komposition des Waschpulvers mit dem Parfümöl E2 insgesamt mehr Frische aus als die des Waschpulvers mit dem Parfümöl E1.

### BEISPIEL 14A

### Allzweckreiniger (Mengenangaben als Gew.-%)

| **Material** | **Hersteller** | **Chemischer Name** | **Funktion** | **Anteil** |
|---|---|---|---|---|
| Entionisiertes Wasser | | Water | Lösungsmittel | 59,6 |
| Mergal K9N | Troy Chemie, Seelze | 5-Chloro-2-methyl-3-(2H)-isothiazolone und 2-methyl-3-(2H)-isothiazolone | Konservierungs-mittel | 0,1 |
| Trinatriumcitrat Dihydrat | Verschiedene | Tri Sodium Citrate Dihydrate | Komplexbildner | 3,0 |
| Zetesol NL-2 | Zschimmer and Schwarz, Deutschland | Fatty alcohol C12-14-sulfate, Sodium | Anionisches Tensid | 30,0 |
| Imbentin | Dr. W. Kolb AG | Fatty alcohol C12-C15, | Nichtionisches | 5,0 |
| C/125/055 | Chem. | 8EO | Tensid | |
| Ethanol 96 % | Verschiedene | Ethanol | Lösungsmittel | 2,0 |
| Parfumöl F1 bzw. F2 | Symrise | | Parfum (Fragrance) | 0,3 |

### BEISPIEL 14B

### Zusammensetzung des Parfümöls F1 bzw. F2 (Mengenangaben als Gew.-%)

| **RIECHESTOFFE** | **Parfümöl F1 (Vergleich)** | **Parfümöl F2 (Erfindugsgemäß)** |
|---|---|---|
| ALDEHYDE C10 | 0,5 | 0,5 |
| ALCOHOL C10 | 6,0 | 6,0 |
| HEXENOL CIS-3 | 1,0 | 1,0 |
| LIMONENAL | 4,0 | 4,0 |
| VERTOCITRAL | 8,0 | 8,0 |
| PHENYLACETALDEHYDE 50 % DPG | 1,0 | 1,0 |
| MINTONAT | 15,5 | 15,5 |
| METHYL ANTHRANILATE | 1,0 | 1,0 |
| HEXYLACETATE | 20,0 | 20,0 |
| ALLYL HEPTOATE | 1,0 | 1,0 |
| LINALOOL | 30,0 | 30,0 |
| DIMETHYL BENZYL CARBINYL ACETATE | 2,0 | 2,0 |
| PHENYLETHYL ALCOHOL | 150,0 | 150,0 |
| CITRONELLOL 950 | 130,0 | 130,0 |
| GERANIOL SUPER | 35,0 | 35,0 |
| GERANYL ACETATE PURE | 10,0 | 10,0 |
| BENZYL ACETATE | 55,0 | 55,0 |
| HEDIONE | 15,0 | 15,0 |
| HEXYL CINNAMIC ALDEHYDE ALPHA | 130,0 | 130,0 |
| CINNAMIC ALCOHOL | 6,0 | 6,0 |
| ACETOPHENONE 10 % DPG | 1,0 | 1,0 |
| JACINTHAFLOR(R) | 4,0 | 4,0 |
| ISO E SUPER | 20,0 | 20,0 |
| GALAXOLIDE 50 % IN IPM | 35,0 | 35,0 |
| INDOFLOR(R) CRYST. | 1,0 | 1,0 |
| BHT IONOL | 8,0 | 8,0 |
| **3-[(4*R*)-4-Isopropylcyclohexen-1-yl]propanal** | | **60,0** |
| DIPROPYLENE GLYCOL | 370,0 | 310,0 |
| | 1.000,0 | 1.000,0 |

Bei einer Dosierung von 0.3 Gew.- % des Parfümöls F1 bzw. F2 im Allzweckreiniger ergibt sich folgender Befund: der Anteil von 3-[(4R)-4-Isopropylcyclohexen-1-yl)propanal bewirkt in dem das Parfümöl F2 enthaltenden Allzweckreiniger eine Verstärkung der blumigen Note gegenüber dem Allzweckreiniger, der das Parfümöl F1 (ohne 3-[(4R)-4-Isopropylcyclohexen-1-yl]propanal) enthält. Ferner erscheint die Komposition des Allzweckreinigers mit dem Parfümöl F2 viel cremiger und frischer als die des Allzweckreinigers mit dem Parfümöl F1.

### BEISPIEL 15A

### Shampoo (Mengenangaben als Gew.-%)

| **Material** | **Hersteller** | **INCI-Name** | **Anteil** |
|---|---|---|---|
| Entionisiertes Wasser | | Water | 71,5 |
| Plantacare PS 10 | Cognis Deutschland GmbH | Sodium Laureth Sulfate, Lauryl Glucoside | 20,0 |
| Euperlan PK 771 | Cognis Deutschland GmbH | Glycol Distearate, Sodium Lauryl Sulfate, Cocamide MEA, Laureth-1 0 | 6,0 |
| Dragocid Liquid | Symrise | Phenoxyethanol, Methylparaben, Ethylparaben, Butylparaben, Propylparaben, Isobutylparaben | 0,5 |
| Natrium Chlorid | | Sodium Chloride | 1,4 |
| Citronensäure Monohydrat kristallin | | Citric Acid | 0,1 |
| Parfumöl G1 bzw. G2 | Symrise | Parfum (Fragrance) | 0,5 |

### BEISPIEL 15B

### Zusammensetzung des Parfümöls G1 bzw. G2 (Mengenangaben als Gew.-%)

| **RIECHSTOFFE** | **Parfümöl G1 (Vergleich)** | **Parfümöl G2 (Erfindungsgemäß)** |
|---|---|---|
| FARENAL(R) | 3,0 | 3,0 |
| FLORAZON | 0,5 | 0,5 |
| HEXENYL ACETATE CIS-3 | 3,0 | 3,0 |
| VERTOCITRAL | 1,0 | 1,0 |
| DYNASCONE 10 % DPG | 2,0 | 2,0 |
| CYCLOGALBANAT(R) | 2,0 | 2,0 |
| STYRALYL ACETATE | 1,5 | 1,5 |
| DIHYDRO MYRCENOL | 6,0 | 6,0 |
| OXANTHIA 50 % IN TEC 10 % DPG | 10,0 | 10,0 |
| LEMON OIL ITAL. | 10,0 | 10,0 |
| ORANGE OIL BRASIL | 50,0 | 50,0 |
| HEXYL ACETATE | 2,0 | 2,0 |
| ISOAMYL ACETATE | 0,5 | 0,5 |
| PRENYLACETATE | 0,5 | 0,5 |
| ETHYL BUTYRATE 10 % DPG | 2,0 | 2,0 |
| ALDEHYDE C14 SO-CALLED | 25,0 | 25,0 |
| DECALACTONE GAMMA | 5,0 | 5,0 |
| ETHYL METHYL BUTYRATE-2 | 1,0 | 1,0 |
| ALLYL CAPROATE | 1,5 | 1,5 |
| ALLYL CYCLOHEXYL PROPIONATE | 3,0 | 3,0 |
| ALLYL HEPTOATE | 2,5 | 2,5 |
| MELOZONE | 2,0 | 2,0 |
| CALONE 1951 | 0,5 | 0,5 |
| MUGETANOL | 10,0 | 10,0 |
| LINALOOL | 25,0 | 25,0 |
| DIMETHYL BENZYL CARBINYL ACETATE | 6,0 | 6,0 |
| DIMETHYL BENZYL CARBINYL BUTYRAT | 4,0 | 4,0 |
| PHENYLETHYL ACETATE | 1,5 | 1,5 |
| PHENYLETHYL ALCOHOL | 15,0 | 15,0 |
| CITRONELLOL 950 | 10,0 | 10,0 |
| GERANIOL SUPER | 5,0 | 5,0 |
| GERANYL ACETATE PURE | 15,0 | 15,0 |
| ISODAMASCON(R) | 2,0 | 2,0 |
| BENZYL ACETATE | 15,0 | 15,0 |
| HEDIONE | 90,0 | 90,0 |
| HEXYL CINNAMIC ALDEHYDE ALPHA | 35,0 | 35,0 |
| JASMONE CIS | 0,5 | 0,5 |
| BENZYL SALICYLATE | 80,0 | 80,0 |
| HEXENYL SALICYLATE CIS-3 | 30,0 | 30,0 |
| ISORALDEINE 70 | 35,0 | 35,0 |
| HERBAFLORAT | 15,0 | 15,0 |
| ISO E SUPER | 15,0 | 15,0 |
| ISOBORNYL CYCLOHEXANOL | 30,0 | 30,0 |
| BRAHMANOL(R) | 10,0 | 10,0 |
| AMBROXIDE | 1,5 | 1,5 |
| GLOBALIDE(R) | 5,0 | 5,0 |
| GALAXOLIDE 50 % IN DPG | 120,0 | 120,0 |
| **3-[(4*R*)-4-Isopropylcyclohexen-1-yl]propanal** | | **65,0** |
| DIPROPYLENE GLYCOL | 290,0 | 225,0 |
| | 1.000,0 | 1.000,0 |

Bei einer Dosierung von 0.5 Gew.- % des Parfümöls G1 bzw. G2 im Shampoo ergibt sich folgender Befund: der Zusatz 3-[(4*R*)-4-Isopropylcyclohexen-1-yl]propanal verstärkt in dem das Parfümöl G2 enthaltenden Shampoo den wässrigen-fruchtigen Charakter gegenüber dem Shampoo, welches das Parfümöl G1 (ohne 3-[(4*R*)-4-Isopropylcyclohexen-1-yl]propanal) enthält. Zudem ist in dem Shampoo mit dem Parfümöl G2 die Diffusivität erhöht gegenüber dem Shampoo mit der Parfümöl G1.

### BEISPIEL 16A

### Duschgel (Mengenangaben als Gew.-%)

| **Material** | **Hersteller** | **INCI-Name** | **Anteil** |
|---|---|---|---|
| Entionisiertes Wasser | | Wasser | 76,3 |
| Plantacare PS 10 | Cognis Deutschland GmbH | Sodium Laureth Sulfate, Lauryl Glucoside | 20,0 |
| Dragocid Liquid | Symrise | Phenoxyethanol, Methylparaben, Ethylparaben, Butylparaben, Propylparaben, Isobutylparaben | 0,5 |
| Natriumchlorid | | Sodium Chloride | 1,4 |
| Citronensäure Monohydrat kristallin | | Citric Acid | 1,3 |
| Parfumöl G1 bzw. G2 aus Beispiel 15 | Symrise | Parfum (Fragrance) | 0,5 |

Bei einer Dosierung von 0.5 Gew.- % des Parfümöls G1 bzw. G2 im Duschgel ergibt sich folgender Befund: der Zusatz von 3-[(4*R*)-4-Isopropylcyclohexen-1-yl]propanal verleiht dem das Parfümöl G2 enthaltenden Duschgel eine natürlichere und pflegendere Note gegenüber dem Duschgel, welches das Parfümöl G1 (ohne 3-[(4**R**)-4-Isopropylcyclohexen-1-yl]propanal) enthält.

### BEISPIEL 17A

### Transparente Deo-Stifte (Formulierungen A und B) bzw. Deo-Creme-Stifte (Formulierungen C und D) (Mengenangaben als Gew.-%)

| **KOMPONENTE** | **A** | **B** | **C** | **D** |
|---|---|---|---|---|
| Aluminium Zirconium Tetrachlorohydrat - Glycin Komplex | 25,00 | 20,00 | 25,00 | 20,00 |
| Dimethicon (10 Cst) | - | - | 5,00 | 5,00 |
| Cyclopentasiloxan | - | 0,50 | 1,00 | 0,50 |
| Petrolatum | 5,00 | 4,70 | 5,00 | 5,00 |
| Ozokerit | 1,00 | 1,50 | - | - |
| Stearylalkohol | 12,00 | 12,00 | - | - |
| 2-Butyloctansäure | 0,50 | - | 0,50 | - |
| Wachs | - | - | 1,25 | 1,25 |
| PPG-14 Butylether | 9,00 | 9,00 | - | - |
| Gehärtetes Rapsöl | - | - | 5,00 | 5,00 |
| Siliciumdioxid | - | - | 1,00 | - |
| Farnesol | 0,25 | - | 0,25 | - |
| Paraffinöl | 0,50 | 0,50 | - | - |
| Hydriertes Rizinusöl (castor wax) | 3,50 | 3,50 | - | - |
| Talk | 4,00 | 4,00 | - | - |
| Behenylalkohol | 0,20 | 0,20 | - | - |
| d-Panthenyltriacetat | 1,00 | 1,00 | - | - |
| Konservierungsmittel | q.s. | q.s. | q.s. | q.s. |
| Parfümöl H1 bzw. H2 | 1,50 | - | 1,15 | - |
| Parfümöl G2 aus Beispiel 15 | | 0,90 | - | 0,75 |
| Wasser | Ad 100 | Ad 100 | Ad 100 | Ad 100 |

### BEISPIEL 17B

### Zusammensetzung des Parfümöls H1 bzw. H2 (Mengenangaben als Gew.-%)

| **RIECHSTOFFE** | **Parfümöl H1 (Vergleich)** | **Parfümöl H2 (Erfindugsgemäß)** |
|---|---|---|
| ALDEHYDE C10 | 0,5 | 0,5 |
| ALDEHYDE C12 MNA 10 % DPG | 2,0 | 2,0 |
| MINTONAT | 40,0 | 40,0 |
| MELOZONE | 0,5 | 0,5 |
| VERTOCITRAL | 6,0 | 6,0 |
| DIHYDRO MYRCENOL | 120,0 | 120,0 |
| TETRAHYDRO MYRCENOL | 10,0 | 10,0 |
| AGRUNITRIL | 1,0 | 1,0 |
| ORANGE OIL BRASIL | 10,0 | 10,0 |
| TAMARINE TYPE BASE | 10,0 | 10,0 |
| HEXYLACETATE | 6,0 | 6,0 |
| ALDEHYDE C14 SO-CALLED | 1,0 | 1,0 |
| TETRAHYDRO LINALOOL | 40,0 | 40,0 |
| DIMETHYL BENZYL CARBINYL BUTYRAT | 5,0 | 5,0 |
| ORANGE FLOWER ETHER | 5,0 | 5,0 |
| PHENYLETHYL ACETATE | 6,5 | 6,5 |
| PHENYLETHYL ALCOHOL | 15,0 | 15,0 |
| GERANIOL SUPER | 30,0 | 30,0 |
| ROSAPHEN(R) | 25,0 | 25,0 |
| ISODAMASCON(R) | 0,5 | 0,5 |
| HEDIONE | 300,0 | 300,0 |
| UNDECAVERTOL | 0,5 | 0,5 |
| ALLYL IONONE | 2,5 | 2,5 |
| IONONE BETA | 10,0 | 10,0 |
| EUGENOL | 4,0 | 4,0 |
| ISO E SUPER | 30,0 | 30,0 |
| BRAHMANOL(R) | 2,0 | 2,0 |
| AMBROXIDE | 2,0 | 2,0 |
| GLOBALIDE(R) | 12,0 | 12,0 |
| GLOBANONE(R) | 8,0 | 8,0 |
| MACROLIDE(R) SUPRA | 45,0 | 45,0 |
| 3-[(4*R*)-4-Isopropylcyclohexen-1-yl]propanal | | 50,0 |
| DIPROPYLENE GLYCOL | 300,0 | 250,0 |
| | 1.000,0 | 1.000,0 |

### BEISPIEL 18

### Antiperspirant-Roll-On (Mengenangaben als Gew.-%)

| **Komponente** | **A** | **B** |
|---|---|---|
| Caprylyl Trimethicon (SilCare TM Silicone 31 M 50) | 0,30 | 0,30 |
| Steareth-20 (GENAPOL TM HS 200) | 3,00 | 3,00 |
| Steareth-2 (GENAPOLTM HS 020) | 1,50 | 1,50 |
| Dicaprylyl Ether (Cetiol TM Ö) | 2,00 | 2,00 |
| Coco Caprylat/Caprat (Cetiol TM LC) | 2,00 | 2,00 |
| Glycerin | 2,00 | 2,00 |
| Glyceryl Stearat (Cutina TM GMS) | 2,00 | 2,00 |
| Octyldodecanol (Eutanol TM G) | 1,00 | 1,00 |
| Stearyl alkohol | 2,50 | 2,50 |
| Aluminiumchlorohydrat gemäss Beispiel 1 der EP 1321431 | 10,00 | 10,00 |
| Avocado Extract Persea Gratissima | 0,30 | 0,20 |
| Parfümöl G2 aus Beispiel 15 | 0,50 | - |
| Parfümöl H2 aus Beispiel 17 | - | 0,60 |
| Wasser | Ad 100 | Ad 100 |

### BEISPIEL 19

### Antiperspirant-Stick (Mengenangaben als Gew.-%)

| **Komponente** | **A** | **B** |
|---|---|---|
| Phenyl Trimethicon (SilCare TM Silicone 15 M 50) | 13,50 | 13,50 |
| Cetearyl Alkohol | Ad 100 | Ad 100 |
| Cetiol CC (Dicaprylyl Carbonat) | 13,50 | 13,50 |
| Stearinsäure | 3,50 | 3,50 |
| PEG-40-Hydriertes Rizinusöl (Emulsogen TM HCO 040) | 4,10 | 4,10 |
| PEG-8 Distearate (Cithrol 4 DS) | 4,10 | 4,10 |
| Petrolatum | 6,90 | 6,90 |
| Aluminiumchlorohydrat | 13,80 | 13,80 |
| Aluminium Zirconium Trichlorohydrex Gly | 19,50 | 20,00 |
| Ethylhexylglycerin (Octoxyglycerin) | 0,30 | 0,20 |
| 4-Methyl-4-phenyl-2-pentanol (Vetikol) | 0,25 | 0,10 |
| Parfümöl A2 aus Beispiel 9 | 1,00 | - |
| Parfümöl G2 aus Beispiel 15 | - | 0,80 |

### BEISPIEL 20

### Aerosolspray (Mengenangaben als Gew.-%)

| **Komponente** | **A** | **B** | **C** |
|---|---|---|---|
| Octyldodecanol | 0,50 | - | 0,50 |
| Phenoxyethanol | - | - | 0,30 |
| 1,2-Pentandiol | 1,00 | 1,00 | 0,50 |
| 1,2-Hexandiol | 0,25 | 0,15 | 0,25 |
| 1,2-Octandiol | 0,25 | 0,25 | 0,25 |
| Farnesol | - | 0,25 | 0,15 |
| Ethylhexylglycerin (Octoxyglycerin) | 0,50 | 0,30 | 0,50 |
| Parfümöl A2 aus Beispiel 9 | 0,80 | - | 0,50 |
| Parfümöl H2 aus Beispiel 17 | - | 1,15 | 0,50 |
| Ethanol | Ad 100 | Ad 100 | Ad 100 |

Die nach Zusammenmischen der jeweils angegebenen Komponenten erhaltene Mischung wird mit einem Propan-Butan-Gemisch (2:7) im Gewichtsverhältnis 2 : 3 in einen Aerosolbehälter abgefüllt.

### BEISPIEL 21

### Haarconditioner mit UV-Schutz (Mengenangaben als Gew.-%)

| **Komponente** | **INCI Name** | **A** | **B** |
|---|---|---|---|
| Lanette O | Cetearyl Alcohol | 4,00 | 4,00 |
| Dragoxat 89 | Ethylhexyl Isononanoate | 4,00 | 4,00 |
| Emulsiphos | Potassium Cetyl Phosphate, Hydrogenated Palm Glycerides | 0,50 | 0,50 |
| Natrosol 250 HR | Hydroxyethylcellulose | 0,25 | 0,25 |
| Neo Heliopan Hydro | Phenylbenzimidazole Sulfonic Acid | 2,00 | 2,00 |
| L- Arginin | Arginine | 1,20 | 1,20 |
| Benzophenon-4 | Benzophenone-4 | 0,50 | 0,50 |
| Neo Heliopan AP | Disodium Phenyl Dibenzimidazole Tetrasulfonate | 0,50 | 1,00 |
| Edeta BD | Disodium EDTA | 0,05 | 0,05 |
| Dragocide Liquid | Phenoxyethanol (and) Methylparaben (and) Butyparaben (and) Ethyparaben (and) Propylparaben | 0,80 | 0,80 |
| Dow Corning 949 Cationic Emulsion | Amodimethicone, Cetrimonium Chloride, Trideceth-12 | 2,00 | 2,00 |
| Dow Corning 5200 | Laurylmethicone Copolyol | 0,50 | 0,50 |
| Parfümöl B2 | Parfum | 0,95 | - |
| Parfümöl H2 | Parfum | | 1,25 |
| Wasser | Water (Aqua) | Ad 100 | Ad 100 |

### BEISPIEL 22

### Sonnenschutzspray (Mengenangaben als Gew.-%)

| **Phase** | **Rohstoffe** | **INCI Bezeichnung** | **Anteil** |
|---|---|---|---|
| **A** | Wasser, demineralisiert | Water (Aqua) | 69,50 |
| | Glycerin | Glycerin | 4,00 |
| | 1,3 Butylenglykol | Butylene Glycol | 5,00 |
| | D-Panthenol | Panthenol | 0,50 |
| | Lara Care A-200 | Galactoarabinan | 0,25 |
| **B** | Baysiloneöl M 10 | Dimethicone | 1,00 |
| | Edeta BD | Disodium EDTA | 0,10 |
| | Copherol 1250 | Tocopheryl Acetate | 0,50 |
| | Cetiol Ö | Dicaprylyl Ether | 3,00 |
| | Neo Heliopan ^{(R)} HMS | Homosalate | 5,00 |
| | Neo Heliopan ^{(R)} AV | Ethylhexyl Methoxycinnamate | 6,00 |
| | Neo Heliopan^{(R)} 357 | Butyl Methoxydibenzoylmethane | 1,00 |
| | Corapan TQ | Diethylhexylnaphthalate | 2,00 |
| | Alpha Bisabolol | Bisabolol | 0,10 |
| | Pemulen TR-2 | Acrylates/C10-30 Alkyl Acrylate Crosspolymer | 0,25 |
| **C** | Phenoxyethanol | Phenoxyethanol | 0,70 |
| | Solbrol M | Methylparaben | 0,20 |
| | Solbrol P | Propylparaben | 0,10 |
| **D** | NaOH, 10 % ig | Sodium Hydroxide | 0,60 |
| **E** | Parfümöl B2 | Fragrance (Parfum) | 0,20 |

Herstellungsverfahren: Lara Care A-200 unter Rühren in den anderen Bestandteilen von Teil A lösen. Alle Rohstoffe des Teils B (ohne Pemulen) einwiegen und die kristallinen Substanzen unter Erwärmen lösen. Pemulen eindispergieren. Teil B zu Teil A geben und 1 Minute homogenisieren. Teile C-E zugeben und nochmals 1-2 Minuten mit dem Ultra Turrax homogenisieren.

### BEISPIEL 23

### Sonnenschutz Softcreme (W/O). Lichtschutzfaktor (SPF) 40 (Mengenangaben als Gew.-%)

| **Phase** | **Rohstoffe** | **INCI Bezeichnung** | **Anteil** |
|---|---|---|---|
| **A** | Dehymuls PGPH | Polyglyceryl-2 Dipolyhydroxystearate | 5,00 |
| | Copherol 1250 | Tocopheryl acetate | 0,50 |
| | Permulgin 3220 | Ozokerite | 0,50 |
| | Zinkstearat | Zinc Stearate | 0,50 |
| | Tegosoft TN | C12-15 Alkyl Benzoate | 10,00 |
| | Neo Heliopan^{(R)} E1000 | Isoamyl-p-Methoxycinnamate | 2,00 |
| | Neo Heliopan^{(R)} 303 | Octocrylene | 5,00 |
| | Neo Heliopan^{(R)} MBC | 4-Methylbenzylidene Camphor | 3,00 |
| | Zinkoxid neutral | Zinc Oxide | 5,00 |
| B | Wasser, destilliert | Water (Aqua) | Ad 100 |
| | EDETA BD | Disodium EDTA | 0,10 |
| | Glycerin | Glycerin | 4,00 |
| | Phenoxyethanol | Phenoxyethanol | 0,70 |
| | Solbrol M | Methylparaben | 0,20 |
| | Solbrol P | Propylparaben | 0,10 |
| | Magnesiumsulfat | Magnesium Sulfate | 0,50 |
| **C** | Parfümöl B2 aus Beispiel 10 | Parfum (Fragrance) | 0,30 |

Herstellungsverfahren: Teil A auf ca. 85 °C erhitzen. Teil B (ohne Zinkoxid) auf ca. 85 °C erhitzen; Zinkoxid mit dem Ultra Turrax eindispergieren. B zu A geben. Unter Rühren abkühlen lassen. Teil C zugeben und anschließend homogenisieren.

### BEISPIEL 24

### Sonnenschutzmilch (W/O) (Mengenangaben als Gew.-%)

| **Phase** | **Rohstoffe** | **INCI Bezeichnung** | **Anteil** |
|---|---|---|---|
| **A** | Dehymuls PGPH | Polyglyceryl-2 Dipolyhydroxystearate | 3,00 |
| | Bienenwachs 8100 | Beeswax | 1,00 |
| | Monomuls 90-0-18 | Glyceryl Oleate | 1,00 |
| | Zinkstearate | Zinc stearate | 1,00 |
| | Cetiol SN | Cetearyl Isononanoate | 5,00 |
| | Cetiol Ö | Dicaprylyl Ether | 5,00 |
| | Tegosoft TN | C12-15 Alkyl Benzoate | 4,00 |
| | Vitamin E | Tocopherol | 0,50 |
| | Solbrol P | Propylparaben | 0,10 |
| | Neo Heliopan^{(R)} OS | Ethylhexyl Salicylate | 5,00 |
| | Neo Heliopan^{(R)} AV | Ethylhexyl Methoxycinnamate | 7,50 |
| | Uvinul^{(R)} T150 | Ethylhexyl Triazone | 1,50 |
| **B** | Wasser, destilliert | Water (Aqua) | Ad 100 |
| | Trilon BD | Disodium EDTA | 0,10 |
| | Glycerin | Glycerin | 5,00 |
| | Solbrol M | Methylparaben | 0,20 |
| | Phenoxyethanol | Phenoxyethanol | 0,70 |
| | Neo Heliopan^{(R)} AP 10 % ige Lösung, neutralisiert mit NaOH | Disodium Phenyl Dibenzimidazole Tetrasulfonate | 15,00 |
| **C** | Parfümöl G2 aus Beispiel 15 | Parfum (Fragrance) | 0,25 |
| | Alpha Bisabolol | Bisabolol | 0,10 |

Herstellungsverfahren: Teil A auf ca. 85 °C erhitzen. Teil B auf ca. 85 °C erhitzen. B zu A geben. Unter Rühren abkühlen lassen. Teil C zugeben und anschließend homogenisieren.

### BEISPIEL 25A

### Permanent-Haarfärbemittel Teil AHaarfärbungsbase (Mengenangaben als Gew.-%)

| **Rohstoffe** | **Anteil** |
|---|---|
| Natrium Myreth Sulfat (z. B. Texapon K14 S/K, Cognis) | 2,80 |
| Linoleamidopropyl PG-dimonium chlorid phosphat (Arlasilk Phospholipid EFA, Uniqema) | 1,00 |
| Caprylyl- / Capryl glucosid (Plantacare 810 UP, Cognis) | 2,00 |
| Natrium Laureth-6 Carboxylat (Akypo Soft 45 NV, Kao) | 10,00 |
| Cetearylalkohol | 8,00 |
| Octyldodecanol | 1,00 |
| Ceteareth-12 | 0,50 |
| Ceteareth-20 | 0,50 |
| KOH, 50 % ig in Wasser | 0,70 |
| Toluol-2,5-diamine sulfat (oxidierende Farbstoff) | 0,90 |
| Resorcin | 0,20 |
| m-aminophenol | 0,06 |
| 4-chlororesorcin | 0,15 |
| Ascorbinsäure | 0,10 |
| Natriumsulfit | 0,15 |
| Ammoniak, 25 % in Wasser | 6,00 |
| Etidronsäure | 0,20 |
| Polyquaternium-2 (Mirapol A15, Rhodia) | 0,20 |
| Parfümöl G2 aus Beispiel 15 | 0,30 |
| Wasser | 65,24 |

### BEISPIEL 25A

### Permanent-Haarfärbemittel Teil B - Entwickler (Mengenangaben als Gew.-%)

| **Rohstoffe** | **Anteil** |
|---|---|
| Ketostearylalkohol | 8.00 |
| Ceteareth-20 | 2.50 |
| Steartrimonium Chlorid (Dehyquart B, Cognis) | 1.00 |
| 2,6-dicarboxypyridin | 0.10 |
| Paraffinöl | 0.30 |
| Etidronsäure | 0.40 |
| Propylenglykol | 0.40 |
| Natriumbenzoat | 0.04 |
| Wasserstoffperoxid, 50 % in Wasser | 12.00 |
| KOH, 50 % in Water, zugegeben bis pH = 3.5 | q.s. |
| Wasser | Ad 100 |

Anwendung: Die erfindungsgemäße Haarfärbungsbasis (Teil A) und der erfindungsgemäße Entwickler (Teil B) werden in einem Gewichtsverhältnis von 1:1 Verhältnis zusammen gerührt und auf die Haare aufgetragen.

### BEISPIEL 26

### Haarfärbungscreme für eine blonde Farbtönung (Mengenangaben als Gew.-%)

| **Phase** | **Material** | **Hersteller** | **INCI-Name** | **Anteil** |
|---|---|---|---|---|
| **A** | Oxowax | LCW | Cethyl alcohol, Oleyl alcohol, Cetearyl alcohol, Stearic acid | 21,00 |
| | Volpo CS 25 | Croda | Ceteareth25 | 4,00 |
| | Berol 175 | Brenntag Eurochem | Laureth-8 | 10,00 |
| | Lanette(R) E | Cognis | Sodium Cetearyl Sulfate | 1,00 |
| | Covaquat 16 | LCW | Polyquaternium-6 | 4,00 |
| | Wasser | | Water (Aqua) | 49,30 |
| **B** | Ammoniak (20 % in Wasser) | Merk Eurolab | Ammonia | 10,20 |
| **C** | Parfümöl G2 | Symrise | Parfum | 0,50 |

Herstellungsverfahren: Teil A, B und C außer Covaquat 16 zusammen mischen und 20 Minuten bei 80 °C erhitzen. Nachdem das Produkt homogen ist, abkühlen lassen. Covaquat 16 bei 50 °C dazugeben. Wenn das Produkt anfängt zu verdicken die Rührung stoppen. Bei Raumtemperatur NH₄OH dazugegeben und die Mischung bis zur Homogenisierung rühren.

### BEISPIEL 27

### Lufterfrischer Aerosolspray, aus Wasserbasis (w/o Alkohol) (Mengenangaben als Gew.-%)

| **Material** | **Hersteller** | **INCI-Name** | **Anteil** |
|---|---|---|---|
| Imbentin C/125/030 | Symrise | C12-15 pareth-3 | 0,25 |
| Imbentin AG/100/080 | Symrise | deceth-8, Ziegler alcohol | 1,00 |
| Parfümöl H2 aus Beispiel 17 | Symrise | Parfum | 1,25 |
| Wasser, demineralisiert | | Aqua | 22,50 |
| Treibgas 4,2 bar | | | 75,00 |

Herstellungsverfahren: Alle Rohstoffe in der angegebenen Reihenfolge zusammenmischen.

### BEISPIEL 28

### Flüssiges Lufterfrischer Pumpspray (Mengenangaben als Gew.-%)

| **Meterial** | **Hersteller** | **INCI-Name** | **Anteil** |
|---|---|---|---|
| Wasser, demineralisiert | | Aqua | 90,70 |
| Ethanol 96 % | Symrise | Ethanol | 4,00 |
| Empilan KCL 11/90 | Stockmeier | Alcohol C12-15 11EO | 3,00 |
| Isopropanol | Symrise | Propan-2-ol | 1,00 |
| Natriumhydrogencarbonat p.A.. | Symrise | Sodiumbicarbonat | 0,20 |
| Parmetol A 26 | Hösch Julius | Mixture of 5-Chloro-2-methyl-2H-isothiazol-3-one and 2-Methyl-2*H*-isothiazol-3-one | 0,10 |
| Parfümöl F2 | Symrise | Parfum | 1,00 |

Herstellungsverfahren: Alle Rohstoffe in der angegebenen Reihenfolge zusammen mischen. pH-Wert anpassen.

### BEISPIEL 29

### Deodorierender Lufterfrischer Pumpe/Docht 5 % Duftstoff (Mengenangaben als Gew.-%)

| **Phase** | **Material** | **Hersteller** | **INCI-Name** | **Anteil** |
|---|---|---|---|---|
| **A** | Wasser, demineralisiert | | Aqua | 67,32 |
| | Sequion 40 NA 32 | Polygon Chemie | | 1,25 |
| | Triethanolamin pur C | Symrise | Triethanolamine | 0,30 |
| | Rewoderm S 1333 | Goldschmidt | DiNa-Ricinoleamido MEA-Sulfosuccinat | 2,33 |
| | Tego Sorb Conc. 50 | Evonik | Alcohol C12-14, ethoxylated (2-5 EO) + Zn-Ricinoleat | 0,50 |
| | Ethanol 96 % | Symrise | Ethanol | 20,00 |
| **B** | Parfümöl E2 | Symrise | Parfum | 5,00 |
| | Lösungsvermittler | Symrise | | 3,00 |
| **C** | Milchsäure | Symrise | Propanoic acid, 2-hydroxypropionic acid, Lactol | 0,30 |

Herstellungsverfahren: Materialien des Teils A in der angegebenen Reihenfolge zusammen mischen. Materialien des Teil B zusammen mischen. Wenn Teil A klar ist, Teil B dazugeben. Anschließend Teil C zugeben und rühren bis die Mischung homogen ist.

### BEISPIEL 30

### WC-Stein (Mengenangaben als Gew.-%)

| **Phase** | **Material** | **Hersteller** | **INCI-Name** | **Anteil** |
|---|---|---|---|---|
| **A** | Imbentin C/125/200 | Dr. W. Kolb | C12/15 pareth-20 | 4,00 |
| | Rewomid C 212 | Goldschmidt | Cocamide MEA | 6,00 |
| | Marion ARL | Sasol | C10-13, ABS-Na, Pulver | 42,00 |
| **B** | Natriumsulfat pharm. (E514) | Symrise | Sodium sulfate | 42,00 |
| | Parfümöl F2 | Symrise | Parfum | 6,00 |

Herstellungsverfahren: Teil A schmelzen lassen. Teil B dazu mischen mit Hilfe eines Mischkneters. Im Extruder bei 35-40 °C zu einem WC-Stein formen.

### BEISPIEL 31

### Kerze (Mengenangaben als Gew.-%)

| **Material** | **Anteil** |
|---|---|
| Kerzenwachs | 95,00 |
| Parfümöl D2 | 5,00 |

Herstellungsverfahren: Das Kerzenwachs schmelzen lassen und rühren. Das Parfümöl dazugeben, gut rühren. In die gewünschte Form gießen.

### BEISPIEL 32

### Synthese von 3-[(4S)-4-Isopropylcyclohexen-1-yl]propanal (Ib)

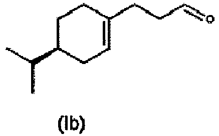

Der Aldehyd 3-[(4*S*)-4-Isopropylcyclohexen-1-yl]propanal (Formel (Ib) wie oben definiert) wird ausgehend von I-(-)-Limonen (79.5 % ee, Formel (Vlb) wie oben definiert) analog zu der Synthese von 3-[(4*R*)-4-Isopropylcyclohexen-1-yl] propanal (la) (Beispiele 4 bis 8) hergestellt. Das Produkt wird mit einer Reinheit von 97 % und mit 78 % ee erhalten.

### BEISPIEL 33

### Substantivität

Der Substantivitätstest ist ein Test zur Bestimmung der sensorischen Haftung eines Stoffes auf Riechstreifen. Die zu untersuchenden Riechstoffe (Verbindung der Formel (la), Verbindung der Formel (Ib) und Lilial® als Benchmark) werden in Form einer 10 %igen Lösung in Triethylcitrat (TEC) jeweils auf einen codierten Riechstreifen getaucht und in definierten zeitlichen Abständen von 15 Prüfern bezüglich der Intensität beurteilt. Die 15 Probanden bewerten die Geruchsintensität auf einer Skala von 1 = geruchlos bis 9 = sehr stark. Die Ergebnisse (Geruchsintensität I in Abhängigkeit von der seit dem Eintauchen des Riechstreifen vergangenen Zeit t in Tagen d) sind in Figur 1 dargestellt.

Die Geruchsintensität wird in folgenden zeitlichen Abständen nach dem Eintauchen der Riechstreifen bewertet: 1 Stunde oder weniger (in Figur 1 als Zeitpunkt "0 Tage" bezeichnet); 1 Tag; 2 Tage; 3 Tage; 4 Tage; 7 Tage; 8 Tage; 9 Tage. Um sicherzustellen, dass die Probanden nicht beeinflusst werden, sind die codierten Riechstreifen nicht in einer chronologischen Reihenfolge auf dem Riechstreifenständer angeordnet.

Die Geruchsintensität der Verbindung der Formel (la) (schwarz ausgefüllter Balken) wird im frischen Zustand und über einen Zeitraum bis zu 3 Tagen von den Probanden im Vergleich zu Lilial® (schwarz schraffierter Balken) als stärker, und nach 4 bis 7 Tagen zumindest vergleichbar mit Lilial® eingeschätzt. Nach 9 Tagen wird sogar erneut eine im Vergleich zu Lilial(R) höhere Geruchsintensität der Verbindung der Formel (la) beobachtet.

Auch die Geruchsintensität der Verbindung (Ib) (schwarz gepunkteter Balken) ist im frischen Zustand stärker als die von Lilial® (schwarz schraffierter Balken) und nach einem Tag vergleichbar mit Lilial®. Danach nimmt die Geruchsintensität der Verbindung der Formel (Ib) zunächst stärker ab als bei Lilial® und der Verbindung der Formel (la), nach 7 bis 9 Tagen sind die Werte jedoch vergleichbar oder leicht höher als bei Lilial®.

## Patentansprüche

1. Verbindung ausgewählt der Formel (la)

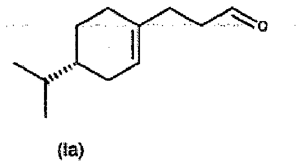

2. Mischung, umfassend-oder bestehend aus
(i) Verbindung der Formel (la) 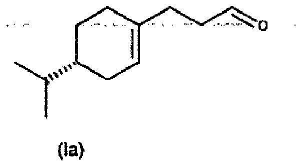
(ii) Verbindung der Formel (Ib)

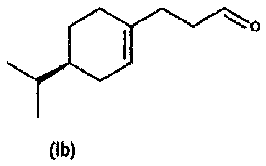

3. Mischung nach Anspruch 2, **dadurch gekennzeichnet, dass**
(a) das Massenverhältnis der Verbindung der Formel (la) zu der Verbindung der Formel (Ib) im Bereich von 0.01 bis 99.99 liegt, vorzugsweise im Bereich von 0.1 bis 99.9, bevorzugt im Bereich von 0.5 bis 99.5, bevorzugt im Bereich von 1.0 bis 99.0, bevorzugt im Bereich von 1.5 bis 98.5, bevorzugt im Bereich von 2.0 bis 98.0 und/oder
(b) der Gesamtanteil der Verbindungen der Formeln (la) und (Ib) grösser ist als 0.01 Gew.- %, vorzugsweise grösser als 1 Gew.- %, bevorzugt grösser als 10 Gew.- %, weiter bevorzugt grösser als 50 Gew.- %, besonders bevorzugt grö- beta er als 90 Gew.- %, bezogen auf das Gesamtgewicht der Mischung umfassend Verbindungen (la) und/oder (Ib).

4. Verwendung einer Verbindung der Formel (la) wie in Anspruch 1 definiert oder einer Mischung bestehend aus der Verbindung der Formel (la) wie in Anspruch 1 definiert und der Verbindung der Formel (Ib) wie in Anspruch 2 definiert
(i) als Riechstoff, insbesondere als Maiglöckchen-Riechstoff und/oder
(ii) als Mittel zum Erhöhen der Substantivität und/oder der Retention einer Riechstoffzubereitung; und/oder
(iii) als Fixateur.

5. Verwendung nach Anspruch 4, wobei
(a) die Verbindung der Formel (la) wie in Anspruch 1 definiert verwendet wird zum Vermitteln, Modifizieren oder Verstärken einer Maiglöckchen-Geruchsnote und optional einer, mehrerer oder sämtlicher Geruchsnoten ausgewählt aus der Gruppe bestehend aus blumig, süß, wässrig, pudrig und ozonig; und/oder
(b) die Verbindung der Formel (Ib) wie in Anspruch 1 definiert verwendet wird zum Vermitteln, Modifizieren oder Verstärken einer Maiglöckchen-Geruchsnote und optional einer, mehrerer oder sämtlicher Geruchsnoten ausgewählt aus der Gruppe bestehend aus fruchtig, grün, wässrig und aldehydig.

6. Riechstoffzubereitung, insbesondere Parfümöl, vorzugsweise mit einer Maiglöckchen-Geruchsnote, bestehend aus oder umfassend
(i) Verbindung der Formel (la) wie in Anspruch 1 definiert oder eine Mischung bestehend aus der Verbindung der Formel (la) wie in Anspruch 1 definiert und der Verbindung der Formel (Ib) wie in Anspruch 2 definiert, und
(ii) einen, 2, 3, 4, 5, 6, 7, 8, 9, 10 oder mehr weitere Riechstoffe, die keine Verbindung der Formel (I) 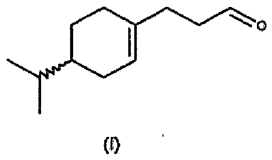 sind.

7. Riechstoffzubereitung, insbesondere Parfümöl, nach Anspruch 6 **dadurch gekennzeichnet, dass** die Menge der Verbindung der Formel (la) wie in Anspruch 1 definiert und/oder der Verbindung der Formel (Ib) wie in Anspruch 2 definiert im Bereich von 0,0001 bis 40 Gew.- % liegt, vorzugsweise im Bereich von 0,001 bis 25 Gew.- %, jeweils bezogen auf das Gesamtgewicht der Riechstoffzubereitung.

8. Riechstoffzubereitung, insbesondere Parfümöl, nach einem der Ansprüche 6 oder 7, umfassend einen, 2, 3 oder mehr weitere Riechstoffe mit einer Maiglöckchen-Geruchsnote und optional einer, mehreren oder sämtlichen Geruchsnoten ausgewählt aus der Gruppe bestehend aus blumig, süß, wässrig, ozonig, holzig, ambriert, Moschus, fruchtig, animalisch.

9. Riechstoffzubereitung, insbesondere Parfümöl, nach einem der Ansprüche 6 bis 8, wobei
(i) die Menge der Verbindung der Formel (la) ausreicht, um der Riechstoffzubereitung eine Maiglöckchen-Geruchsnote und optional eine, mehrere oder sämtliche Geruchsnoten ausgewählt aus der Gruppe bestehend aus blumig, süß, wässrig, pudrig und ozonig zu vermitteln, und/oder
(ii) ein, mehrere oder sämtliche weiteren Riechstoffe der Riechstoffzubereitung eine Maiglöckchen-Geruchsnote und optional eine, mehrere oder sämtliche Geruchsnoten ausgewählt aus der Gruppe bestehend aus blumig, süß, wässrig, pudrig und ozonig vermitteln und die Menge der Verbindung der Formel (la) ausreicht, um im Vergleich zu einer ansonsten identisch zusammengesetzten Vergleichs-Riechstoffzubereitung ohne die Verbindung der Formel (la) die Maiglöckchen-Geruchsnote und optional eine, mehrere oder sämtliche der Geruchsnoten blumig, süß, wässrig, pudrig und ozonig zu modifizieren und/oder zu verstärken, und/oder
(iii) die Menge der Verbindung der Formel (Ib) ausreicht, um der Riechstoffzubereitung eine Maiglöckchen-Geruchsnote und optional eine, mehrere oder sämtliche Geruchsnoten ausgewählt aus der Gruppe bestehend aus fruchtig, grün, wässrig und aldehydig zu vermitteln, und/oder
(iv) ein, mehrere oder sämtliche weiteren Riechstoffe der Riechstoffzubereitung eine Maiglöckchen-Geruchsnote und optional eine, mehrere oder sämtliche Geruchsnoten ausgewählt aus der Gruppe bestehend aus fruchtig, grün, wässrig und aldehydig vermitteln und die Menge der Verbindung der Formel (Ib) ausreicht, um im Vergleich zu einer ansonsten identisch zusammengesetzten Vergleichs-Riechstoffzubereitung ohne die Verbindung der Formel (Ib) die Maiglöckchen-Geruchsnote und optional eine, mehrere oder sämtliche der Geruchsnoten ausgewählt aus der Gruppe bestehend aus fruchtig, grün, wässrig und aldehydig zu modifizieren und/oder zu verstärken.

10. Parfümiertes Produkt umfassend Verbindung der Formel (la) wie in Anspruch 1 definiert oder eine Mischung bestehend aus der Verbindung der Formel (la) wie in Anspruch 1 definiert und der Verbindung der Formel (Ib) wie in Anspruch 2 definiert, vorzugsweise in einer sensorisch wirksamen Menge.

11. Parfümiertes Produkt nach Anspruch 10, **dadurch gekennzeichnet, dass** die Menge der Verbindung der Formel (la) wie in Anspruch 1 definiert im Bereich von 0,0001 bis 5 Gew.- % liegt, vorzugsweise im Bereich von 0,001 bis 2,5 Gew.- %, jeweils bezogen auf das Gesamtgewicht des Produkts.

12. Parfümiertes Produkt nach Anspruch 10 oder 11, ausgewählt aus der Gruppe bestehend aus Wasch- und Reinigungsmitteln, Hygiene- oder Pflegeprodukten, insbesondere aus dem Bereich der Körper- und Haarpflege, der Kosmetik und des Haushalt, Fine Fragrance (Parfüm), Air Care (insbesondere Kerzen), Lufterfrischer.

13. Verfahren zum Versehen von Haut, Haaren, Oberflächen oder textilen Fasern mit einer Maiglöckchen-Geruchsnote mit folgenden Schritten:
(a) Bereitstellen einer Riechstoffzubereitung, vorzugsweise nach einem der Ansprüche 6 bis 9, umfassend
(b) Verbindungen der Formel (la) wie in Anspruch 1 definiert oder eine Mischung bestehend aus der Verbindung der Formel (la) wie in Anspruch 1 definiert und der Verbindung der Formel (Ib) wie in Anspruch 2 definiert und
(c) einen oder mehrere weitere Riechstoffe, die keine Verbindung der Formel (la) oder (Ib) sind wobei in der Riechstoffzubereitung die Menge der Verbindung der Formel (I) ausreicht, um eine Maiglöckchen-Geruchsnote zu vermitteln, zu modifizieren und/oder zu verstärken;
(d) Applizieren der Riechstoffzubereitung auf Haut, Haare oder textile Fasern.

14. Verfahren zum Herstellen der Verbindung der Formel (Ia) wie in Anspruch 1 definiert umfassend den Schritt säurekatalysiertes Umsetzen eines Acetals der Formel (II), so dass die Verbindung der Formel (I) gebildet wird 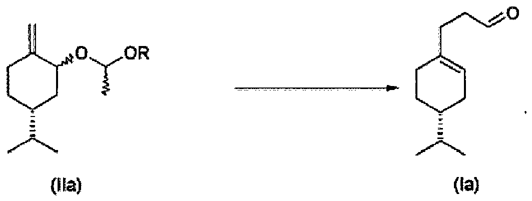 wobei die Gruppe R in dem Acetal der Formel (IIa) eine verzweigte oder unverzweigte, gesättigte oder ungesättigte Alkylgruppe mit 1 bis 10 Kohlenstoff-Atomen ist, bevorzugt eine Butylgruppe.

15. Acetal der Formel (II) 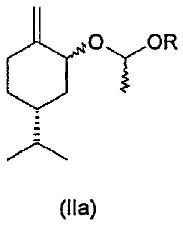 wobei die Gruppe R in dem Acetal der Formel (IIa) eine verzweigte oder unverzweigte, gesättigte oder ungesättigte Alkylgruppe mit 1 bis 10 Kohlenstoff-Atomen ist, bevorzugt eine Butylgruppe.

16. Verwendung
(i) von d-(+) Limonen als Edukt zur Herstellung der Verbindung der Formel (la) wie in Anspruch 1 definiert, oder
(ii) einer Mischung enthaltend d-(+) Limonen und I-(-) Limonen als Edukt zur Herstellung einer Mischung enthaltend die Verbindung der Formel (la) wie in Anspruch 1 definiert und die Verbindung der Formel (Ib) wie in Anspruch 2 definiert.

17. Verfahren zum Vermitteln, Verstärken und/oder Modifizieren einer Maiglöckchen-Geruchsnote und optional einer, mehrerer oder sämtlicher Geruchsnoten ausgewählt aus der Gruppe bestehend aus blumig, süß, wässrig, pudrig und ozonig umfassend den folgenden Schritt: Mischen oder in Kontakt bringen der Verbindung der Formel (la) wie in Anspruch 1 definiert, oder einer Mischung nach einem der Ansprüche 2 oder 3, oder einer Riechstoffzubereitung nach einem der Ansprüche 6 bis 9 vorzugsweise in einer sensorisch wirksamen Menge mit einem Erzeugnis.

## Claims

1. Compound selected from formula (Ia)

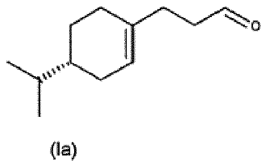

2. Mixture, comprising or consisting of
(i) a compound of formula (Ia) 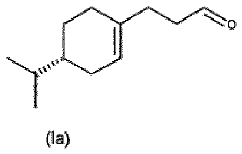
(ii) a compound of formula (Ib)

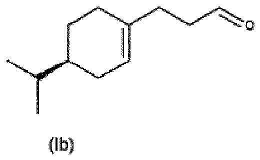

3. Mixture of claim 2, **characterized in that**,
(a) the mass ratio of the compound of formula (Ia) to the compound of formula (Ib) is in the range from 0.01 to 99.99, preferably in the range from 0.1 to 99.9, preferably in the range from 0.5 to 99.5, preferably in the range from 1.0 to 99.0, preferably in the range from 1.5 to 98.5, preferably in the range from 2.0 to 98.0 and/or
(b) the total amount of the compounds of formula (Ia) and (Ib) is greater than 0.01 w.t.%, preferably greater than 1 w.t.%, preferably greater than 10 w.t.%, more preferably greater than 50 w.t.%, most preferred greater than 90 w.t.%, relative to the total weight of the mixture comprising compounds (Ia) and/or (Ib).

4. Use of a compound of formula (Ia) as defined in claim 1 or a mixture consisting of the compound of formula (Ia) as defined in claim 1 and the compound of formula (Ib) as defined in claim 2
(i) as fragrance, in particular as lily of the valley fragrance and/or
(ii) as an agent for increasing the substantivity and/or the retention of a fragrance preparation; and/or
(iii) as fixative.

5. The use of claim 4, wherein
(a) the compound of formula (Ia) as defined in claim 1 is used for mediating, modifying or intensifying an odor note of lily of the valley and optionally one, several or all odor notes selected from the group consisting of floral, sweet, watery, powdery and ozone-like; and/or
(b) the compound of formula (Ia) as defined in claim 1 is used for mediating, modifying or intensifying an odor note of lily of the valley and optionally one, several or all odor notes selected from the group consisting of fruity, green, watery and aldehyde-like.

6. A fragrance preparation, in particular perfume oil, preferably with an odor note of lily of the valley, consisting of or comprising
(i) compound of formula (Ia) as defined in claim 1 or a mixture consisting of the compound of formula (Ia) as defined in claim 1 and the compound of formula (Ib) as defined in claim 2, and
(ii) one, 2, 3, 4, 5, 6, 7, 8, 9, 10 or more further fragrances, which are not the compound of formula (I)

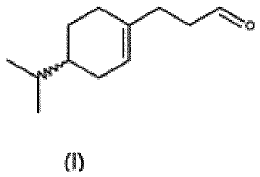

7. A fragrance preparation, in particular a perfume oil of claim 6, **characterized in that**, the amount of the formula (Ia) as defined in claim 1 and/or the compound of formula (Ib) as defined in claim 2, is in the range from 0,0001 to 40 w.t.%. preferably in the range from 0,001 to 25 w.t.%, each based on the total weight of the fragrance preparation.

8. A fragrance preparation, in particular a perfume oil according to any of claims 6 or 7, comprising one, 2, 3 or more further fragrances with an odor note of lily of the valley and optionally one, several or all odor notes selected from the group consisting of floral, sweet, watery, ozone-like, woody, ambered, musky, fruity, animal.

9. A fragrance preparation, in particular a perfume oil according to any of claim 6 to 8,
(i) the amount of compound of formula (Ia) is sufficient to mediate an odor note of lily of the valley and optionally one, several or all odor notes mediate notes selected from the group consisting of floral, sweet, watery, powdery and ozone-like to the fragrance preparation, and/or
(ii) one, several or all further fragrances of the fragrance preparation mediate an odor note of lily of the valley and optionally one, several or all odor notes mediate notes selected from the group consisting of floral, sweet, watery, powdery and ozone-like and the amount of the compound of formula (Ia) is sufficient in comparison to an identical composed comparison fragrance preparation without the compound of formula (Ia) to modify and/or intensify the odor note of lily of the valley and optionally one, several or all odor notes selected from the group consisting of floral, sweet, watery, powdery and ozone-like, and/or
(iii) the amount of compound of formula (Ia) is sufficient to mediate an odor note of lily of the valley and optionally one, several or all odor notes mediate notes selected from the group consisting of fruity, green, watery and aldehyde-like to the fragrance preparation, and/or
(iv) one, several or all further fragrances of the fragrance preparation mediate an odor note of lily of the valley and optionally one, several or all odor notes mediate notes selected from the group consisting of fruity, green, watery and aldehyde-like and the amount of the compound of formula (Ib) is sufficient in comparison to an identical composed comparison fragrance preparation without the compound of formula (Ib) to modify and/or intensify the odor note of lily of the valley and optionally one, several or all odor notes selected from the group consisting of fruity, green, watery and aldehyde-like.

10. Perfumed product comprising compound of formula (Ia) as defined in claim 1 or a mixture consisting of the compound of formula (Ia) as defined in claim 1 and the compound of formula (Ib) as defined in claim 2, preferably in a sensory effective amount.

11. Perfumed product of claim 10, **characterized in that**, the amount of the formula (Ia) as defined in claim 1 is in the range from 0,0001 to 5 w.t.%. preferably in the range from 0,001 to 2,5 w.t.%, each based on the total weight of the product.

12. Perfumed product of claims 10 or 11, selected from the group consisting of washing and cleaning products, hygiene or care products, in particular from the area of body and hair care, cosmetics and household products, fine fragrance (perfume), air care (especially candles), air fresheners.

13. Method of providing skin, hair, surfaces, or textile fibers with an odor note of lily of the valley comprising the following steps:
(a) Providing a fragrance preparation, preferably according to any claims 6 to 9, comprising
(b) compounds of the formula (Ia) as defined in claim 1 or a mixture consisting of the compound of formula (Ia) as defined in claim 1 and the compound of formula (Ib) as defined in claim 2, and
(c) one or several further fragrances which are not compound of formula (Ia) or (Ib), wherein the amount of compound of formula (I) in the fragrance preparation is sufficient for mediating, modifying and/or intensifying an odor note of lily of the valley;
(d) applying the fragrance preparation to the skin, hair, surfaces, or textile fibers.

14. Method for producing the compound of formula (I) as defined in claim 4, comprising the step of acid catalyzed reaction of an acetal of formula (II), so that the compound of formula (I) is formed 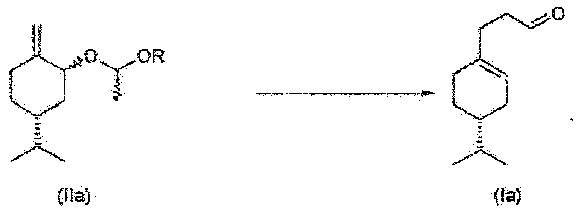 wherein the group R in the acetal of formula (IIa) is a branched or unbranched, saturated or unsaturated alkyl group with 1 to 10 carbon atoms, preferably a butyl group.

15. Acetal of formula (II) 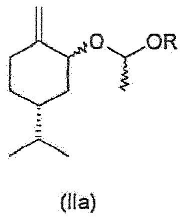 wherein the group R in the acetal of formula (IIa) is a branched or unbranched, saturated or unsaturated alkyl group with 1 to 10 carbon atoms, preferably a butyl group.

16. Use of
(i) d-(+) limonene as educt for producing the compound of formula (I) as defined in claim 1, or
(ii) a mixture containing d-(+) limonene and 1-(-) limonene as educt for producing a mixture containing the compound of formula (Ia) as defined in claim 1 and the compound of formula (Ib) as defined in claim 2.

17. Method for mediating, intensifying and/or modifying an odor note of lily of the valley and optionally one, several or all odor notes selected from the group consisting of floral, sweet, watery, powdery and ozone-like, comprising the following steps: mixing or bringing in contact of the compound of formula (Ia) as defined in claim 1 or a mixture according toany claims 2 or 3, or a fragrance preparation of any claims 6 to 9, preferably in a sensory effective amount with a product.

## Revendications

1. Composé sélectionné de Formule (la)

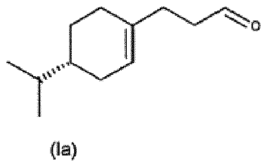

2. Mélange, comprenant, ou en étant constitué,
(i) un composé de Formule (la) 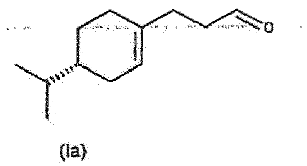
(ii) un composé de Formule (Ib)

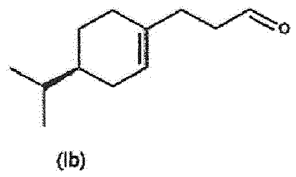

3. Mélange selon la revendication 2, **caractérisé en ce que**
(a) le rapport en masse du composé de formule (la) au composé de formule (Ib) est compris dans la plage de 0,01 à 99,99, de préférence dans la plage de 0,1 à 99,9, préférentiellement dans la plage de 0,5 à 99,5, préférentiellement dans la plage de 1,0 à 99,0, préférentiellement dans la plage de 1,5 à 98,5, préférentiellement dans la plage de 2,0 à 98,0, et/ou
(b) la proportion totale des composés de formule (la) et (Ib) est supérieure à 0,01 % en poids, de préférence supérieure à 1 % en poids, préférentiellement supérieure à 10 % en poids, d'une manière encore plus préférée supérieure à 50 % en poids, d'une manière particulièrement préférée supérieure à 90 % en poids, par rapport au poids total du mélange comprenant les composés (la) et/ou (Ib).

4. Utilisation d'un composé de formule (la) tel que défini dans la revendication 1 ou d'un mélange consistant en le composé de formule (la) tel que défini dans la revendication 1 et le composé de formule (Ib) tel que défini dans la revendication 2,
(i) en tant que parfum, en particulier en tant que parfum de muguet, et/ou
(ii) en tant qu'agent pour augmenter la substantivité et/ou la rétention d'une préparation parfumante ; et/ou
(iii) en tant que fixateur.

5. Utilisation selon la revendication 4, pour laquelle
(a) le composé de formule (la) tel que défini dans la revendication 1 est utilisé pour conférer, modifier ou renforcer une note de muguet et en option l'une, plusieurs ou la totalité des notes choisies dans le groupe consistant en les notes florale, sucrée, aqueuse, poudrée et/ou d'ozone ; et/ou
(b) le composé de formule (Ib) tel que défini dans la revendication 1 est utilisé pour conférer, modifier ou renforcer une note de muguet et en option l'une, plusieurs ou la totalité des notes choisies dans le groupe consistant en les notes fruitée, verte, aqueuse et aldéhydée.

6. Préparation parfumante, en particulier huile de parfum, ayant de préférence une note de muguet, comprenant, ou en étant constituée
(i) le composé de formule (la) tel que défini dans la revendication 1 ou un mélange consistant en le composé de formule (la) tel que défini dans la revendication 1 et du composé de formule (Ib) tel que défini dans la revendication 2, et
(ii) un, 2, 3, 4, 5, 6, 7, 8, 9, 10 ou plus autres parfums, qui ne sont pas des composés de formule (I)

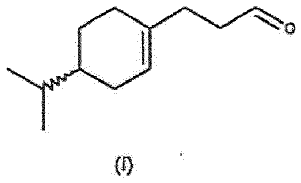

7. Préparation parfumante, en particulier huile de parfum selon la revendication 6, **caractérisée en ce que** la quantité du composé de formule (la) tel que défini dans la revendication 1 et/ou du composé de formule (Ib) tel que défini dans la revendication 2 est comprise dans la plage de 0,0001 à 40 % en poids, de préférence dans la plage de 0,001 à 25 % en poids, dans chaque cas par rapport au poids total de la préparation parfumante.

8. Préparation parfumante, en particulier huile de parfum selon l'une des revendications 6 ou 7, comprenant un, 2, 3 ou plus autres parfums ayant une note de muguet et en option l'une, plusieurs ou la totalité des notes choisies dans le groupe consistant en les notes florale, sucrée, aqueuse, d'ozone, boisée, ambrée, musquée, fruitée, animale.

9. Préparation parfumante, en particulier huile de parfum selon l'une des revendications 6 à 8, dans laquelle
(i) la quantité du composé de formule (la) est suffisante pour conférer à la préparation parfumante une note de muguet et en option l'une, plusieurs ou la totalité des notes choisies dans le groupe consistant en les notes florale, sucrée, aqueuse, poudrée et d'ozone, et/ou
(ii) un, plusieurs ou la totalité d'autres parfums confèrent à la préparation parfumante une note de muguet et en option l'une, plusieurs ou la totalité des notes choisies dans le groupe consistant en les notes florale, sucrée, aqueuse, poudrée et d'ozone, et la quantité du composé de formule (la) est suffisante pour modifier et/ou pour renforcer, par comparaison avec une préparation parfumante comparative ayant une composition par ailleurs identique, sans le composé de formule (la), la note de muguet et en option l'une, plusieurs ou la totalité des notes florale, sucrée, aqueuse, poudrée et d'ozone, et/ou
(iii) la quantité du composé de formule (Ib) est suffisante pour conférer à la préparation parfumante une note de muguet et en option l'une, plusieurs ou la totalité des notes choisies dans le groupe consistant en les notes fruitée, verte, aqueuse et aldéhydée, et/ou
(iv) un, plusieurs ou la totalité d'autres parfums de la préparation parfumante confèrent une note de muguet et en option l'une, plusieurs ou la totalité des notes choisies dans le groupe consistant en les notes fruitée, verte, aqueuse et aldéhydée, et la quantité du composé de formule (Ib) est suffisante pour modifier et/ou pour renforcer, par rapport à une préparation parfumante comparative ayant une composition par ailleurs identique, sans le composé de formule (Ib), la note de muguet et en option l'une, plusieurs ou la totalité des notes choisies dans le groupe consistant en les notes fruitée, verte, aqueuse et aldéhydée.

10. Produit parfumé comprenant un composé de formule (la) tel que défini dans la revendication 1 ou un mélange consistant en les composé de formule (la) tel que défini dans la revendication 1 et du composé de formule (Ib) tel que défini dans la revendication 2, de préférence en une quantité à effet sensoriel.

11. Produit parfumé selon la revendication 10, **caractérisé en ce que** la quantité du composé de formule (la) tel que défini dans la revendication 1 est comprise dans la plage de 0,0001 à 5 % en poids, de préférence dans la plage de 0,001 à 2,5 % en poids, dans chaque cas par rapport au poids total du produit.

12. Produit parfumé selon la revendication 10 ou 11, choisi dans le groupe consistant en les produits lavants et détergents, les produits d'hygiène ou de soin, en particulier du domaine des soins corporels et du soin des cheveux, de la cosmétique et du ménage, des Fine Fragrances (parfums), de l'Air Care (en particulier les bougies), des rafraîchisseurs d'air.

13. Procédé pour conférer à la peau, aux cheveux, aux surfaces ou aux fibres textiles une note de muguet, comportant les étapes suivantes :
(a) fourniture d'une préparation parfumante, de préférence selon l'une des revendications 6 à 9, comprenant
(b) des composés de formule (la) tels que définis dans la revendication 1 ou un mélange consistant en le composé de formule (la) tel que défini dans la revendication 1 et le composé de formule (Ib) tel que défini dans la revendication 2, et
(c) un ou plusieurs autres parfums, qui ne sont pas des composés de formule (la) ou (Ib), la quantité du composé de formule (I) étant, dans la préparation parfumante, suffisante pour conférer, pour modifier et/ou pour renforcer une note de muguet ;
(d) application de la préparation parfumante sur la peau, les cheveux ou les fibres textiles.

14. Procédé de fabrication du composé de formule (la) tel que défini dans la revendication 1, comprenant l'étape de conversion catalysée par un catalyseur acide d'un acétal de formule (II), de façon à former le composé de formule (I) 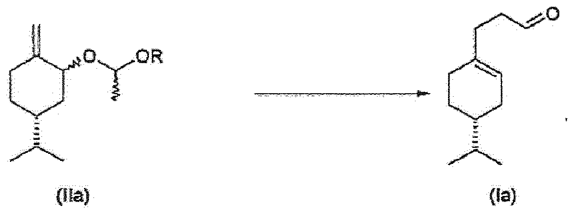 le groupe R de l'acétal de formule (IIa) étant un groupe alkyle à chaîne droite ou ramifiée, saturé ou insaturé, ayant 1 à 10 atomes de carbone, de préférence un groupe butyle.

15. Acétal de formule (II) 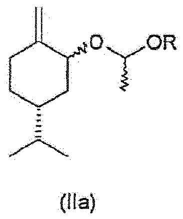 dans lequel le groupe R de l'acétal de formule (IIa) est un groupe alkyle à chaîne droite ou ramifiée, saturé ou insaturé, ayant 1 à 10 atomes de carbone, de préférence un groupe butyle.

16. Utilisation
(i) de d-(+)-limonène en tant que matière de départ pour la fabrication du composé de formule (la) tel que défini dans la revendication 1, ou
(ii) d'un mélange contenant du d-(+)-limonène et/ou du I-(-)-limonène en tant que matière de départ pour la fabrication d'un mélange contenant le composé de formule (la) tel que défini dans la revendication 1 et le composé de formule (Ib) tel que défini dans la revendication 2.

17. Procédé pour conférer, renforcer et/ou modifier une note de muguet et en option l'une, plusieurs ou la totalité des notes choisies dans le groupe consistant en les notes florale, sucrée, aqueuse, poudrée et d'ozone, comprenant l'étape suivante : mélange ou mise en contact du composé de formule (la) tel que défini dans la revendication 1, ou
d'un mélange selon l'une des revendications 2 ou 3, ou
d'une préparation parfumante selon l'une des revendications 6 à 9, de préférence en une quantité à effet sensoriel avec un seul produit.
